# EUROPEAN PATENT SPECIFICATION

(11) **EP 3 455 344 B1**
(45) Date of publication and mention of the grant of the patent: **20.11.2024**
(21) Application number: 17795198.5
(22) Date of filing: 12.05.2017
(51) Int. Cl.: C12N 5/02, C12N 5/0789

(54) **HAEMATOPOIETIC STEM/PROGENITOR CELLS**
HÄMATOPOETISCHE STAMM-/VORLÄUFERZELLEN
CELLULES SOUCHES/PROGÉNITRICES HÉMATOPOÏÉTIQUES

(30) Priority: 13.05.2016 AU 2016901785
(43) Date of publication of application: 20.03.2019
(73) Proprietor: Murdoch Childrens Research Institute, Parkville, Victoria 3052 (AU)
(72) Inventor: NG, Elizabeth, Parkville Victoria 3052 (AU); STANLEY, Edouard, Ascot Vale Victoria 3032 (AU); ELEFANTY, Andrew, Surrey Hills Victoria 3127 (AU)
(74) Representative: De Clercq & Partners
(86) International application number: PCT/AU2017/050440
(87) International publication number: WO 2017/193177

(56) References cited:
- E NG: "HOXA gene expression defines definitive fetal hematopoietic cells differentiated from hESCs", EXPERIMENTAL HEMATOLOGY, vol. 43, no. 9, 1 September 2015 (2015-09-01), pages s46, XP055602102, DOI: 10.1016/j.exphem.2015.06.056
- MARION KENNEDY ET AL: "T Lymphocyte Potential Marks the Emergence of Definitive Hematopoietic Progenitors in Human Pluripotent Stem Cell Differentiation Cultures", CELL REPORTS, vol. 2, no. 6, 1 December 2012 (2012-12-01), pages 1722 - 1735, XP055171288, ISSN: 2211-1247, DOI: 10.1016/j.celrep.2012.11.003
- MARK TUTHILL ET AL: "Hematopoietic stem cell transplantation", STEM CELLS AND CLONING: ADVANCES AND APPLICATIONS, 1 August 2010 (2010-08-01), pages 105, XP055640704, DOI: 10.2147/SCCAA.S6815
- CHRISTOPHER M STURGEON ET AL: "Wnt signaling controls the specification of definitive and primitive hematopoiesis from human pluripotent stem cells", NATURE BIOTECHNOLOGY, vol. 32, no. 6, 18 May 2014 (2014-05-18), New York, pages 554 - 561, XP055640710, ISSN: 1087-0156, DOI: 10.1038/nbt.2915
- PICK MARJORIE ET AL: "Differentiation of human embryonic stem cells in serum-free medium reveals distinct roles for bone morphogenetic protein 4, vascular endothelial growth factor, stem cell factor, and fibroblast growth factor 2 in hematopoiesis", STEM CELLS, ALPHAMED PRESS, INC, UNITED STATES, vol. 25, no. 9, 1 September 2007 (2007-09-01), pages 2206 - 2214, XP008092704, ISSN: 1549-4918, [retrieved on 20070607], DOI: 10.1634/STEMCELLS.2006-0713
- PEARSON STELLA ET AL: "The stepwise specification of embryonic stem cells to hematopoietic fate is driven by sequential exposure to Bmp4, activin A, bFGF and VEGF", DEVELOPMENT (SPECIAL ISSUE ON DEVELOPMENT AT THE SINGLE CELL LEVEL), THE COMPANY OF BIOLOGISTS LTD, GB, vol. 135, no. 8, 1 April 2008 (2008-04-01), pages 1525 - 1535, XP008092701, ISSN: 0950-1991, DOI: 10.1242/DEV.011767
- NG, E. ET AL.: "HOXA gene expression defines definitive fetal hematopoietic cells differentiated from hESCs", EXPERIMENTAL HEMATOLOGY, vol. 43, no. 9, 2015, pages s46, XP055602102, DOI: 10.1016/j.exphem.2015.06.056
- BRUVERIS, F. ET AL.: "Blood cells form from phenotypically distinct precursors during human blast colony differentiation", EXPERIMENTAL HEMATOLOGY, vol. 43, no. 9, 2015, pages s60, XP055602109, DOI: 10.1016/j.exphem.2015.06.113
- KITAJIMA, K. ET AL.: "GSK3p inhibition activates the CDX/HOX pathway and promotes hemogenic endothelial progenitor differentiation from human pluripotent stem cells", EXPERIMENTAL HAEMATOLOGY, vol. 44, no. 1, 23 October 2016 (2016-10-23), pages 68 - 74, XP029348376, DOI: 10.1016/j.exphem.2015.09.007
- WANG, C. ET AL.: "TGF beta inhibition enhances the generation of hematopoietic progenitors from human ES cell -derived hemogenic endothelial cells using a stepwise strategy", CELL RESEARCH, vol. 22, 23 August 2011 (2011-08-23), pages 194 - 207, XP055602518, DOI: 10.1038/cr.2011.138
- FERRELL, P.I. ET AL.: "The RUNX1 +24 enhancer and PI promoter identity a unique subpopulation of hematopoietic progenitor cells derived from human pluripotent stem cells", STEM CELLS, vol. 33, no. 4, April 2015 (2015-04-01), pages 1130 - 1141, XP055602521, ISSN: 1066-5099, DOI: 10.1002/stem.1940
- CHANDA, B. ET AL.: "Retinoic acid signaling is essential for embryonic hematopoietic stem cell development", CELL, vol. 155, 2013, pages 215 - 227, XP028729738, DOI: doi:10.1016/j.cell.2013.08.055
- CLARKE, R.L. ET AL.: "The expression of Soxl7 identifies and regulates hemogenic endothelium", NATURE CELL BIOLOGY, vol. 15, no. 5, May 2013 (2013-05-01), pages 502 - 510, XP055516926, DOI: 10.1038/ncb2724
- DOU, D. ET AL.: "Medial HOXA genes demarcate haematopoietic stem cell fate during human development", NATURE CELL BIOLOGY, vol. 18, no. 6, June 2016 (2016-06-01), pages 595 - 606, XP055602527, ISSN: 1465-7392, DOI: 10.1038/ncb3354
- NG, E. ET AL.: "Differentiation of human embryonic stem cells to HOXA+ hemogenic vasculature that resembles the aorta-gonad-mesonephros", NATURE BIOTECHNOLOGY, vol. 34, no. 11, 17 October 2016 (2016-10-17), pages 1168 - 1179, XP055602531, ISSN: 1087-0156, DOI: 10.1038/nbt.3702
- NG, E. ET AL.: "A protocol describing the use of a recombinant protein-based, animal product-free medium (APEL) for human embryonic stem cell differentiation as spin embryoid bodies", NATURE PROTOCOLS, vol. 3, no. 5, 2008, pages 768 - 776, XP008097880, DOI: doi:10.1038/nprot.2008.42

## Description

### FIELD

The invention relates to differentiating a pluripotent stem cell (PSC) into a definitive haematopoietic stem/progenitor cell. The specification also relates to therapeutic uses of the haematopoietic stem/progenitor cell.

### BACKGROUND

Hematopoietic stem cell (HSC) transplantation permits the reconstitution of the blood cell compartment, for example in patients receiving myeloablative therapy. Because many patients do not have an optimal matched donor, the provision of HSCs from differentiated PSCs would be beneficial. Despite considerable efforts, *in vitro* haematopoietic differentiation remains skewed towards embryonic, yolk-sac like blood that lacks re-population activity, i.e. primitive haematopoiesis.

It follows that production of definitive haematopoietic stem/progenitor cells from PSCs, in particular human PSCs (hPSCs), remains a significant challenge, and identification of differentiation conditions that replicate early definitive mammalian blood cell development is needed.

Ng et al., Experimental Hematology, vol. 43, no. 9, page S46 (2015) describe that HOXA gene expression defines definitive fetal hematopoietic cells differentiated from human embryonic stem cells.

Kennedy et al., Cell Reports, 2, 1722-1735 (2012) describe the use of T-cell potential to monitor hematopoietic development in human embryonic stem cell and induced pluripotent stem cell cultures. In this study, manipulation of Activin/Nodal signaling during early stages of differentiation revealed that development of the definitive hematopoietic progenitor population was not dependent on this pathway, distinguishing it from primitive hematopoiesis.

It is to be understood that if any prior art publication is referred to herein, such reference does not constitute an admission that the publication forms a part of the common general knowledge in the art in Australia or any other country.

### SUMMARY

*RUNX1* and SOX17 encode transcription factors critical to definitive haematopoiesis. SOX17 expression is a marker of haemogenic endothelium, whereas one isoform of *RUNX1, RUNX1C* is a marker of haematopoietic stem/progenitor cells. In view of this, the inventors produced PSCs comprising a SOX17 reporter and/or a *RUNX1C* reporter and used these PSCs to investigate definitive haematopoiesis.

Investigation of homozygous and heterozygous *RUNX1C* reporter PSCs together with repopulation-competent PSCs from cord blood revealed the surprising finding that HOXA genes were not expressed in the *RUNX1C* reporter PSCs.

The inventors then set about increasing expression of *HOXA* genes in *RUNX1C*/*SOX17* dual reporter PSCs using a combination of WNT agonist and ACTIVIN antagonist, and established a robust in vitro differentiation system that encompasses the developmental period from mesoderm formation to AGM-like definitive haematopoiesis, providing a platform for definitive haematopoiesis and the generation of repopulating HSCs.

Based on the inventors' findings, a first aspect of the specification provides an isolated cell having, after culturing a pluripotent stem cell (PSC) in a medium comprising a WNT agonist and an ACTIVIN antagonist, increased HOXA gene expression relative to a PSC not cultured in a medium comprising a WNT agonist and an ACTIVIN antagonist, wherein the cell with increased HOXA gene expression is capable of generating a definitive haematopoietic stem/precursor cell.

A second aspect of the specification provides an isolated cell, comprising a SOX17 reporter and a *RUNX1C* reporter, wherein the SOX17 reporter and the *RUNX1C* reporter are distinguishable.

A third aspect of the specification provides use of the cell of the first aspect for generating a definitive haematopoietic stem/precursor cell.

A fourth aspect of the specification provides use of the cell of the second aspect for tracking differentiation of a PSC into a definitive haematopoietic stem/precursor cell.

In a fifth aspect, the invention provides a method for differentiating a PSC into a definitive haematopoietic stem/precursor cell, the method comprising increasing HOXA gene expression by culturing the PSC in medium comprising a WNT agonist and an ACTIVIN antagonist for about 2 days, wherein increased HOXA gene expression is relative to a PSC not cultured in medium comprising a WNT agonist and an ACTIVIN antagonist.

A sixth aspect of the specification provides a definitive haematopoietic stem/progenitor cell differentiated from a PSC by the method of the fifth aspect.

A seventh aspect of the specification provides a therapeutic composition comprising the cell of the first or sixth aspect.

An eighth aspect of the specification provides an infusion bag comprising the cell of the first or sixth aspect or the therapeutic composition of the seventh aspect.

A ninth aspect of the specification provides a method for treating a condition, disease or disorder requiring HSC transplantation, the method comprising administering to a subject the cell of the first or sixth aspect or the therapeutic composition of the seventh aspect.

Alternatively or additionally to the ninth aspect, the specification provides the use of the cell of the first or sixth aspect in the manufacture of a medicament for treating a condition, disease or disorder requiring HSC transplantation.

Alternatively or additionally, the specification provides the cell of the first or sixth aspect or the therapeutic composition of the seventh aspect for use in a method for treating a condition, disease or disorder requiring HSC transplantation, the method comprising administering to a subject the cell or therapeutic composition.

### BRIEF DESCRIPTION OF THE FIGURES

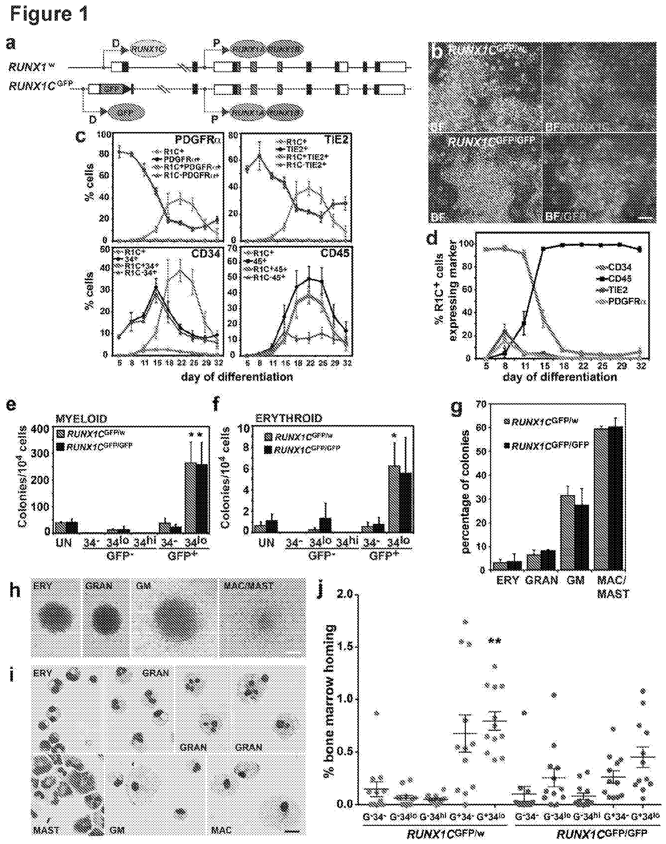

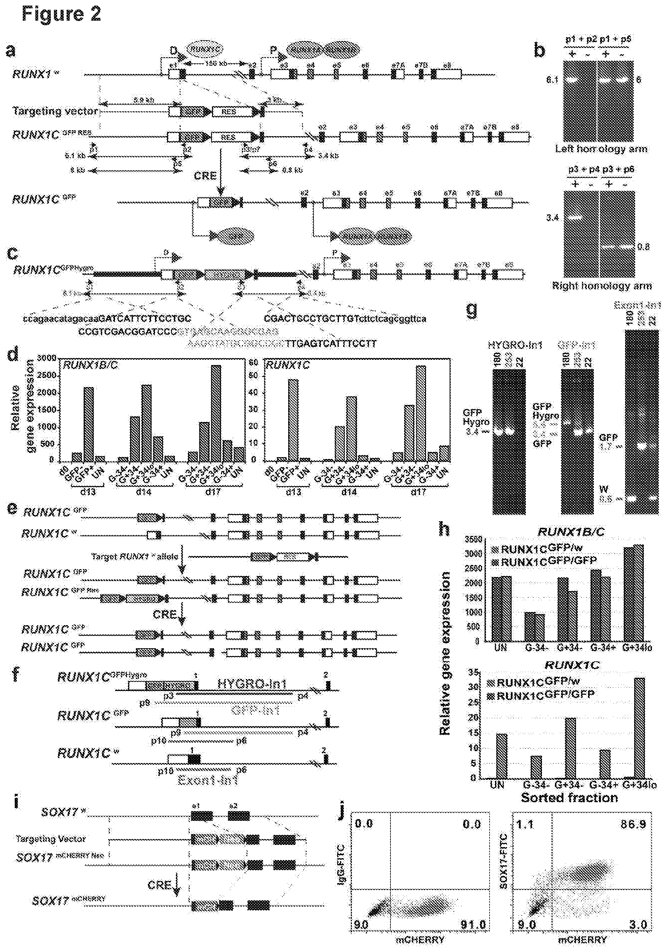

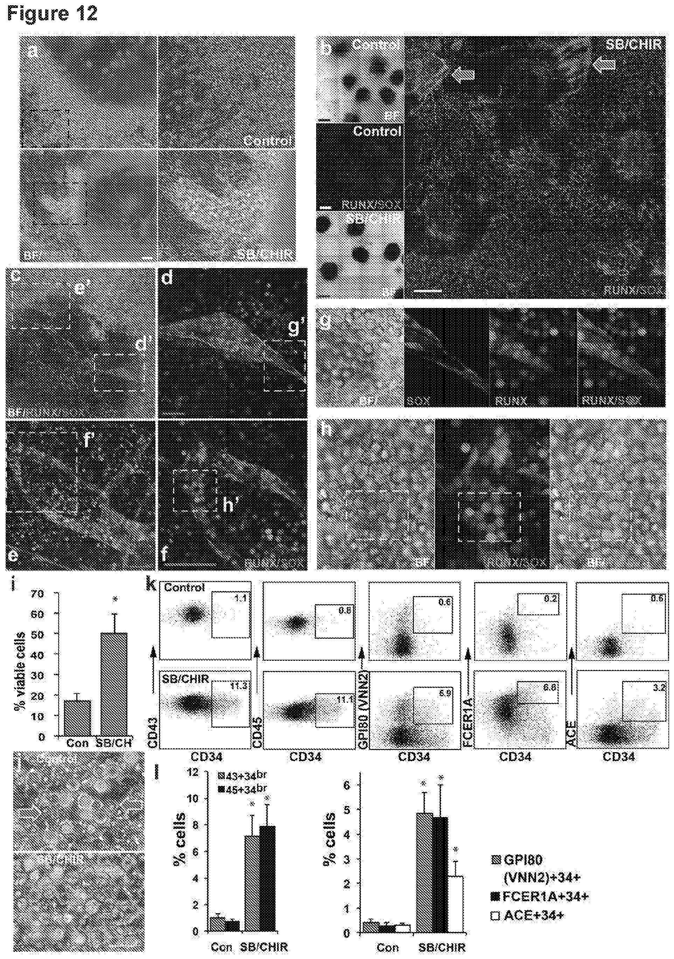

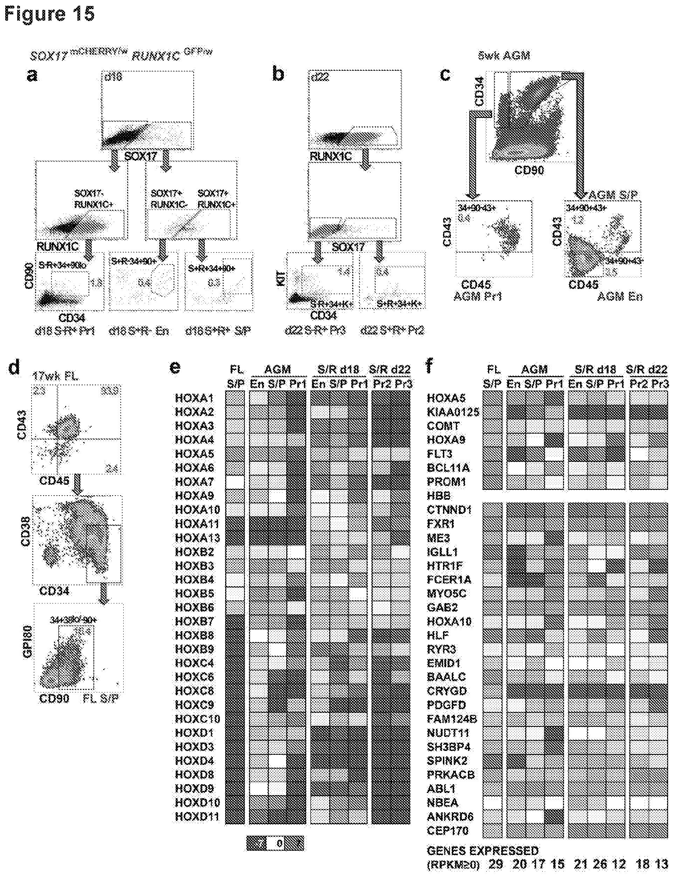

**Figure 1****. Characterization of *RUNX1C*^{GFP/w} and *RUNX1C*^{GFP/GFP} hESCs. (a)** Schematic of the wild type and the targeted *RUNX1C* allele with GFP sequences inserted into exon 1. The distal (D) and proximal (P) promoters are shown. Non-coding regions of exons are in white, coding in black and the *runt* domain in red. See also Figure 2. **(b)** Brightfield (BF) and overlaid fluorescence images comparing haematopoietic differentiation of d22 *RUNXIC*^{GFP/w} and *RUNXIC*^{GFP/GFP} cultures. Scale bar, 100µM. **(c)** Summary of flow cytometric analyses of differentiating *RUNX1C*^{GFP/W} EBs, correlating the percentage of cells expressing RUNX1C⁺ (R1C) with the expression of mesodermal (PDGFRα) and haematoendothelial cell surface markers (TIE2, CD34 and CD45). **(d)** Time course analysis showing the percentage of RUNX1C⁺ (R1C) cells expressing each indicated marker. Data taken from experiments in (c). Data in (c, d) is the mean±SEM for 4 experiments. **(e, f)** Bar plots showing distribution of myeloid and erythroid colony forming cells in *RUNXIC*^{GFP/w} and *RUNXIC*^{GFP/GFP} cultures. Data is the mean±SEM of 3 independent experiments. * p <0.05, repeated measures ANOVA comparing all groups to GFP⁺CD34^{lo} population with Tukey's Multiple Comparison Test. **(g)** Bar plot showing a similar distribution of colony morphology between *RUNX1C* heterozygous and nullizygous lines. Data represents mean±SEM of three independent experiments. **(h)** Photomicrographs illustrating examples of erythroid (ERY), granulocytic (GRAN), granulocyte-macrophage (GM) and macrophage or mast cell (MAC/MAST) colony types. Scale bar, 200µM. **(i)** Cytocentrifuge preparations of haematopoietic colonies stained with May-Grunwald-Giemsa stain illustrating the morphology of cells derived from different colony types. Examples of nucleated erythroid cells (ERY), granulocytes (GRAN), mast cells (MAST), mixed macrophage and granulocyte (GM) and macrophage (MAC) colonies are shown. Scale bar, 20µM. (j) Short-term bone marrow homing of d14 *RUNX1C*^{GFP/w} and *RUNX1C*^{GFP/GFP} EBs sorted on GFP (G) and CD34 (34) expression. Data for 12 mice from 4 independent experiments are shown, with the mean±SEM marked. Only the GFP⁺CD34⁺ population derived from *RUNX1C*^{GFP/w} cells displayed significant short-term homing. **, p<0.01, one way ANOVA comparing all groups to GFP⁻CD34⁻ from *RUNX1C*^{GFP/w} with Holm-Sidak's multiple comparison test.
**Figure 2****. Generation of RUNX and SOX reporter lines.**
   **(a)** Schematic representation of the *RUNX1* locus, the targeting vector and the targeted allele with GFP sequences inserted into exon 1(e1). The distal (D) and proximal (P) promoters are shown. Exons (e) are numbered with non-coding exons boxed in white, coding exons in black and exons encoding the runt homology domain in red. e7A and e7B are alternately spliced exons that give rise to *RUNX1A* and *RUNX1B* transcripts. RES indicates a cassette encoding either G418 (Neo) or hygromycin resistance flanked by loxP sites (black triangles). The positions of primers used to identify homologously integrated alleles are shown (p1-p7), with the sizes of PCR products indicated. p3 identifies the hygromycin resistance gene whilst p7 is specific for the G418 resistance gene **(b)** A representative example of the PCR screen to detect homologous integration of the left and right homology arms with the hygromycin cassette containing vector. **'+'** refers to a clone where the targeting construct was homologously integrated into the *RUNX1* locus, and '-' indicates a randomly integrated clone. The combinations of p1/p2 and p3/p4 identify correctly integrated left and right homology arms of the vector. p1/p5 identifies sequences of a wild type allele and p3/p6 identifies vector sequences present even in random integrations. Sequences for each primer are indicated in Table 1. **(c)** Homologous integration into the *RUNX1* locus was further verified by cloning and sequencing the integration sites. An example using primers p1/p2 and p3/p4 is shown. Junction sequences from the genomic locus are shown in lower case. **(d)** *RUNX1c*^{*GFP*/*w*} EBs harvested on day 13, 14 or 17 were flow-sorted on the basis of GFP and CD34 expression. cDNA synthesized from the sorted samples was analysed by RT-PCR using primers that detected both *RUNX1B* and *RUNX1C (RUNX1B*/*C)* or *RUNX1C* alone. Note that *RUNX1B*/*C* primers revealed expression in GFP⁻CD34⁺ cells, consistent with expression of the *RUNX1B* isoform in a subset of CD34⁺ endothelial cells. Gene expression is shown relative to GAPDH. UN, unfractionated cells. **(e)** Schematic outlining the strategy for generation of the *RUNX1C* ^{GFP/GFP} cell line by re-targeting the wild type *RUNX1C* allele with the hygromycin targeting vector. **(f)** Schematic outlining the PCR based screening strategy to identify doubly targeted cell lines, prior to excision of the targeting cassette. **(g)** PCR analysis using primers shown in (e) to identify GFPHygro, GFP and wild type (w) alleles. Clone 180 depicts an example where the GFPHygro targeting vector has replaced the previous GFP allele, leaving one wild type allele, clone 22 represents depicts one previously targeted GFP allele and one wild type allele and clone 253 represents a clone in which GFPHygro has correctly integrated into the wild type allele resulting in both *RUNX1C* alleles being targeted. **(h)** PCR analysis of sorted fractions of *RUNX1C* ^{GFP/w} and *RUNX1C* ^{GFP/GFP} differentiated cells confirming the absence of *RUNX1C* transcripts from the *RUNX1C* ^{GFP/GFP} line without disruption of the *RUNX1B* transcripts. Cells were sorted at day 14 based on GFP (G) and CD34 expression. UN, unfractionated cells. **(i)** Schematic representation of the *SOX17* locus, the targeting vector and the targeted allele with mCHERRY sequences inserted into exon 1(e1). The loxP flanked Neo selectable marker was excised with CRE recombinase. **(j)** Intracellular flow cytometry profiles of *SOX17*^{mCHERRY/w} hESC differentiated for 5d to endoderm, showing specific staining of mCHERRY⁺ cells with FITC-conjugated SOX17 antibody.

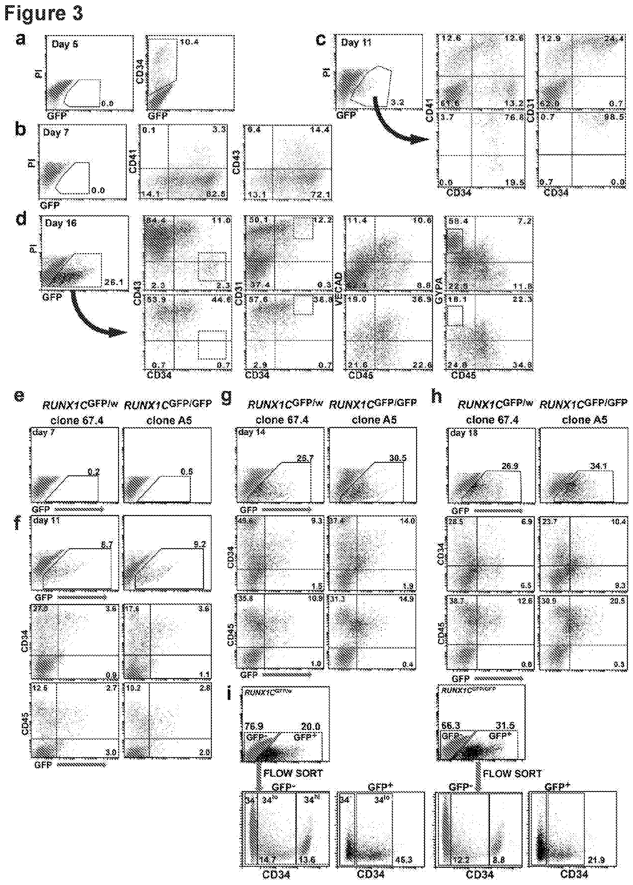

**Figure 3****. Analysis of cell surface markers expressed on differentiating RUNX1C^{GFP/w} and *RUNX1C*^{GFP/GFP} cells.**
   **(a - d)** Expression of CD34, CD43 and CD41 precedes GFP expression in *RUNXIC*^{GFP/w} EBs. Representative flow cytometry analyses of differentiating *RUNXIC*^{GFP/w} EBs indicating that expression of CD34 at day 5 **(a)** followed by CD41 and CD43 at day 7 **(b)** antedated GFP expression. Correlation of GFP expression with haematopoietic cell surface markers at day 11 **(c)** and day 16 **(d)** revealed that all GFP⁺ cells initially co-expressed CD34, CD31, and most expressed CD41. At day 16, GFP expression remained confined to haematopoietic cells expressing the indicated cell surface markers. Red boxed areas represent CD34⁺CD31⁺CD41⁻CD43⁻CD33⁻CD45⁻ endothelial cells and the blue boxes mark GYPA⁺ erythroid cells, both of which were GFP⁻. A high proportion of d16 GFP⁺ cells co-expressed VECAD and CD45, a combination of surface markers found in mouse haematopoietic stem/progenitor cells. **(e - h)** Flow cytometry analysis of differentiation timecourse comparing *RUNXIC*^{GFP/w} heterozygous cells (clone 67.4) with *RUNX1C*^{GFP/GFP} nullizygous clone A5 at **(e)** day 7, **(f)** day 11, **(g)** day 14 and **(h)** day 18. The percentage of GFP⁺ cells and CD34 and CD45 expression patterns were similar for all clones. **(i)** Strategy for flow cytometry sorting of d14 differentiating *RUNXIC*^{GFP/w} and *RUNXIC*^{GFP/GFP} cultures based on GFP and CD34 expression.

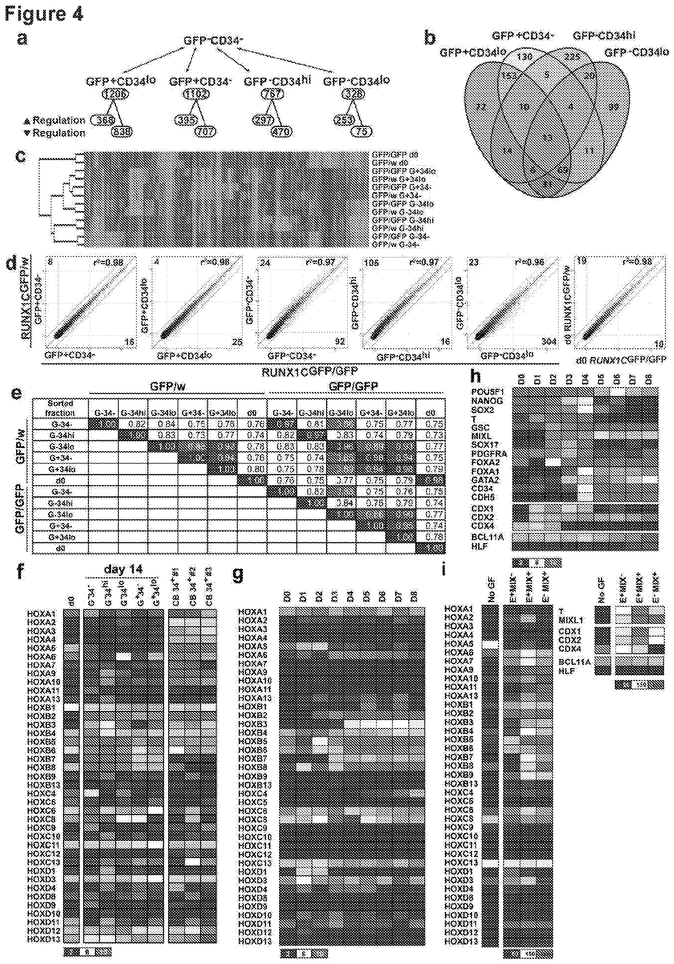

**Figure 4****. Comparison of gene expression between *RUNX1C*^{GFP/w} and *RUNX1C*^{GFP/GFP} EBs**.
   **(a)** Differentially expressed probe sets between the indicated fractions and GFP⁻CD34⁻ cells for samples including both d14 *RUNX1C*^{GFP/w} and *RUNX1C*^{GFP/GFP} EBs. **(b)** Venn diagram showing overlap between probe sets up-regulated in each cell fraction in (a). **(c)** Hierarchical cluster analysis of the fractions sorted on the basis of CD34 and GFP expression, as well as undifferentiated hESCs isolated from the heterozygote *RUNX1C*^{GFP/w} 67.4 line and the homozygous null *RUNX1C*^{GFP/GFP} A5 line, based on the top 25% of probes with the greatest variance. **(d)** Scatter plot representations of the logarithmic signal intensities of the pairwise comparisons between the CD34 and GFP fractions as well as undifferentiated hESCs from the heterozygous and homozygous null lines. Numbers in the top left and bottom right corners indicate the number of probes that are outside of the 2-fold cut-off (indicated by red lines). The correlation co-efficient for each differentiated population is similar to the correlation co-efficient for the undifferentiated cells, indicating the similarity of the differentiated progeny. **(e)** Table indicating the Pearson correlation co-efficient for each of the crosswise comparisons. Note the high correlation coefficients (r²≥0.96) between the equivalent fractions from heterogygous and homozygous *RUNX1C* cell lines and between the *RUNX1C* expressing populations (G⁺34⁻ and G⁺34^{lo}; r2≥0.94). **(f)** Heatmap showing HOX gene expression in sorted d14 *RUNX1C*^{GFP/v} EBs and three samples of CB 34⁺ cells. **(g)** Heatmap depicting expression of *HOX* cluster genes during differentiation of *RUNX1C*^{GFP/w} cells for 8 days. Note that the probes for *HOXA7* and *HOXB9* function poorly in this array, based on comparisons by RT-PCR and RNA-seq, Figure 15e. **(h)** Heatmap depicting expression of key pluripotency *(POU5F1, NANOG, SOX2*), mesendoderm *(T, GSC, MIXL, SOX17, PDGFRA, FOXA2, FOXA1)* and hematopoietic genes *(GATA2, CD34, CDH5),* the homeobox regulatory *CDX* genes, and transcription factors *BCL11A* and *HLF*, expressed in definitive haematopoietic lineages. Heatmap legend represents log₂ transformed values of signal intensity applied to (g-i). **(i)** Heatmap showing *HOX* gene expression in d4 *MIXL1*^{GFP/w} hESC differentiated in BMP4 and sorted into undifferentiated (ECADHERIN⁺MIXL1⁻ [E⁺MIXL⁻]), primitive streak-like (E⁺MIXL⁺) and nascent mesoderm (E⁻MIXL⁺). Also shown is the expression of selected mesendoderm genes *(T, MIXL1), CDX* genes, *BCL11A* and *HLF.* As a comparison, gene expression in EBs differentiated in the absence of growth factors is shown (No GF). In this case, EBs adopt an early neural fate and *HOX* genes are not expressed.

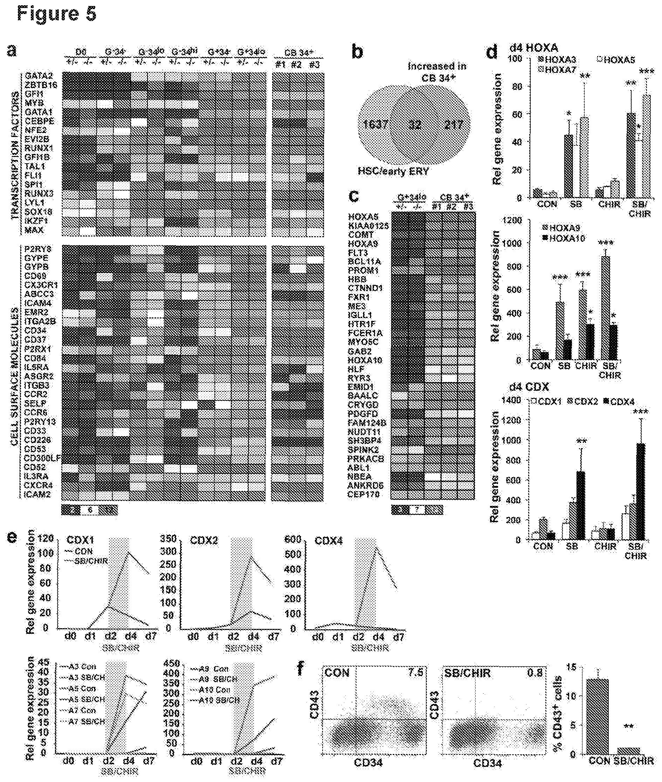

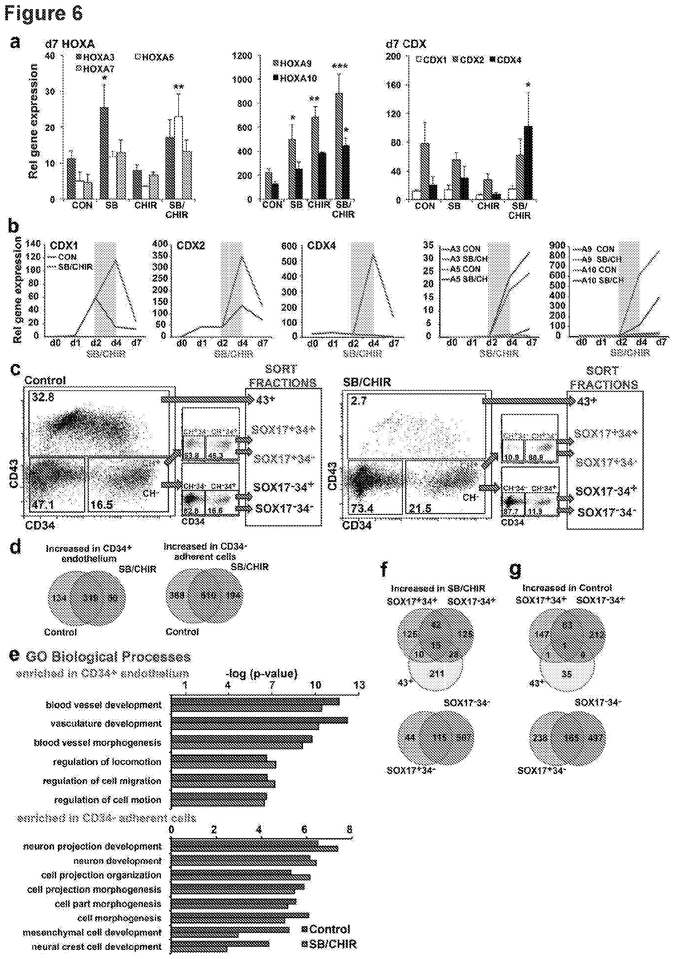

**Figure 5****. Differential *HOXA* gene expression between hESC-derived haematopoietic cells and cord blood modulated by patterning with SB/CHIR. (a)** Heatmap showing haematopoietic transcription factors and cell surface molecules differentially expressed between GFP⁺CD34^{lo} and GFP⁻CD34⁻ cells. Expression of these genes in CD34⁺ cord blood (CB 34⁺) samples is shown. Scale reflects signal intensity as log₂. **(b)** Venn diagram showing overlap between 249 genes up-regulated in CB 34⁺ cells (compared with GFP⁺CD34^{lo} cells) and 1669 HSC genes. **(c)** Heat map showing gene expression of the 32 overlap genes identified in (b) in GFP⁺CD34^{lo} sorted samples from *RUNX1C* heterozygous(+/-) and null(-/-) lines and three samples of CB 34⁺ cells. **(d)** Relative expression by RT-PCR of *HOXA* and *CDX* genes at d4 of differentiation in cultures supplemented with SB431542 (SB), CHIR99021 (CHIR) and the combination of these agents (SB/CHIR). Data is the mean±SEM for 3-4 experiments. *, p<0.05; **, p<0.01; ***, p<0.001 compared to control using 2 way ANOVA with Holm-Sidak's multiple comparisons test. Data for the d7 timepoint is shown in Figure 6a. **(e)** Flow cytometry profiles demonstrating reduced CD43 in d7 SB/CHIR EBs compared to controls. Bar graph of CD43 expression, with data showing the mean±SEM for 4 experiments. **, p<0.01, using Student's t-test. **(f)** Time course correlating *CDX* and *HOXA* gene induction in control and SB/CHIR cultures. An independent *CDX* time course experiment is shown in Figure 6b.
**Figure 6****. HOXA expression in differentiating cultures and generation of RNA-Seq transcriptional profiling data.**
   **(a)** Relative expression by RT-PCR of *HOXA* and *CDX* genes at d7 of differentiation in cultures supplemented with SB431542 (SB), CHIR99021 (CHIR) and the combination of these agents (SB/CHIR). Data is the mean±SEM for 3-4 experiments. *, p<0.05; **, p<0.01; ***, p<0.001 compared to control using 2 way ANOVA with Holm-Sidak's multiple comparisons test. Data for the d4 timepoint is shown in Figure 5d. **(b)** Time course correlating *CDX* and *HOXA* gene induction in control and SB/CHIR cultures. An independent *CDX* time course experiment is shown in Figure 5f. **(c)** Gating strategy and representative FACS plots showing cell fractions sorted from Control and SB/CHIR treated cultures at day 10. Cells were sorted on the basis of CD43, CD34 and SOX17 (mCHERRY) to identify haematopoietic (CD43⁺), and *SOX17*⁺ and *SOX17*⁻ endothelial (abbreviated to SOX17⁺34⁺ and SOX17⁻34⁺) and adherent cells (abbreviated to SOX17⁺34⁻ and SOX17⁻34⁻). **(d)** Venn diagrams showing overlap in genes whose expression was selectively increased in CD34⁺ endothelium or CD34⁻ adherent cells between Control and SB/CHIR cultures. **(e)** Gene ontology (GO) indicating that similar biological processes were enriched in control and SB/CHIR cultures in the CD34⁺ and CD34⁻ fractions from panel (d). **(f, g)** Venn diagrams showing overlap in (f) SB/CHIR and (g) Control culture differentially up-regulated genes between CD34⁺ and CD34⁻ fractions. Genes up-regulated in CD43⁺ haematopoietic cells clustered more closely to the CD34⁺ endothelial cells.

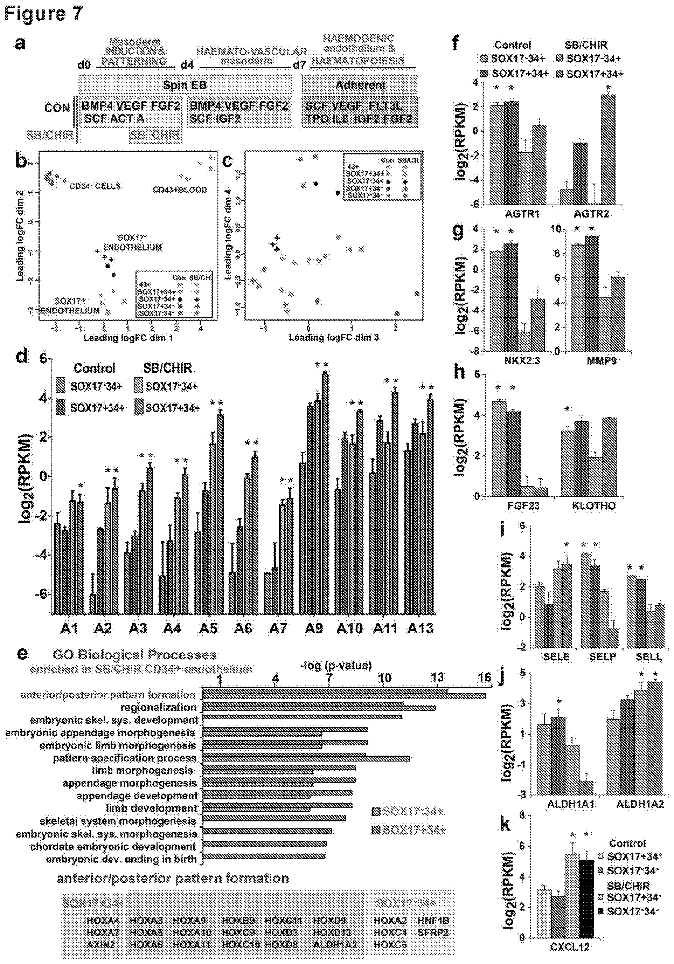

**Figure 7****. Transcriptional profiling reveals differential expression of *HOXA* and endothelial identity genes in SB/CHIR cultures.**
   **(a)** Control and SB/CHIR differentiation protocols. **(b, c)** Multidimensional scaling plots of RNA-Seq data from d10 control (Con) and SB/CHIR (SB/CH) cultures sorted into endothelium, blood and CD34⁻ adherent cell fractions demonstrating the clustering of sorted fractions by immunophenotype (b) and by culture condition (c). **(d)** Log₂ scale bar plot showing the expression by RNA-Seq of *HOXA* genes in SOX17⁺34⁺ and SOX17⁻34⁺ endothelial cells in control and SB/CHIR cultures (n=3, mean±SEM). Expression in SB/CHIR cultures was higher for asterisked (*) genes (p<0.05). See also *HOXA* expression in other sort fractions in Figure 12f, g. **(e)** Gene ontology (GO) biological processes enriched in genes more highly expressed in SB/CHIR cultures compared to control in the CD34⁺ endothelial fractions. The genes comprising the top category are shown. **(f - j)** Log₂ scale bar plots showing the expression of selected differentially expressed endothelial genes in SOX17⁺34⁺ and SOX17⁻34⁺ endothelium from SB/CHIR and control cultures. **(k)** Log₂ scale bar plot showing expression of *CXCL12* in SOX17⁺34⁻ and SOX17⁻34⁻ adherent cells between SB/CHIR and control cultures. Asterisks (*) indicate significance (p<0.05) compared to the alternate culture condition (n=3, mean ± SEM).

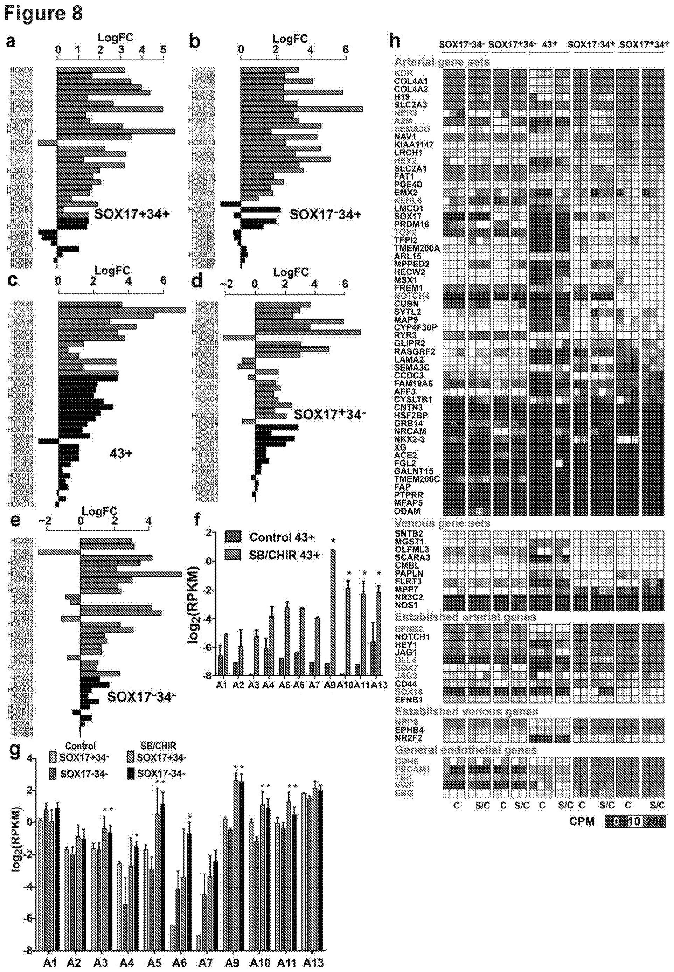

**Figure 8****. Differential HOX gene expression and expression of vascular genes in sorted fractions from control and SB/CHIR cultures.**
   **(a-e)** Bar plots showing fold change (log₂) in *HOX* gene expression in SB/CHIR versus control cultures for each sorted fraction. Red fill indicates the difference was statistically significant (p<0.05). Bars are arranged in order of increasing p value from top to bottom. **(f, g)** Bar plot showing the expression by RNA-Seq of *HOXA* genes in **(f)** CD43⁺ blood cells and **(g)** *SOX17*⁺ and *SOX17⁻* CD34⁻ adherent cells in control and SB/CHIR cultures (n=3, mean±SEM). Expression in SB/CHIR cultures was higher for asterisked (*) genes (p<0.05). **(h)** Expression of genes by RNA-Seq analysis discriminating arterial and venous gene signatures in umbilical artery and vein endothelium as reported by Aranguren et al. Blood 122, 3982-3992 (2013). Genes shown in red type were selectively expressed in CD34⁺ sorted fractions as shown in Figure 6d. The sorted cell fraction is indicated at the top of each pair of columns and the Control (C) or SB/CHIR (S/C) culture condition at the bottom.

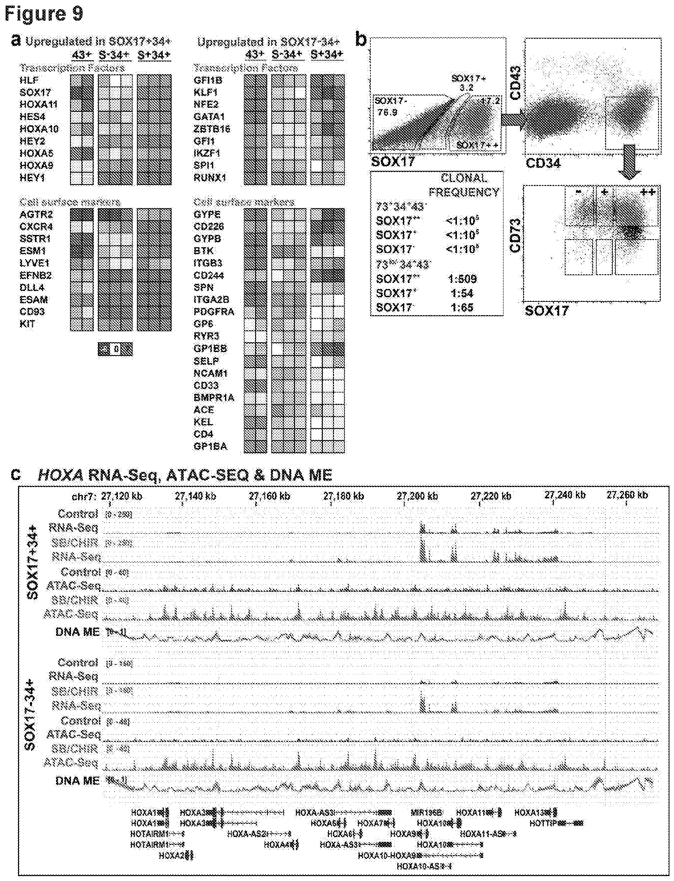

**Figure 9****. Characterization of SB/CHIR endothelium reveals differences in gene expression and haemogenic function.**
   **(a)** Heatmap showing transcription factors and cell surface markers differentially expressed between d10 SOX17⁺ and SOX17⁻ endothelial cells in SB/CHIR cultures. Expression in sorted haematopoietic cells (43⁺) is shown to demonstrate that genes increased in SOX17⁻ endothelium include many haematopoietic regulators and surface markers. **(b)** Sorting strategy to determine the clonal frequency of haemogenic capacity of SB/CHIR endothelium, showing sequential gating on SOX17, CD34 and CD43, and CD73. SOX17 expression was stratified as bright (++), dull (+) and negative (-). Most CD73^{lo/-} cells were also DLL4⁻ and CXCR4⁻. **(c)** RNA-Seq and DNA methylation (ME) array, and chromatin accessibility assessed by ATAC-Seq, comparing the *HOXA* cluster in control and SB/CHIR CD34⁺ endothelial fractions. In the DNA ME array, the blue and red lines represent the fraction of methylated probe at each locus in control and SB/CHIR cultures, respectively. The shading represents 95% confidence intervals for the DNA ME value. Scale represents counts per million for RNA-Seq and ATAC-Seq. DNA ME is scored for mean probe methylation from 0 (unmethylated) to 1 (methylated).

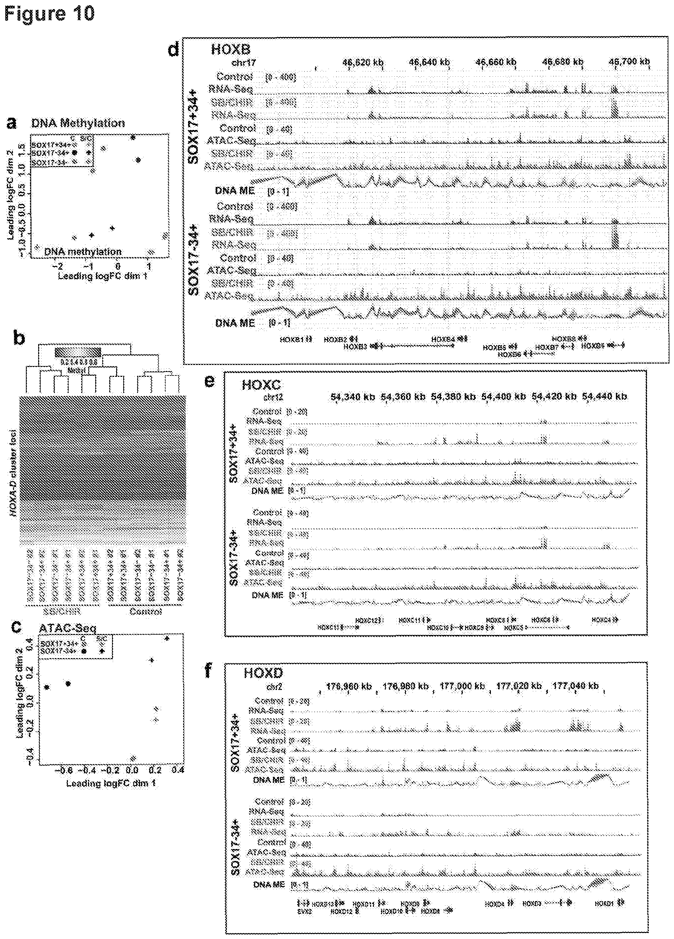

**Figure 10****. DNA methylation, RNA-Seq and ATAC-Seq analyses of HOX genes in d10 *SOX17*^{mCHERRY/w}*RUNX1C*^{GFP/w} cells.**
   **(a)** Multidimensional scaling plots of DNA methylation array data from d10 control (C) and SB/CHIR (S/C) cultures sorted into SOX17⁺ and SOX17⁻ endothelium, and SOX17⁻34⁻ adherent cell fractions demonstrating the clustering of sorted fractions by phenotype and by culture condition. The closer clustering of the control and SB/CHIR samples for the SOX17⁺CD34⁺ endothelium suggests more similar levels of methylation in these samples. **(b)** Heatmap of the 1002 methylation probes that encompassed the *HOXA-D* loci showing global hypomethylation in all samples. Hierarchical clustering indicates additional grouping of samples by treatment type. **(c)** Multidimensional scaling plots of ATAC-Seq data from d10 control (C) and SB/CHIR (S/C) cultures sorted into SOX17⁺ and SOX17⁻ endothelium, demonstrating the clustering of sorted fractions by phenotype and by culture condition. **(d - f)** RNA-Seq and DNA methylation (ME) array, and chromatin accessibility assessed by ATAC-Seq, comparing the (d) *HOXB,* (e) *HOXC,* and (f) *HOXD* clusters in control and SB/CHIR CD34⁺ endothelial fractions. In the DNA ME array, the blue and red lines represent the fraction of methylated probe at each locus in control and SB/CHIR cultures cells respectively.

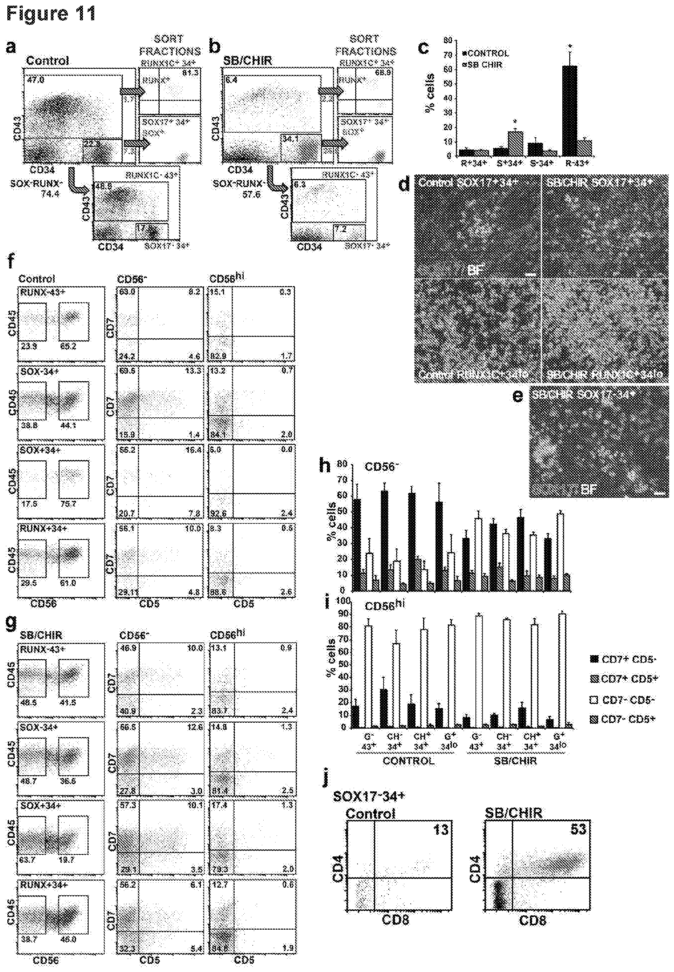

**Figure 11****. Analysis of T cell generation in *SOX17*^{mCHERRY/w}*RUNX1C*^{GFP/w} cells.**
   **(a, b)** Strategy used to sort *RUNXIC*^{GFP/w} cells differentiated for 7 days in spinner cultures under Control **(a)** and SB/CHIR **(b)** conditions into RUNX1C⁺34⁺, SOX17⁺34⁺, RUNX1C⁻43⁺ and SOX17⁻34⁺ fractions prior to co culture with OP9 DL4 stromal cells. **(c)** Bar plot comparing the frequency of cells in each fraction indicating that RUNX1C⁻43⁺ (R⁻43⁺) cells were less frequent in SB/CHIR cultures, consistent with suppression of primitive hematopoieisis, and that SOX17⁺34⁺ (S⁺34⁺) cells increased in response to SB/CHIR. Data represent mean±SEM from 4 experiments. *, p<0.05 Student's t test. **(d)** Day 7 SOX17⁺CD34⁺ and RUNX1C⁺CD34^{lo} populations from control and SB/CHIR cultures after 5d on OP9 DL4 showing emerging haematopoietic cells. Scale bar, 50µM **(e)** Image of SOX17⁻CD34⁺ sorted cells from SB/CHIR cultures after 9 days on OP9 DL4 showing the generation of SOX17⁺ cells and emergence of haematopoietic cells. Scale bar, 50µM. **(f, g)** Representative flow cytometry profiles of control (f) and SB/CHIR (g) d7 sorted fractions after 21 days co-culture on OP9 DL4. Live cells were gated for CD45, then for the NK cell marker CD56 (left panels). T cell progenitors (CD45⁺CD56⁻CD7⁺CD5⁺) are shown in red and NK cells (CD45⁺CD56^{hi}CD7^{±}) are shown in blue. Whilst T cells and NK cells were generated from all sorted fractions, the yield appeared greater from the SB/CHIR treated cultures. **(h, i)** Bar plot showing the percentages of cells falling into the CD7 and CD5 quadrants shown in (e, f) for the CD56⁻ T cell progenitors (h) and the CD56^{hi} NK cells (i). **(j)** Generation of CD4⁺CD8⁺ T cells following 27 days co-culture on OP9 DL4 of SOX17-CD34+ cells from SB/CHIR treated cultures.
**Figure 12****. Development of AGM-like SOX17⁺ vascular structures in SB/CHIR cultures.**
   **(a)** Day 11 EBs from control and SB/CHIR cultures showing developing SOX17⁺ vascular structures within and growing out from the EB, contrasting with the lack of identifiable vessels and the early production of blood cells in the control EBs. Boxed areas in left panels are magnified on the right. Scale Bar, 100µM. **(b)** Tiled images of d15 EBs from control and SB/CHIR cultures showing multiple SOX17⁺ vascular structures emanating from the SB/CHIR EBs. Arrows indicate two of the vascular structures. Scale bar, 500µM. **(c-f)** Images of a d20 SB/CHIR EB in which some SOX17⁺ vascular segments enclose RUNX1C⁺ haematopoietic cells. Panels (d,e,f) show higher power views of boxed areas **(d',e',f').** Scale bars, (c) 300µM, (d-f) 100µM. **(g)** Image of boxed area **(g')** showing RUNX1C⁺ cells enclosed by SOX17⁺ endothelium **(h)** Image of boxed area **(h')** showing RUNX1C⁺ and RUNX1C⁻ cells within a SOX17⁺ vascular structure. Scale bar, (g,h) 50µM.**(i)** increased viability of haematopoietic cells in d23-d29 cultures of SB/CHIR cells (n=6, mean±SEM; *, p<0.05 Student's t test). **(j)** Brightfield and RUNX1C⁺ overlaid images of control and SB/CHIR cultures at d18 showing dying cells (arrows) in the control cultures. Scale bar, 50µM.**(k)** Flow cytometry plots showing increased frequency of haematopoietic genes expressing the indicated stem cell markers at d23 in SB/CHIR cultures. **(1)** Bar plots showing the increased percentage of haematopoietic cells expressing CD34, GPI80, FCER1A and ACE in d23-29 SB/CHIR cultures (n=4, mean±SEM; *, p<0.05 Student's t test).

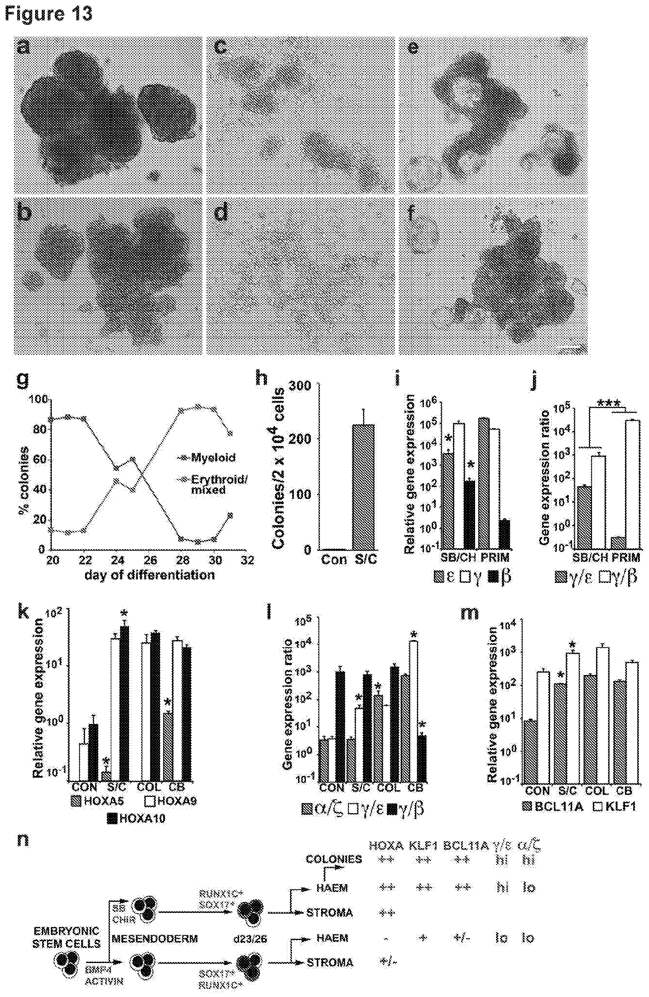

**Figure 13****. Generation of foetal stage erythroid cells from *HOXA*⁺ cultures.**
   **(a-f)** Multifocal erythroid burst colonies (a,b), mixed erythroid myeloid colony (c), myeloid colony (d), and erythroid colonies apposed to stromal cysts in MATRIGEL^{™} supplemented methycellulose (e, f). Panel (a) was a colony from a d23 culture, the remaining panels came from d31 colonies. Scale bar, 100µM. **(g)** Proportion of myeloid and erythroid/mixed colony types in methylcellulose in SB/CHIR treated cultures assayed at the day of differentiation indicated. Data represent results from 7 independent experiments with two of the experiments assayed at both earlier and later time points. **(h)** Bar plot showing the frequency of CFCs in control and SB/CHIR (S/C) cultures. Data is the mean±SEM for 5 experiments (d23-33), p<0.01 for S/C versus control using Student's t test. **(i)** Log scale bar graph showing the relative gene expression of β globin genes in SB/CHIR colonies compared with primitive erythroid colonies (PRIM). Data is the mean±SEM for 6 SB/CHIR and 14 primitive erythroid samples. *, p<0.005 using Student's t test. **(j)** Log scale bar graph showing the ratio of γ/ε and γ/β globin expression in the same data set as **(i).** ***, p<0.001 using Student's t test. **(k)** Log scale bar graph of the relative gene expression of *HOXA* genes in control (CON), SB/CHIR cultures (S/C), erythroid colonies (COL) and d10 differentiated cord blood CD34⁺ cells (CB). Data is the mean±SEM for 3 experiments. *, p<0.05 versus CON using Student's t test **(l)** Log scale bar graph of the ratio of γ/ζ, γ/ε and γ/β globin chain expression in the same experiments as **(k).** *, p<0.01 for γ/ε□□□□S/C versus CON and CB versus S/C, for α/ζ in COL versus S/C and for γβ in CB versus CON, using Student's t test. **(m)** Log scale bar graph of the relative gene expression of *BCL11A* and *KLF1* in the same experiments as **(k)** *, p<0.05 versus CON using Student's t test. **(n)** Summary of the experiments in panels (k-m), showing that *HOXA* expression induced by SB/CHIR translates into persistent clonogenic cells in 3-4 week cultures. These late colonies have increased *BCL11A* and *KLF1* expression and downregulated embryonic *EPSILON* and *ZETA* globin genes, similar to an early foetal liver stage of development.

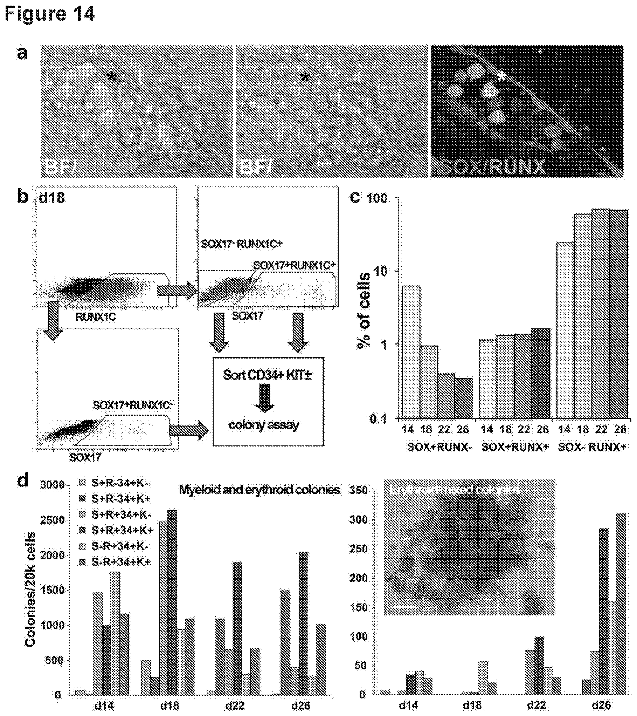

**Figure 14****. Emergence of SOX17 and RUNX1C expressing stem/progenitors.**
   **(a)** Confocal images of a d18 SB/CHIR culture showing a vessel containing a cell co-expressing SOX17 and RUNX1C. **(b)** Gating strategy showing the isolation of SOX⁺RUNX⁻, SOX⁺RUNX⁺ and SOX⁻RUNX⁺ populations that were further stratified by CD34 and KIT expression. **(c)** Bar plot of the percentage of cells in each indicated fraction across a developmental time course from d14 to d26 of differentiation, showing reciprocal changes in proportions of SOX⁺RUNX⁻ and SOX⁻RUNX⁺ populations. **(d)** Clonogenic frequency at each time point of populations sorted on SOX17 (S), RUNX1C (R), CD34 (34), and KIT (K). The left panel shows the total number of myeloid and erythroid colonies and the right panel shows only the erythroid-containing colony frequency. The inset shows a multifocal mixed erythroid/myeloid colony from a d26 S⁺R⁺34⁺K⁺ culture. Scale bar, 100µM. These data represent a series of 4 independent experiments.
**Figure 15****. RNA-Seq Analysis of human FL, AGM and sorted SOX/RUNX d18 and d22 samples.**
   Sorting strategy for generating **(a)** *SOX17*^{mCHERRY/w}*RUNX1C*^{GFP/w} d18 endothelial (S⁺R⁻34⁺90⁺) (d18 S⁺R⁻ En), stem/progenitor (S⁺R⁺34⁺90⁺) (d18 S⁺R⁺S/P) and progenitor (S⁻R⁺34⁺90^{lo}) (d18 S⁻R⁺ Pr1) populations, **(b)** *SOX17*^{mCHERRY/w}*RUNX1C*^{GFP/w} d22 progenitor (S⁺R⁺34⁺K⁺) (d22 S⁺R⁺ Pr2) and (S⁻R⁺34⁺K⁺) (d22 S⁻R⁺Pr3) populations, **(c)** 5 week AGM endothelium (34⁺90⁺43⁻) (En), cells transiting from haemogenic endothelium to haematopoietic stem/progenitors (34⁺90⁺43⁺) (S/P) and committed progenitor cell (34⁺90⁻43⁺) (Pr1) populations and **(d)** 17 week foetal liver (FL) sample (CD43⁺CD45⁺) enriched for haematopoietic stem/progenitor cells (34⁺38^{l0/-}90⁺) (S/P). For panels **(a-d),** the percentage of live cells sorted for each fraction is shown in red type. The RUNX1C⁺ fractions in panels (a, b) also expressed CD43 or CD45. **(e)** Expression of *HOX* genes by RNA-Seq in FL, AGM, and d18 and d22 SOX/RUNX fractions sorted as shown in panels **(a-d). (f)** Expression of the list of 32 genes differentially expressed between cord blood and d14 RUNX1C⁺34⁺ cells (see Figure 5c) by RNA-Seq in FL, AGM, and d18 and d22 SOX/RUNX fractions sorted as shown in panels **(a-d).** HBB was not detected in any sample. The number of genes expressed at ≥ 0 RPKM in each sort fraction is indicated. Scale is in log₂ RPKM.

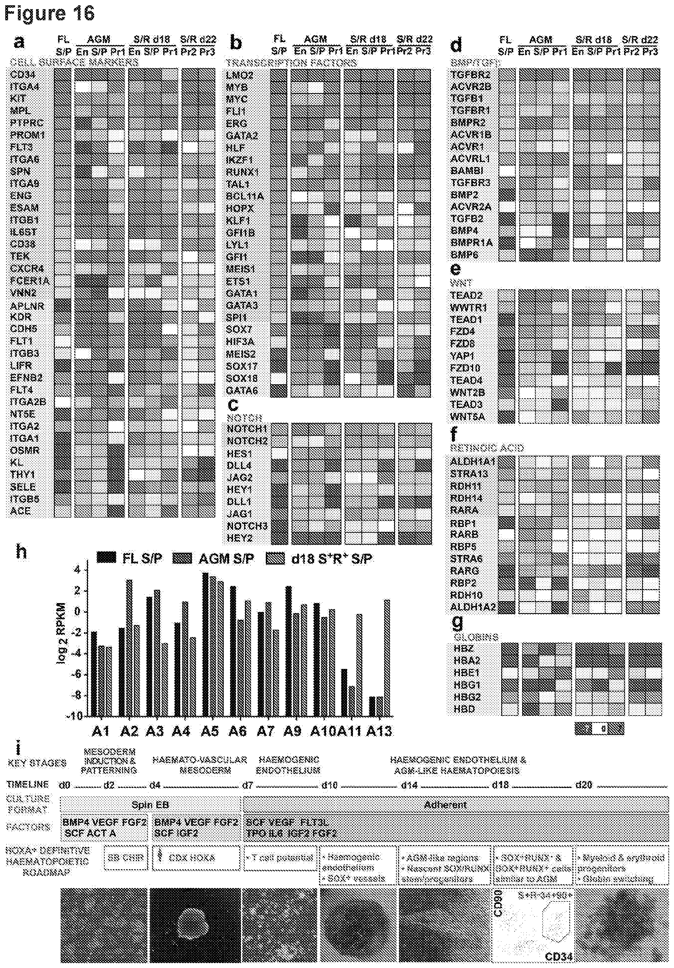

**Figure 16****. Transcriptional similarity between SOX/RUNX expressing cells and human AGM.**
   RNA-seq profiling comparing human foetal liver (FL), human AGM and SOX/RUNX (S/R) populations sorted at d18 and d22. 'S/P' refers to populations with a stem/progenitor phenotype, 'En' marks endothelium and 'Pr1-3' refer to committed progenitor populations with an increasingly mature phenotype. See text for further details. Heatmaps show **(a)** cell surface markers, **(b)** transcription factors, **(c)** NOTCH, **(d)** BMP/TGFβ **(e)** WNT, **(f)** retinoic acid signaling pathway genes and **(g)** globin genes. Red shaded gene groups are expressed in FL S/P cells, and blue shaded genes are selectively expressed in the AGM S/P fraction. Scale is in log₂ RPKM. **(h)** Bar graph showing expression of HOXA genes in FL, AGM and d18 S⁺R⁺ S/P populations. These data represent the results of a single flow sorting experiment. **(i)** Summary of the developmental stages, culture format, growth factors and the haematopoietic differentiation pathway during the transition from hPSC to AGM-like stem/progenitor cells. Thumbnail images illustrate the developmental stages.

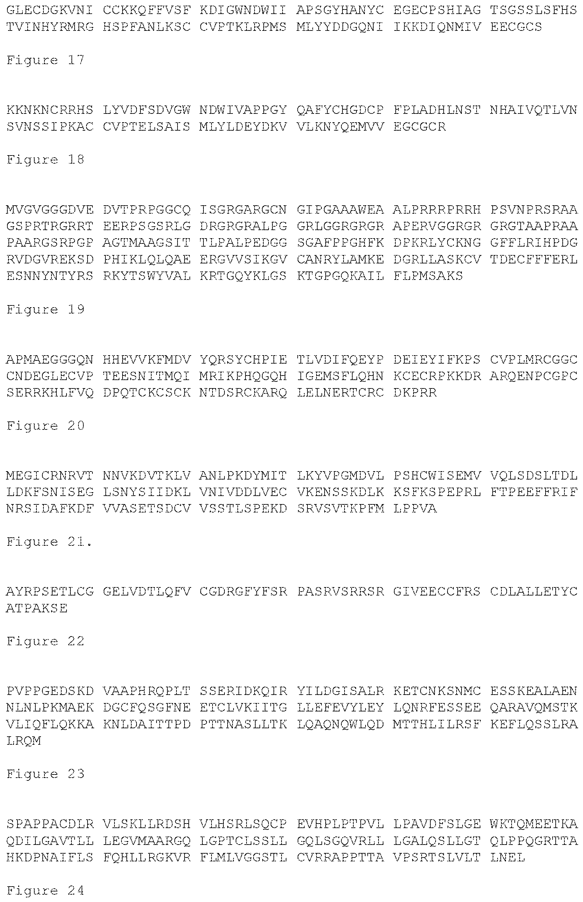

**Figure 17** is an amino acid sequence (SEQ ID NO: 1) representing a polypeptide that as a homodimer is one example of human ACTIVIN A.
**Figure 18** is an amino acid sequence (SEQ ID NO: 2) representing a polypeptide that as a homodimer is one example of human BMP4. SEQ ID NO: 2 corresponds to amino acids 303 to 408 of an amino acid sequence of a full length BMP4 precursor.
**Figure 19** is an amino acid sequence (SEQ ID NO: 3) representing a polypeptide that is one example of human FGF2.
**Figure 20** is an amino acid sequence (SEQ ID NO: 4) representing a polypeptide that as a homodimer is one example of human vascular endothelial growth factor (VEGF), also known as VEGF-A.
**Figure 21** is an amino acid sequence (SEQ ID NO: 5) representing a polypeptide that is one example of human stem cell factor (SCF), corresponding to the secreted soluble form of SCF.
**Figure 22** is an amino acid sequence (SEQ ID NO: 6) representing a polypeptide that is one example of human insulin-like growth factor 2 (IGF2).
**Figure 23** is an amino acid sequence (SEQ ID NO: 7) representing a polypeptide that is one example of human interleukin 6 (IL-6).
**Figure 24** is an amino acid sequence (SEQ ID NO: 8) representing a 174 amino acid polypeptide that is one example of human thrombopoietin (TPO) comprising the erythropoietin-like domain of the full length TPO protein.
**Figure 25** is an amino acid sequence (SEQ ID NO: 9) representing a polypeptide that is one example of human FLT3 receptor ligand (FLT3L).
**Figure 26** is an amino acid sequence (SEQ ID NO: 10) representing a polypeptide that is one example of human interleukin 3 (IL-3).
**Figure 27** is an amino acid sequence (SEQ ID NO: 11) representing a polypeptide that is one example of human erythropoietin (EPO).
**Figure 28** is an amino acid sequence (SEQ ID NO: 12) representing a polypeptide that is one example of human granulocyte-macrophage colony-stimulating factor (GM-CSF).
**Figure 29** is a bar graph showing expression of 'anterior' genes HOXA2, HOXA3, HOXA5 and HOXA7, and 'posterior' genes HOXA9 and HOXA10 in hPSCs differentiated in medium supplemented with CHIR99021, BMP4, VEGF, SCF, ACTIVIN A and FGF2 (denoted CH BVSAF) from d0-4, or with an additional pulse of SB431542 and CHIR99021 (denoted SB CHIR) from d2-4 in the absence (denoted 0) or presence of the retinoic acid analogue EC23 at 10⁻⁵ M or 10⁻⁷ M concentrations from d2-4 (denoted 10-5 and 10-7, respectively).

### DETAILED DESCRIPTION

Repopulating HSCs have an origin distinct from the yolk sac, and are first located in the aorta-gonad-mesonephros (AGM) and placenta. Cell clusters budding from the ventral aortic endothelium in the AGM harbour the earliest HSCs and express the transcription factor *RUNX1*, without which clusters do not form and foetal liver haematopoiesis fails.

Three isoforms of *RUNX1 (RUNX1A-C)* are transcribed from two promoters in humans and mice. In mouse embryos, *Runx1b* marks haemogenic endothelium, whilst *Runx1c* is expressed in haematopoietic progenitor cells and is the dominant isoform in foetal liver haematopoietic cells. *Runx1b* is required in AGM endothelium for definitive haematopoiesis and *Runx1b* heterozygous mice display defects in HSC and progenitor generation. Conversely, loss of both *Runx1c* alleles only modestly impacts haematopoiesis and a phenotype for haploinsufficient *Runx1c* has not been reported.

*SOX17* is a second critical transcription factor required for early definitive haematopoiesis in the mouse. In haematovascular development, *SOX17* is largely expressed in the arterial system of the embryo. It marks haemogenic endothelium in the AGM and is required for the generation of nascent HSCs from the AGM and for their maintenance in the foetal liver. As with *RUNX1C,* a haploinsufficient phenotype was not reported for *SOX17.*

Based on these key roles in haemogenic endothelium and in identifying haematopoietic progenitors, the inventors generated reporter cell lines for *SOX17* and/or *RUNX1C.* In initial studies, *RUNX1C* marked a subset of CD34⁺ cells highly enriched for haematopoietic progenitors that homed to the bone marrow, but did not engraft. Exploring molecular differences between PSC-derived and repopulation-competent cord blood (CB) CD34⁺ cells, the inventors revealed that the RUNX1C⁺CD34⁺ cells failed to express HOXA genes, indicating incorrect mesoderm patterning.

Consequently, the inventors have shown that simultaneous modulation of WNT and ACTIVIN signaling yields haematopoietic stem/progenitor cells with *HOXA* codes that more closely resemble those of cord blood. These cultures generate a network of aorta-like SOX17⁺ vessels, from which RUNX1C⁺ blood cells emerge - reminiscent of definitive haemogenesis within the AGM. Transcriptional profiles of nascent haematopoietic progenitors and corresponding cells sorted from human AGM displayed striking similarity in expression of cell surface receptors, signaling molecules and transcription factors. The inventors demonstrate that *HOXA* codes are an indicator of mesoderm patterning, thereby enabling generation of definitive haematopoietic lineages from PSCs.

Using *HOXA* gene expression of human CB progenitor cells as a guide, the inventors devised a differentiation protocol that generates AGM-like structures from hPSCs (Figure 16i). The development of this system was facilitated by a unique *SOX17*^{mCHERRY/w}*RUNX1C*^{GFP/w} reporter line and included an EB-based differentiation that retained cellular associations between endothelial, stromal and haematopoietic cells, contrasting with previous approaches that purified endothelial precursors prior to emergence of haematopoietic cells. Providing the combination of a WNT agonist and ACTIVIN antagonist, non-limiting examples of which are CHIR99021 and SB431542 (SB/CHIR) respectively, during a specific temporal window patterned nascent mesoderm to induce sustained *HOXA* gene expression, and switched differentiation from yolk sac-type primitive haematopoiesis towards definitive haematopoiesis. SB/CHIR treatment led to the development of haemogenic SOX17⁺ vascular structures and erythroid colony forming cells that displayed a globin profile switching from embryonic to foetal development. Most notably, transcriptional profiling of the most immature hPSC-derived haematopoietic stem/progenitor cells revealed very similar patterns of expression to human AGM, validating the fidelity of the differentiation protocol in generating definitive haematopoietic cells from hPSCs.

The inventors were struck by the low levels of *HOXA* expression in hPSC-derived vascular and haematopoietic progenitors derived under control culture conditions. Given that *HOXA5, HOXA7, HOXA9* and HOXA10 are expressed in HSCs, the inventors hypothesized that acquisition of a *HOXA* 'signature' during hPSC differentiation would underpin the capacity to eventually generate repopulating cells.

Because *HOX* genes are first expressed in the primitive streak, the inventors trialed factors that influenced mesodermal patterning. These experiments led to the finding that 2 days of culture with the combination of SB and CHIR up-regulated *CDX* and *HOXA* gene expression to a greater extent than either agent alone. The inventors found that 2 days was important, as no or minimal effect was observed at 24 hours. The inventors also observed that the synergistic effect of SB/CHIR was unexpected owing to the fundamental unpredictability in the art.

Chromatin accessibility was increased following SB/CHIR and, in the context of hypomethylation of the *HOX* clusters, indicated that a permissive environment was generated for transcription factor-mediated *HOXA* gene expression.

A striking consequence of SB/CHIR patterning was the emergence of SOX17⁺ vessels from day 10 (d10) of differentiation. The expression of aorta-associated genes and the appearance of haematopoietic cells from these vessels resembled AGM development. The restriction of haematopoiesis to discrete regions shows that blood cells only arose from a subset of the SOX17⁺34⁺ endothelium, consistent with reports that haemogenic endothelium displays a distinct phenotype. Indeed, these studies identified that the d10 SOX17⁺ haemogenic precursors were restricted to the small CD73-negative fraction and that the frequency was greatly increased in cells with lower *SOX17* expression.

In previous studies, deletion of *Hoxa9* or the whole *Hoxa* cluster in mesoderm led to significant haematopoietic defects. Additionally, knockdown of *HOXA5* or *HOXA7* in human foetal liver depleted cells with an HSC-like phenotype and engraftment ability, confirming the importance of *HOXA* genes in human definitive haematopoiesis. Despite this, studies aiming to generate HSCs through overexpression of *HOXA* genes have not been successful. A cocktail of genes, of which *HOXA9* was one component, was insufficient to generate long term engraftable cells from hPSC-derived CD34⁺ cells in one study. In a second study, enforced *HOXA9* expression in hESCs enhanced haematopoiesis but did not generate HSCs. In the *HOXA5* or *HOXA7* knockdown study above, it was concluded that even overexpression of multiple *HOXA* genes did not confer HSC properties on hPSC derived haematopoietic progenitors. These results show that the ability of *HOXA* genes to orchestrate a functional HSC phenotype requires the correct cellular context.

In contrast to control cultures, SB/CHIR cultures generated late progenitors with globin chain synthesis indicative of a switch to a foetal developmental stage. Erythroid differentiated hPSCs usually express *EPSILON* and *GAMMA* globin, although prolonged culture in human plasma or on stromal layers has yielded predominantly *GAMMA* or *GAMMA* and *BETA* globin expressing cells. A switch from *EPSILON* to *GAMMA* globin exclusively in SB/CHIR cultures, associated with the increased expression of *KLF1* and its target gene *BCL11A* was identified. In addition, *ZETA* globin down-regulation was observed later, in the progeny of colony forming cells from SB/CHIR cultures, indicating that distinct factors regulate silencing at the *ALPHA* globin locus (Figure 131, n). The residual *EPSILON* and *ZETA* expression in SB/CHIR erythroid colonies resembles early mouse foetal liver BFU-e that express some βH1 despite expression of *BCL11A* and *KLF1.*

Molecular analysis of human AGM has been limited to date, and the present study represents the first example of transcriptional profiling of sorted fractions from this tissue, with comparisons to human foetal liver and AGM-like populations derived from hPSCs. It has been shown elsewhere that AGM-derived HSCs exclusively derived from the ventral aorta, possessed a CD34⁺CDH5⁺CD45⁺KIT⁺THY1⁺ ENG⁺RUNX1⁺CD38^{-/lo}CD45RA⁻ phenotype. It is noteworthy that the AGM S/P and d18 S⁺R⁺ S/P fractions match this phenotype (Figure 16a, b), validating the human AGM sample and supporting the similarity of the hPSC generated cells to these primary cells. It has been shown that, in the mouse, haematopoietic stem/progenitor cells emerging from *Runx1*⁺ AGM endothelium expressed *Fli1, Kdr, Gata2, Tall, Lmo2, Lyl1, Erg, Tek* and *Itga2b* in both haemogenic endothelium and emerging haematopoietic progenitor cells, with the added expression of *Ptprc, Gfi1b, Runx1, Itgb3, Sfpi1, Gfi1* and *Myb* in the haematopoietic progenitors. These results show a high degree of concordance with this data set, with most of the genes expressed in the AGM S/P and d18 S⁺R⁺ S/P fractions (Figure 16a, b).

One laboratory transcriptionally profiled endothelial and haematopoietic populations from day 10.5-11 AGM. The laboratory identified transcription of the 'heptad' transcription factors *(Tall, Lyl1, Erg, Fli1, Runx1, Gata2* and *Lmo2),* in addition to *Cdh5, Tek, Esam, Kdr* and *Eng* in haemogenic endothelium and in the HSC compartment. They observed that many additional transcription factors influencing HSC development were selectively expressed in their HSC compartment. The majority of the genes they described were enriched in both the AGM and d18 S⁺R⁺ En and S/P fractions (Figure 16a, b).

These transcriptional data of human primary AGM and hPSC-derived samples show a high degree of concordance with existing human and mouse data, supporting the inventors' conclusion that the hPSC-derived samples represent definitive, human AGM haematopoietic cells, and that there is substantial conservation of definitive haematopoietic development between the species.

In summary, the *in vitro* differentiation of human PSCs that are 'switched' to definitive haematopoiesis through exposure to SB/CHIR, timed to overlap with the peak expression of primitive streak genes is described herein. Analysis of these cultures revealed the early and sustained expression of *HOXA* genes, followed by the development of SOX17⁺ vascular structures from which haematopoietic cells then emerged, resembling the developing mammalian AGM. This is the first report of a culture system that encompasses human haematopoietic development from mesoderm specification to an AGM-like stage and provides robust generation of transplantable human haematopoietic stem cells from pluripotent stem cells.

Unless defined otherwise in this specification, technical and scientific terms used herein have the same meaning as commonly understood by the person skilled in the art to which this invention belongs and by reference to published texts.

It is to be noted that the term "a" or "an" refers to one or more, for example, "a molecule," is understood to represent one or more molecules. As such, the terms "a" or "an", "one or more," and "at least one" may be used interchangeably herein.

In the claims which follow and in the description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

The term "about" as used herein contemplates a range of values for a given number of ±25% the magnitude of that number. In other embodiments, the term "about" contemplates a range of values for a given number of ±20%, ±15%, ±10%, or ±5% the magnitude of that number. For example, in one embodiment, "about 3 µM" indicates a value of 2.7 to 3.3 µM (i.e. 3 µM ±10%), and the like.

Similarly, while differentiation processes include ordered, sequential events, the timing of the events may be varied by at least 25%. For example, while a particular step may be disclosed in one embodiment as lasting one day, the event may last for more or less than one day. For example, "one day" may include a period of about 18 to about 30 hours. In other embodiments, periods of time may vary by ±20%, ±15%, ±10%, or ±5% of that period of time. Periods of time indicated that are multiple day periods may be multiples of "one day," such as, for example, two days may span a period of about 36 to about 60 hours, and the like. In another embodiment, time variation may be lessened, for example, where day 2 is 48±3 hours from day 0; day 4 is 96±3 hours from day 0, and day 5 is 120 hours±3 hours from day 0.

As used herein, a "WNT agonist" is a substance that mimics or increases WNT signaling. A WNT agonist is not to be restricted to a substance acting directly on WNT as the substance may act elsewhere in the WNT signaling pathway.

Non-limiting examples of WNT agonists according to embodiments of the invention include small molecules CHIR99021 (CAS 252917-06-9), a 2-amino-4,6-disubstituted pyrimidine, e.g. BML 284 (CAS 853220-52-7), SKL 2001 (CAS 909089-13-0), WAY 262611 (CAS 1123231-07-1), WAY 316606 (CAS 915759-45-4), SB 216763 (CAS 280744-09-4), IQ 1 (CAS 331001-62-8), QS 11 (CAS 944328-88-5), deoxycholic acid (CAS 83-44-3), BIO (CAS 667463-62-9), kenpaullone (CAS 142273-20-9), or a (hetero)arylpyrimidine. A WNT agonist may also be an agonist antibody or functional fragment thereof or an antibody-like polypeptide.

In one embodiment of the invention, the WNT agonist is CHIR99021 ((CHIR) CAS 252917-06-9), and the PSC is cultured in a medium comprising about 3 µM. In other embodiments, the medium may comprise about 2 µM, about 2.1 µM, about 2.2 µM, about 2.3 µM, about 2.4 µM, about 2.5 µM, about 2.6 µM, about 2.7 µM, about 2.8 µM, about 2.9 µM, about 3 µM, about 3.1 µM, about 3.2 µM, about 3.3 µM, about 3.4 µM, about 3.5 µM, about 3.6 µM, about 3.7 µM, about 3.8 µM, about 3.9 µM, about 4 µM, about 4.1 µM, about 4.2 µM, about 4.3 µM, about 4.4 µM, about 4.5 µM, about 4.6 µM, about 4.7 µM, about 4.8 µM, about 4.9 µM, or about 5 µM CHIR.

Lithium chloride (LiCl) activates WNT signaling and is included in differentiation medium to improve mesoderm induction during the first 48 hours of differentiation, which is understood in the art. The concentration of LiCl in the differentiation medium may be about 0.5 mM, about 0.6 mM, about 0.7 mM, about 0.8 mM, about 0.9 mM, about 1 mM, about 1.1mM, about 1.2 mM, about 1.3 mM, about 1.4 mM, about 1.5 mM, about 1.6 mM, about 1.7 mM, about 1.8 mM, about 1.9 mM, about 2 mM, about 2.1 mM, about 2.2 mM, about 2.3 mM, about 2.4 mM, or about 2.5 mM. Preferably, as understood in the art, concentration of LiCl in the medium may be about 2 mM.

As used herein, an "ACTIVIN antagonist" is a substance that inhibits or decreases ACTIVIN signaling. An ACTIVIN antagonist is not to be restricted to a substance acting directly on ACTIVIN as the substance may act elsewhere in the ACTIVIN signaling pathway.

Non-limiting examples of ACTIVIN antagonists according to embodiments of the invention include small molecules SB 431542 (CAS 301836-41-9), SB 505124 (CAS 694433-59-5), LDN 193189 (CAS 1062368-24-4), LDN 193719 (CAS 1062368-49-3), Dorsomorphin (CAS 866405-64-3), A 83-01 (CAS 909910-43-6), DMH 1 (CAS 1206711-16-1), RepSox (CAS 446859-33-2), or LY 364947 (CAS 396129-53-6). An ACTIVIN antagonist may also be an antagonist antibody or functional fragment thereof or an antibody-like polypeptide.

In one embodiment of the invention, the ACTIVIN antagonist is SB 431542 ((SB) CAS 301836-41-9), and the PSC is cultured in a medium comprising about 3-4 µM SB. In other embodiments, the medium may comprise about 2 µM, about 2.1 µM, about 2.2 µM, about 2.3 µM, about 2.4 µM, about 2.5 µM, about 2.6 µM, about 2.7 µM, about 2.8 µM, about 2.9 µM, about 3 µM, about 3.1 µM, about 3.2 µM, about 3.3 µM, about 3.4 µM, about 3.5 µM, about 3.6 µM, about 3.7 µM, about 3.8 µM, about 3.9 µM, about 4 µM, about 4.1 µM, about 4.2 µM, about 4.3 µM, about 4.4 µM, about 4.5 µM, about 4.6 µM, about 4.7 µM, about 4.8 µM, about 4.9 µM or about 5 µM SB.

In some embodiments, the medium may further comprise a retinoid, e.g. retinol or all *trans* retinoic acid, or a retinoic acid analogue, e.g. AM580 or EC23. The retinoid or retinoic acid analogue may be used in culturing at concentrations between 1 × 10⁻⁹ M and 1 × 10⁻⁴ M, e.g. 1 × 10⁻⁹ M, 0.5 × 10⁻⁸ M, 1 × 10⁻⁸ M, 0.5 × 10⁻⁷ M, 1 × 10⁻⁷ M, 0.5 × 10⁻⁶ M, 1 × 10⁻⁶ M, 0.5 × 10⁻⁵ M, 1 × 10⁻⁵ M, 0.5 × 10⁻⁴ M, or 1 × 10⁻⁴ M for a period ranging from one day to one week during differentiation. The retinoid or retinoic acid analogue may be added at or about d1, d2, d3, d4, d5, d6, or d7, d8, d9, d10, d11, or at later time points of differentiation. The retinoid or retinoic acid analogue may be added at or about d4 of differentiation, at or about d7 of differentiation or at or about d12 of differentiation. The retinoid or retinoic acid analogue may be added at more than one differentiation time point.

Determining an effective concentration of a WNT agonist other than SB and/or determining an effective concentration of an ACTIVIN antagonist

As used herein, "pluripotent stem cell" or "PSC" refers to a cell that has the ability to reproduce itself indefinitely, and to differentiate into any other cell type. There are two main types of pluripotent stem cell: embryonic stem cells (ESCs) and induced pluripotent stem cells (iPSCs).

For reference only, as used herein, "embryonic stem cell" or "ESC" refers to a cell isolated from a five to seven day-old embryo donated with consent by subjects who have completed *in vitro* fertilisation therapy, and have surplus embryos. The use of ESCs has been hindered to some extent by ethical concerns about the extraction of cells from human embryos.

For reference only, suitable ESCs for use in an embodiment of the invention include, but are not limited to, H1 and H9 human ESCs.

As used herein, "induced pluripotent stem cell" or "iPSC" refers to an ESC-like cell derived from adult cells. iPSCs have very similar characteristics to ESCs, but avoid the ethical concerns associated with ESCs, since iPSCs are not derived from embryos. Instead, iPSCs are typically derived from fully differentiated adult cells that have been "reprogrammed" back into a pluripotent state.

Suitable human iPSCs for use in an embodiment of the invention include, but are not limited to, iPSC 19-9-7T, MIRJT6i-mND1-4 and MIRJT7i-mND2-0 derived from fibroblasts and iPSC BM119-9 derived from bone marrow mononuclear cells. Other suitable iPSCs may be obtained from Cellular Dynamics International of Madison, WI, USA.

As used herein, "definitive haematopoietic stem/precursor cell" refers to a cell or population of cells responsible for producing all mature blood cells throughout the lifespan of an organism. Clinically, they are important for transplantation in blood-related diseases, particularly in subjects undergoing myeloablative therapy. Definitive haematopoiesis is distinguished from primitive haematopoiesis that occurs transiently during early development and arises from the yolk sac.

A definitive haematopoietic stem/precursor cell as described herein or produced by the method of the invention may be further characterized by gene and protein expression as detailed in the examples and figures.

As used herein, "embryoid body" and "EB" refers to a three-dimensional aggregate of PSCs. Advantageously, EBs may be cultured in suspension, thus making EB cultures scalable for clinical applications. Additionally, the three-dimensional structure of EBs, including the establishment of complex cell adhesions and paracrine signaling within the EB microenvironment, enables differentiation and morphogenesis which yields microtissues that are similar to native tissue structures, for example the AGM as disclosed herein.

In one embodiment of the invention, the PSCs of the present disclosure are cultured as an EB.

Methods for culturing EBs are known in the art. A specific example of a spin EB method that may be used in the present invention is described in Ng et al. Nature Protocols 3, 768-776 (2008).

As used herein, "increased *HOXA* gene expression" refers to a PSC expressing one or more genes from the *HOXA* cluster at a greater level as a result one or more specific culture conditions relative to expression of one or more genes from the *HOXA* cluster by a reference PSC cultured in the absence of the specific culture conditions. The specific culture conditions encompassed by the present invention are culture of the PSC in the presence of a WNT agonist and an ACTIVIN antagonist for about 2 days.

Gene expression may be measured by a number of means known in the art. For example, gene expression may be measured quantitatively by real time RT-PCR, or gene expression may be measured qualitatively by microarray.

Increased *HOXA* gene expression may be binary, either "on" or "off". Alternatively, increased *HOXA* gene expression may be about a 1% increase, about a 2% increase, about a 3% increase, about a 4% increase, about a 5% increase, about a 6% increase, about a 7% increase, about a 8% increase, about a 9% increase, about a 10% increase, about a 20% increase, about a 30% increase, about a 40% increase, about a 50% increase, about a 60% increase, about a 70% increase, about a 80% increase, about a 90% increase, about a 100%, or greater increase. Alternatively, increased *HOXA* gene expression may be about a 2-fold, about a 3-fold, about a 4-fold, about a 5-fold, about a 6-fold, about a 7-fold, about an 8-fold, about a 9-fold, about a 10-fold, or more increase.

In some embodiments of the invention, the *HOXA* gene is one or more of *HOXA1, HOXA2, HOXA3, HOXA4, HOXA5, HOXA6, HOXA7, HOXA8, HOXA9, HOXA10, HOXA11,* and *HOXA13.* In some embodiments of the invention, the HOXA gene is one or more of *HOXA3, HOXA5* and *HOXA9, HOXA10, HOXA11,* and *HOXA13.* In some embodiments, the *HOXA* gene is one or more of *HOXA3, HOXA7, HOXA9* and *HOXA10.*

As used herein, a "reporter" refers to an expression product, i.e. a nucleic acid or a protein, expressed from a specific locus that is measurable and indicative of transcription from the locus. The reporter may be endogenous or heterologous. Preferably, the reporter is a heterologous protein, examples of which include enzymes, immunoaffinity tags, and fluorescent proteins. Preferably, the reporter is a fluorescent protein. Many examples of fluorescent proteins are known in the art. Examples include GFP, EGFP, YFP, tdTomato, mCherry, mBanana, mTagBFP2 and so on.

According to the present disclosure, a PSC may comprise two or more reporters targeted to different loci, which therefore report expression of the different loci. To be meaningful where multiple reporters are potentially expressed in the same cell, the reporters are selected such that they are distinguishable. With respect to fluorescent proteins, this generally means non-overlapping or minimally-overlapping emission spectra.

Where multiple reporters are potentially expressed in the same cell, the reporters may be expressed at separate times or simultaneously. In one example of the second aspect, the *SOX17* reporter and the *RUNX1C* reporter are expressed at separate times or simultaneously.

In one example of the second aspect, the *SOX17* reporter comprises a first fluorescent protein expressed by the cell and the *RUNX1C* reporter comprises a second fluorescent protein expressed by the cell.

As used herein, "differentiating" and "differentiation" refers to a process of a cell changing from one cell type to another, in particular a less specialized type of cell becoming a more specialized type of cell.

As used herein, "tracking differentiation" refers to the ability to observe differentiation, for example, to observe the endothelial (*SOX17*) to haematopoietic (*RUNX1C*) transition of during PSC differentiation to definitive haematopoietic stem/precursor cells.

As used herein, "medium" or its plural "media" refers to a liquid or gel designed to support the growth of cells. In some embodiments, the cell culture medium comprises APEL medium.

As used herein, "APEL" medium refers to the Albumin Polyvinylalcohol Essential Lipids medium described in Ng et al. Nature Protocols 3, 768-776 (2008). APEL medium is available commercially. In an embodiment of the invention, the medium is APEL medium.

All proteins described herein are known to the person skilled in the art, and most if not all proteins described herein are available commercially.

Although the presently disclosed media may include specific components (e.g. morphogens, small molecules, and hematopoietic cytokines), it is contemplated that other components with the same, equivalent, or similar properties may be used in addition to or in place of those disclosed, as are known in the art.

In some embodiments of the invention, the medium comprises 20-40 ng/mL BMP4, 20-30 ng/mL VEGF, 40 ng/mL SCF, 10-20 ng/mL ACTIVIN A, and 10 ng/mL FGF2.

In some embodiments of the invention, the concentration of BMP4 in the medium is about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, or about 60 ng/mL.

In some embodiments of the invention, the concentration of VEGF in the medium is about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 65 ng/mL, or about 70 ng/mL.

In some embodiments of the invention, the concentration of SCF in the medium is about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 70 ng/mL, about 80 ng/mL, about 90 ng/mL, about 100 ng/mL, about 110 ng/mL, or about 120 ng/mL.

In some embodiments of the invention, the concentration of ACTIVIN A in the medium is about 1 ng/mL, about 2 ng/mL, about 3 ng/mL, about 4 ng/mL, about 5 ng/mL, about 6 ng/mL, about 7 ng/mL, about 8 ng/mL, about 9 ng/mL, about 10 ng/mL, about 11 ng/mL, about 12 ng/mL, about 13 ng/mL, about 14 ng/mL, about 15 ng/mL, about 16 ng/mL, about 17 ng/mL, about 18 ng/mL, about 19 ng/mL, about 20 ng/mL, about 21 ng/mL, about 22 ng/mL, about 23 ng/mL, about 24 ng/mL, about 25 ng/mL, or about 30 ng/mL.

In some embodiments of the invention, the concentration of FGF2, also known as basic fibroblast growth factor, in the medium is about 1 ng/mL, about 2 ng/mL, about 3 ng/mL, about 4 ng/mL, about 5 ng/mL, about 6 ng/mL, about 7 ng/mL, about 8 ng/mL, about 9 ng/mL, about 10 ng/mL, about 11 ng/mL, about 12 ng/mL, about 13 ng/mL, about 14 ng/mL, about 15 ng/mL, about 16 ng/mL, about 17 ng/mL, about 18 ng/mL, about 19 ng/mL, or about 20 ng/mL.

In some embodiments of the invention, the concentration of IGF2 in the medium is about 20 ng/mL, about 25 ng/mL, about 30 ng/mL, about 35 ng/mL, about 40 ng/mL, about 45 ng/mL, or about 50 ng/mL. Preferably, the medium comprises IGF2 at 30 ng/mL.

In some embodiments of the invention, the concentration of IL-6 in the medium is about 10 ng/mL, about 15 ng/mL, about 20 ng/mL, about 25 ng/mL, about 30 ng/mL, about 35 ng/mL, about 40 ng/mL, about 45 ng/mL, or about 50 ng/mL. Preferably, the concentration of IL-6 in the medium is 25 ng/mL to 30 ng/mL.

In some embodiments of the invention, the concentration of IL-3 in the medium is about 10 ng/mL, about 15 ng/mL, about 20 ng/mL, about 25 ng/mL, about 30 ng/mL, about 35 ng/mL, about 40 ng/mL, about 45 ng/mL, or about 50 ng/mL. Preferably, the concentration of IL-3 in the medium is 25 ng/mL to 30 ng/mL.

In some embodiments of the invention, the concentration of TPO in the medium is about 10 ng/mL, about 15 ng/mL, about 20 ng/mL, about 25 ng/mL, about 30 ng/mL, about 35 ng/mL, about 40 ng/mL, about 45 ng/mL, or about 50 ng/mL. Preferably, the concentration of TPO in the medium is 25 ng/mL to 30 ng/mL.

In some embodiments of the invention, the concentration of FLT3L in the medium is about 1 ng/mL, about 2 ng/mL, about 3 ng/mL, about 4 ng/mL, about 5 ng/mL, about 6 ng/mL, about 7 ng/mL, about 8 ng/mL, about 9 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, or about 70 ng/mL.

In some embodiments of the invention, the concentration of EPO in the medium is about 1 U/mL, about 2 U/mL, about 3 U/mL, about 4 U/mL, about 5 U/mL, about 6 U/mL, about 7 U/mL, about 8 U/mL, about 9 U/mL, or about 10 U/mL. Preferably, the medium comprises 3 U/mL, 4 U/mL or 5 U/mL EPO.

In some embodiments of the invention, the concentration of GM-CSF in the medium is about 10 ng/mL, about 15 ng/mL, about 20 ng/mL, about 25 ng/mL, about 30 ng/mL, about 35 ng/mL, about 40 ng/mL, about 45 ng/mL, or about 50 ng/mL. Preferably, the concentration of GM-CSF in the medium is 25 ng/mL.

Media disclosed herein may be also made in concentrated, including dried, forms that are diluted prior to use, such as 2×, 10×, 100×, or 1000× concentrations.

As used herein, "culturing" refers to the process by which cells are grown under controlled, *in vitro* conditions.

As used herein, "extracellular matrix" refers to the non-cellular component of all tissues and organs that provides essential physical scaffolding for the cellular components and initiates crucial biochemical and biomechanical cues that are required for tissue morphogenesis, differentiation and homeostasis. It follows that an "extracellular matrix protein" is a protein present in and/or derived from the extracellular matrix. Examples of extracellular matrix proteins include collagens, elastin, fibronectin, laminin, and proteoglycans. Extracellular matrix proteins, including mixtures, are available commercially, e.g. MATRIGEL^{™}.

As used herein, a "condition, disease or disorder requiring HSC transplantation" may be a malignant condition, disease or disorder, for example acute myeloid leukemia (AML), chronic myeloid leukemia (CML), acute lymphoblastic leukemia (ALL), Hodgkin lymphoma (relapsed, refractory), Non-Hodgkin (relapsed or refractory) lymphoma, neuroblastoma, Ewing sarcoma, multiple myeloma, a myelodysplastic syndrome, a glioma, or other solid tumour, or may be a non-malignant condition, disease or disorder, for example thalassemia, sickle cell anemia, aplastic anemia, Fanconi anemia, an immune deficiency syndrome, or an inborn error of metabolism.

A haematopoietic stem/progenitor cell as described herein or produced according to an embodiment of the invention may be used for treating a condition, disease or disorder requiring HSC transplantation following myeloablative therapy in a subject. Alternatively, haematopoietic stem/progenitor cell as described herein or produced according to an embodiment of the invention may be used for treating a condition, disease or disorder requiring HSC transplantation that does not require myeloablative therapy in a subject.

As used herein, "myeloablative therapy" refers to treatment, generally radiation or chemotherapy, that kills cells in the bone marrow, including cancer cells.

It will be appreciated by the person skilled in the art that the exact manner of administering to a subject a therapeutically effective amount of a haematopoietic stem/progenitor cell as described herein or produced according to the invention for treating a condition, disease or disorder will be at the discretion of the medical practitioner. The mode of administration, including dosage, combination with other agents, timing and frequency of administration, and the like, may be affected by the diagnosis of a subject's likely responsiveness to treatment with the haematopoietic stem/progenitor cell as described herein or produced according to the invention, as well as the subject's condition and history.

As used herein, the term "therapeutic composition" refers to a composition comprising a haematopoietic stem/progenitor cell as described herein or produced according to the invention that has been formulated for administration to a subject. Preferably, the therapeutic composition is sterile. In one example, the therapeutic composition is pyrogen-free.

The haematopoietic stem/progenitor cell as described herein or produced according to the invention will be formulated, dosed, and administered in a fashion consistent with good medical practice. Factors for consideration in this context include the particular type of condition, disease or disorder being treated, the particular subject being treated, the clinical condition of the subject, the site of administration, the method of administration, the scheduling of administration, possible side-effects and other factors known to medical practitioners. The therapeutically effective amount of the haematopoietic stem/progenitor cell as described herein or produced according to the invention to be administered will be governed by such considerations.

The haematopoietic stem/progenitor cell as described herein or produced according to an embodiment of the invention may be administered to a subject by any suitable method including intravenous (IV), intra-arterial, intramuscular, intraperitoneal, intracerobrospinal, subcutaneous (SC), intra-articular, intrasynovial, intrathecal, intracoronary, transendocardial, surgical implantation, topical and inhalation (e.g. intrapulmonary) routes. Most preferably, the haematopoietic stem/progenitor cell as described herein or produced according to the invention is administered IV.

The term "therapeutically effective amount" refers to an amount of the haematopoietic stem/progenitor cell as described herein or produced according to the invention effective to treat a condition, disease or disorder in a subject.

The terms "treat", "treating" or "treatment" refer to both therapeutic treatment and prophylactic or preventative measures, wherein the aim is to prevent or ameliorate a condition, disease or disorder in a subject or slow down (lessen) progression of a condition, disease or disorder in a subject. Subjects in need of treatment include those already with the condition, disease or disorder as well as those in which the condition, disease or disorder is to be prevented.

The terms "preventing", "prevention", "preventative" or "prophylactic" refers to keeping from occurring, or to hinder, defend from, or protect from the occurrence of a condition, a disease or disorder, including an abnormality or symptom. A subject in need of prevention may be prone to develop the condition, disease or disorder.

The term "ameliorate" or "amelioration" refers to a decrease, reduction or elimination of a condition, a disease or disorder, including an abnormality or symptom. A subject in need of treatment may already have the condition, disease or disorder, or may be prone to have the condition, disease or disorder, or may be in whom the condition, disease or disorder is to be prevented.

As used herein, the term "subject" refers to a mammal. The mammal may be a primate, particularly a human, or may be a domestic, zoo, or companion animal. Although it is particularly contemplated that the method and its resulting definitive haematopoietic stem/progenitor cell or population of definitive haematopoietic stem/progenitor cells disclosed herein are suitable for medical treatment of humans, they are also applicable to veterinary treatment, including treatment of domestic animals such as horses, cattle and sheep, companion animals such as dogs and cats, or zoo animals such as felids, canids, bovids and ungulates.

### EXAMPLES

### METHODS

### Generation and validation of targeted RUNX1C^{GFP/w}, RUNX1C^{GFP/GTP}, SOX17 ^{mCHERRY/w} and SOX17^{mCHERRY/w} RUNX1C ^{GFP/w} hESC lines

The *RUNX1C* targeting vectors comprised a 5.9kb 5' homology arm, sequences encoding GFP, a loxP flanked antibiotic resistance cassette with a PGK promoter driving either neomycin phosphotransferase (PGKneo) or hygromycin resistance (PGKhygro) genes and a 3kb 3' homology arm (Figure 2a). The homology arms were amplified by polymerase chain reaction (PCR) from HES3 genomic DNA. The GFP sequences were cloned in frame with the start codon of *RUNX1C* in exon 1, downstream of the distal promoter. For reference only, vectors were linearized with XhoI prior to electroporation into wild-type HES3 cells and selection for antibiotic resistant colony growth. Targeted clones were identified by PCR screening using primer pairs p7/p4 (PGKneo) or p3/p4 (PGKhygro) (Figure 2a, b and Table 1) that produced a 3.4 kb fragment when the 3' homology arm of the vector was correctly integrated. Six targeted clones were identified from a total of 231 screened (2.6%), two from the PGKneo vector and four from the PGKhygro vector. Homologous recombination of the GFP-containing 5' homology arm was confirmed for all positive clones by PCR using primers p1 and p2 (Figure 2b). Correct targeting of the *RUNX1C* locus was further verified by sequencing the PCR-generated fragments from 5' and 3' arms described above. Two independently isolated *RUNX1C*^{*GFP*/*w*} clones (35 and 67) were expanded and the loxP flanked antibiotic resistance cassettes were excised by transient transfection with a Cre-recombinase plasmid, pEFBOS-Cre-IRESpuro. Several subclones were screened by PCR for the removal of the PGKneo or PGKhygro selection cassettes as well as for the absence of the Cre expression plasmid. Subclones 35.3 and 67.4 were selected for further analysis. Both lines expressed surface markers of undifferentiated hESCs, retained a normal karyotype and formed teratomas following injection of 2 × 10⁵ undifferentiated *RUNX1C^{GFPiW}* hESCs under the kidney capsule of NOD/SCID/IL2Rγ-/- mice. These data are shown in Ditadi et al. Nature Cell Biology 17, 580-591 (2015).

A list of primers used in this study, their sequences and locations within the *RUNX1* gene or the targeting vector is provided in Table 1. Primers p1-p10 were used for screening of targeted clones and the remaining primers were used for RT-PCR to distinguish the B (Human Fujita a and Human Fujita c) and C (Human Fujita b and Human Fujita c) isoforms or identify the combination of the B and C isoforms (denoted common RUNX1) (fAMLla and rAML1a). See also Figure 2.

**Table 1. RUNX PCR primers.**

| **Primer** | **Sequence** | **SEQ ID NO:** | **Location** | **Comment** |
|---|---|---|---|---|
| p1 | CCCAAATGCTTGAAAGATCATATCC | 13 | 5' of Exon 1 | |
| p2 | GTCGCCGTCCAGCTCGACCAGGATG | 14 | GFP rev | |
| p3 | CTCCGCATTGGTCTTGACCAACTC | 15 | HYGRO fwd | |
| p4 | GCAGCTTTGTACTTGGCAATTCCC | 16 | Intron 1 rev | |
| p5 | TACGTCGACCATCGCTTCCTCCTGA AAATGCAC | 17 | Exon 1 rev | |
| p6 | TCTCACGCACCGACTGAACACTCC | 18 | Intron 1 rev | |
| p7 | CGGTGGGCTCTATGGCTTCTGAGG | 19 | G418R fwd | |
| p9 | TCGGCATGGACGAGCTGTACAAG | 20 | GFP fwd | |
| p10 | CACACTGAATGCAAACCACAGGG | 21 | Exon 1 fwd | |
| Human Fujita a | ACGTGTATGTTGGTCTCCCG | 22 | Exon 3 fwd (5' UTR) | a,c detects RUNX1A/B |
| Human Fujita b | AAGCGATGGCTTCAGACAG | 23 | Exon 1 fwd | b,c detects RUNX1C |
| Human Fujita c | TCACAGTGACCAGAGTG | 24 | Exon 4 rev | |
| fAML1a | TGTGGTCCTATTTAAGCCAGCCCC | 25 | Exon 8 fwd (3' UTR) | fAML1a and fAML1b detect RUNXB/C |
| rAMLla | TCAGGCTGGGCACGACGAATGCTC | 26 | Exon 8 rev (3' UTR) | |

To generate the *RUNX1C*^{*GFP*/*GFP*} knockout hESC line, the *RUNX1C*^{*GFP*/*w*} line (clone 67.4) was electroporated with the *RUNX1C* PGKhygro construct described above, resulting in the identification of 2/308 hygromycin resistant clones that were targeted at the 3' end following PCR screening using p3/p4 primers (Figure 2e-g). A second PCR using a GFP forward primer (p9) and p4, performed to identify and distinguish the alleles *RUNX1C*^{GFP} (3.4 kb) and *RUNX1C*^{GFPHyg} (5.5 kb), confirmed that both alleles were targeted in one of the two clones (#253) and that the PGKhygro vector replaced the previously targeted allele in the second clone (#180). A third PCR screen with a *RUNX1C* 5' UTR primer (p10) and p6, amplified wild type (600 bp) and GFP targeted (1.7 kb) alleles and confirmed the absence of a wild type allele in *RUNX1C*^{GFPHyg} clone 253 (Figure 2g). The hygromycin selection cassette was removed by Cre recombinase as described above. The *RUNX1C*^{GFP/GFP} null line expressed surface markers of undifferentiated hESCs and retained a normal karyotype (data not shown). Differentiated *RUNX1C* ^{GFP/GFP} did not express *RUNX1C* by PCR (Figure 2h).

For reference only, the *SOX17*^{mCHERRY/w} H9 line has been described previously. Briefly, The *SOX17*-mCHERRY targeting vector comprised an 8.3kb 5' homology arm that encompassed genomic sequences located immediately upstream of the *SOX17* translational start site, sequences encoding mCHERRY, a loxP-flanked PGK-Neo antibiotic resistance cassette and a 3.6kb 3' *SOX17* homology arm. For reference only, the human ESC line H9 (purchased from WiCell) was electroporated with the linearized vector and correctly targeted clones identified using a PCR based screening strategy as described above. The antibiotic resistance cassette was excised using CRE recombinase. The *SOX17*^{mCHERRY/w} human ESC line used in this study was validated by demonstrating the correlation between *SOX17* and protein and mCHERRY expression on endodermally differentiated cells (Figure 2j).

The dual reporter *SOX17*^{mCHERRY/w}*RUNX1C*^{GFP/w} line was generated in H9 by retargeting GFP to the *RUNX1C* locus in the *SOX17*^{mCHERRY/w} human ESC line, as outlined above.

For all lines, genomic integrity was confirmed using the Illumina HumanCytoSNP-12 v2.1 array.

### Gene expression analysis

Total RNA was prepared from ES cells and dissociated EBs using the Qiagen RNeasy kit or the Bioline Isolate II RNA Mini Kit according to the manufacturers' instructions. cDNA was reverse transcribed using random hexamer priming and Superscript III (Invitrogen) or Tetro (Bioline) cDNA synthesis kits as specified by the manufacturer. Human *RUNX1* isoform specific primers (Table 1) were based on those designed for mouse *Runx1* previously. For other genes, TaqMan gene expression probes and reagents (Applied Biosystems) were used for quantitative real-time PCR analysis and *GAPDH* was used as the reference gene to normalize data.

### Culture and differentiation of hESCs

For reference only, the HES3 and H9 human embryonic stem cell lines used in these studies were obtained from ES Cell International (now owned by Biotime Inc) and WiCell. The human ESC work was approved by the Monash Medical Centre and Royal Children's Hospital Human Research Ethics Committees. Culture and enzymatic passaging of hESC lines was performed as reported in Ng et al. Nature Protocols 3, 768-776 (2008). Differentiation of hESC lines was performed using the spin EB method in APEL medium as described in Ng et al. Nature Protocols 3, 768-776 (2008) supplemented for the first 4 days with 20-40 ng/mL recombinant human (rh) bone morphogenetic protein 4 (BMP4, R&D Systems), 20-30 ng/mL rh vascular endothelial growth factor (VEGF, PeproTech) and 20-40 ng/mL rh stem cell factor (SCF, PeproTech), 10-20 ng/mL rh ACTIVIN A (R&D Systems) and 10 ng/mL rh FGF2 (PeproTech) (Figure 7a). Where indicated, cultures included additional CHIR99021 3 µM (Tocris Biosciences), SB431542 3-4 µM (Cayman Chemicals). After 4 days, the differentiation medium on the spin EBs was changed to APEL medium supplemented with 50 ng/mL rhVEGF, 20 ng/mL BMP4, 10 ng/mL FGF2, 50 ng/mL rh SCF and 30 ng/mL rh insulin-like growth factor 2 (IGF2, PeproTech). At d7-8 of differentiation, EBs were transferred onto gelatinized 6-well plates at 20-30 EBs/well in APEL medium including 50 ng/mL rhVEGF, 50-100 ng/mL rh SCF, 25-30 ng/mL rh interleukin (IL)-3 (PeproTech), 25 ng/mL rh IL-6 (PeproTech), 30 ng/mL rh thrombopoietin (TPO, Peprotech), 30 ng/mL rh FLT3 receptor ligand (FLT3L, PeproTech), 3 U/mL rh erythropoietin (EPO, PeproTech), 10 ng/mL FGF2, 50 ng/mL rh SCF and 30 ng/mL rh insulin-like growth factor 2 (IGF2, PeproTech). In later experiments, the IL-3 and EPO were omitted. Medium was changed at least weekly thereafter. For analysis, the EBs were harvested at various time points and dissociated into a single cell suspension using TrypLE Select (Invitrogen) and, for timepoints after d14, Collagenase type I (Worthington) and passaged through a 23-gauge needle as described in Ng et al. Nature Protocols 3, 768-776 (2008). Brightfield and fluorescence images comparing *RUNX1C* ^{GFP/w} with *RUNX1C* ^{GFP/GFP} cell cultures shown in Figure 1b were representative of 3 independent experiments. Images of *SOX17*^{mCHERRY/w}*RUNX1C*^{GFP/w} cell culture in Figure 12a-h were representative of more than 10 independent experiments and those in Figure 11d, e were representative of 5 independent experiments.

### Differentiation of hESCs in spinner flasks

To obtain larger yields of haematopoietic cells for d7 T cell assays, after the first 4 days of spin EB culture, the EBs were transferred to 125 ml spinner flasks (Corning Life Sciences) at a density of 60 EBs per 60 ml and cultured for a further 3 days in APEL medium/0.1% methylcellulose supplemented with 50 ng/mL rhVEGF, 3 U/mL rh EPO, 50 ng/mL rh SCF, 10 ng/mL rh FGF2, 30 ng/mL rh TPO, 30 ng/mL rh FLT3L and 30 ng/mL rh IGF2. For analysis, the EBs were harvested and dissociated into a single cell suspension using TrypLE Select and passaged through a 23-gauge needle prior to cell sorting as described in Ng et al. Nature Protocols 3, 768-776 (2008).

### Flow Cytometry and Cell Sorting

Antibodies directed against the following cell surface antigens (fluorochrome, manufacturer, catalogue number, clone [where known]) were used for staining dissociated EBs for flow cytometric analysis: CD34 (phycoerythrin[pe]-cy7, Biolegend #343515, clone 581) CD45 (brilliant violet, Biolegend #304032, clone H130), VE-CADHERIN (allophycocyanin [apc], Biolegend #348508, clone bv9), CD33 (apc, BD Pharmingen #340474), TIE2 (BD Pharmingen #557039, clone 33), platelet-derived growth factor receptor alpha (PDGFRα) (BD Pharmingen #556001, clone aR1), CD41a (apc, BD Pharmingen #559777, clone HIP8), CD43 (apc, Biolegend #343206, clone 10G7), Glycophorin A (apc, BD Parmingen #551336, clone GA-R2[HIR2]), CD7 (apc, Biolegend #343107, clone 6B7), CD5 (pe-cy7, Biolegend #300622, clone UCHT2), CD4 (apc, BD Pharmingen #555349, clone RPA-T4), CD8 (pe-cy7, Biolegend #344712, clone SK1), CD56 (pe, BD Pharmingen #555516, clone B159), CD90 (apc, BD Pharmingen #559869, clone 5E10), KIT (apc, Biolegend #313206, clone10D2) and DLL4 (apc, Biolegend #346508, MHD4-46). Unconjugated primary antibodies (TIE2 #557039, clone 33 and PDGFRα #556001, clone aR1, both BD Pharmingen) were detected with antimouse secondary antibodies conjugated to apc (BD Pharmingen #550826). Flow cytometric analysis was performed using FACSCALIBUR^{™} and BD FORTESSA^{™} analysers and cell sorting was done using FACS DIVA^{™}, FACS ARIA^{™} and BD INFLUX^{™} cell sorters (BD Biosciences). Flow cytometry plots shown in Figures 2j, 3, 6c, 11a,b, 12k and 14b were representative of 3-5 independent experiments. Each flow cytometry plot shown in Figure 15a - d relates to one sorting experiment.

### T cell assays

Flow sorted fractions of d7-8 differentiated *SOX17*^{mCHERRY/w}*RUNX1C*^{GFP/w} cells were co-cultured with OP9 DL4 cells in αMEM (Invitrogen) supplemented with 20% FCS, 10 ng/nl rh FLT3L, 10 ng/mL rh IL7 (PeproTech) and 25 ng/mL rh SCF. Twenty thousand sorted cells were cultured per well of OP9 DL4 on 12-well plates (Costar). Cells were passaged weekly onto fresh OP9 DL4 layers by pipetting the cultures and passing cell through a 40 µM cell strainer. Cultures were analysed by flow cytometry after ~21 and ~28 days of culture on OP9 DL4. Brightfield and fluorescent images in Figure 11d,e and flow cytometry profiles in Figure 11a,b,f,g were representative of 4 experiments and Figure 11j was representative of 2 experiments.

### Colony forming assays

Hemangioblast colonies (Bl-CFCs) were identified by culturing 5 × 10³ dissociated cells from d2-4 EBs in serum-free MethoCult (StemCell Technologies) or in a formulation denoted MC-APEL (1% methylcellulose in APEL medium) supplemented with 30 ng/mL VEGF, 50 ng/mL SCF and 3 U/mL EPO, with 20 ng/mL BMP4 and 10 ng/mL FGF2 also added to MC-APEL cultures. For the identification of hematopoietic CFCs emerging at later times, 2 - 20 × 10³ differentiated cells were cultured in serum free MethoCult or MC-APEL supplemented with 5 U/mL rh EPO, 50 ng/mL rh VEGF, 30 ng/mL rh IL-3, 30 ng/mL rh IL-6, 25 ng/mL rh GM-CSF, 25 ng/mL rh TPO, 50 ng/mL rh FLT3L and 100 ng/mL rh SCF. Plates were scored for colony formation between 7 and 14 days. Cytocentrifuge preparations of harvested colonies were stained with May-Grunwald-Giemsa (Sigma) according to the manufacturer's instructions. Brightfield and fluorescence images of stained cytocentrifuge preparations shown in Figure 1h, i were representative of 3 independent experiments, and the colonies shown in Figure 13a-f were representative of 5 independent experiments.

### Transcriptional Profiling by Illumina Microarray

In the first series of experiments, differentiated cultures of *RUNXIC*^{GFP/w} cells were harvested daily for 8 days. In a second experiment, differentiated cultures of *RUNX1C*^{GFP/w} and *RUNX1C*^{GFP/GFP} cells were dissociated at d14 and flow sorted based on their expression of CD34 and GFP using a BD INFLUX^{™} cell sorter. Finally, three cord blood cell samples sorted for CD34 expression were profiled in parallel with the d14 differentiated *RUNXIC*^{GFP/w} and *RUNX1C*^{GFP/GFP} samples. In all experiments, total RNA was extracted from the collected cell fractions (High Pure RNA Isolation Kit, Roche Applied Science) and RNA quality was assessed using a NanoDrop 2000 analyzer (Thermo Scientific). Total RNA from each fraction was amplified, labeled, and hybridized to Human HT12 v3 and v4 BeadChips according to Illumina standard protocols (Illumina) at the Australian Genome Research Facility. Initial data analysis was performed using GenomeStudio Version 2010.2 (Illumina), using average normalization across all the samples. Further data analysis was performed in R/BioConductor using algorithms within the lumi package (function: bgAdjust.affy and quantile normalization). Subsequent data analysis was performed using MultiExperiment Viewer. Principle component analysis was performed to determine the overall relationship between samples. Differentially expressed genes were identified using SAM (Significance Analysis for Microarrays) with a zero false discovery rate. Hierarchical clustering of genes was performed using Pearson correlation with average linkage clustering. Differentially expressed genes were subjected to pathway over-representation and functional clustering analysis using the DAVID public database (Database for Annotation, Visualization and Integrated Discovery). The microarray data reported here are deposited with Array Express, accession number E-MEXP-3356, and Gene Expression Omnibus, accession number GSE64876.

The experiment describing *HOX* gene expressing in differentiated cultures of d4 *MIXL1*^{GFP/w} cells used data derived from a previous sorting experiment. The Array Express accession number for this data is E-MEXP-2495.

### Transcriptional profiling using RNA-Seq

Differentiated *SOX17*^{mCHERRY/w}*RUNX1C*^{GFP/w} cells at d10/11 cultured with or without additional SB/CHIR from d2-4 were flow sorted as shown in Figure 6c. Samples were sent for RNA sequencing at the Australian Genome Research Facility. In total, data from 26 samples from three independent experiments were sequenced. In a second series of experiments, differentiated *SOX17*^{mCHERRY/w}*RUNX1C*^{GFP/w} cells at d18 or d22 cultured with SB/CHIR from d2-4 were flow sorted as shown in Figure 15a,b. The 5 samples from two independent experiments were sequenced on the same run as the human AGM and foetal liver samples (Figure 15c,d) at the University of California, Los Angeles, core sequencing facility. Analysis for both series of sequences was performed by the Murdoch Childrens Research Institute bioinformatics research unit. The STAR aligner (v2.4.0h1) was used to map the 100bp single end reads to the human reference genome (hg19) in the two pass mapping mode. The uniquely mapped reads were summarized across genes with featureCounts (v1.4.6) using Gencode Release 19 comprehensive annotation. Lowly expressed genes were filtered out (less than one count per million in fewer than two samples), leaving 18,456 genes for further analysis. The data was TMM normalized, voom transformed and differential expression assessed using moderated t-tests from the R Bioconductor limma package, taking into account the different culture medium base. Genes that had a false discovery rate of less than 5% were called significantly differentially expressed for the various comparisons of interest. Gene ontology analysis was performed using DAVID.

### Assay for Transposase-Accessible Chromatin with high throughput sequencing (ATAC-Seq)

Differentiated *SOX17*^{mCHERRY/w}*RUNX1C*^{GFP/w} cells at d10 cultured with or without additional SB/CHIR from d2-4 were flow sorted as shown in Figure 6c and samples of 5 × 10⁴ SOX17⁺34⁺ and SOX17⁻34⁺ endothelial cells were processed for ATAC-Seq analysis using a protocol published by Buenrostro et al. Curr Protoc Mol Biol 109, 21 29 21-29 (2015) with minor adjustments. Duplicate samples were processed for each of two independent experiments for both control and SB/CHIR differentiations. Nuclei were purified by the addition to the cellular pellet of 250 µl of cold lysis buffer (10 mM Tris-HCl, pH 7.4, 10 mM NaCl, 3 mM MgCl₂, 0.1% IGEPAL CA-630), pelleted and resuspended in the transposition reaction mix (Nextera DNA Library Prep Kit, Illumina) and incubated at 37°C for 30 minutes. Transposed DNA was column purified and used for library amplification with custom made adaptor primers using NEBNext High-Fidelity 2x PCR Master Mix (New England Labs). The amplification was interrupted after 5 cycles and a SyBR green qPCR was performed with 1/10 of the sample to estimate for each sample the additional number of cycle to perform, before saturation was achieved. Total amplification was between 10 and 15 cycles. Purified Libraries were then sequenced using HIseq-2000 (Illumina). The sequencing run for the first experiment used 50bp PE reads, whilst the second run used 50bp SE reads. The reads were trimmed using Trimmomatic (v0.33); bases with a Phred score < 15 were trimmed from both 5' and 3' ends, and using a sliding window of 5bp. Reads < 30bp after trimming were discarded. Reads were then mapped to the human genome (hg19) using bowtie2 (v2.1.0). Duplicates were removed with picard (v1.99(1563)). Reads were summarized across hg19 RefSeq genes (including 2kb upstream) using featureCounts (v1.4.6). The summarised data contained counts for 26,402 genes. Genes with ≥ 10 counts per million (CPM) in ≥ 2 samples were retained for further analysis (17,974). The data was TMM normalized. Differential 'accessibility' was assessed using likelihood ratio tests from the R Bioconductor edgeR package. Genes that had a Benjamini-Hochberg false discovery rate of less than 5% were significantly differentially 'accessible' for the various comparisons of interest.

### DNA methylation analysis using Illumina Infinium 450 mircoarray

Differentiated *SOX17*^{mCHERRY/w}*RUNX1C*^{GFP/w} cells at d10 cultured with or without additional SB/CHIR from d2-4 were flow sorted as shown in Figure 6c and genomic DNA was extracted from control and SB/CHIR samples of 5 × 10⁵ SOX17⁺34⁺ and SOX17⁻34⁺ endothelial cells, and SOX17⁻34⁻ stromal cells for two independent experiments. Genomic DNA was bisulphite converted using MethylEasy Xceed kit according to manufacturer's instructions (Human Genetic Signatures, Australia). The conversion efficiency was assessed by bisulphite-specific PCR. Bisulphite DNA hybridization to Illumina Human Methlylation450 (HM450) arrays was carried out at the Australian Genome Research Facility. Data exploration and analysis was performed in R using the Bioconductor packages minfi and limma. The data was normalized using stratified quantile normalization (SQN). Probes with a detection p-value ≥ 0.01 in one or more samples were excluded from the subsequent analysis. Nonspecific probes and probes with common SNPs at either the CpG or single base extension (SBE) site were also excluded. After filtering, 428,990 probes remained for further analysis. Differential methylation was assessed using moderated t-tests from the R Bioconductor limma package. P values were adjusted for multiple testing using the Benjamini-Hochberg (FDR) method.

### Mice

NOD/SCID mice were purchased from ARC (Perth, Australia) and housed in specific pathogen free (SPF) conditions at Monash University Animal Services (MAS, Melbourne, Australia). NOD.Cg-Prkdc^{scid} Il2rg^{tm1Wjl}/SzJ mice (NOD/SCID/IL2Rγ-/-, NSG) (stock number 005557, The Jackson Laboratory) were bred at MAS in SPF conditions. The colony was regularly checked for γ-c deficiency by PCR according to the Jackson Laboratory protocol. The Monash University institutional animal ethics committee approved all animal protocols, and experiments were carried out under its guidelines for the care and use of laboratory animals.

### Human tissue collection, processing and sequencing

### i) AGM tissue procurement and processing

This study was performed in accordance with institutional guidelines and was approved by the local research ethics committee (University of Tübingen IRB #406/2011B02, #710/2012BO2 and #690/2013BO2). For reference only, human first trimester tissue (5 weeks of gestation) was obtained from an electively aborted fetus following informed consent and de-identification. After procurement, the tissues was immediately washed in sterile Dulbecco's phosphate buffered saline (DPBS, Invitrogen, Cat# 14190-169), placed in medium (DMEM/F12, Gibco, Cat# 31330-095) that was supplemented with 5% foetal bovine serum (ThermoFisher, Cat# 16000-044), 1% Penicillin-Streptomycin (Gibco, Cat# 15140-122) and 2.5 µg/mL Amphotericin B (Sigma Aldrich, Cat# A9528), and processed for gene expression analyses within 48 hours. The embryo was dissected to isolate the AGM region. The tissue was digested in 2.5 U dispase (Gibco), 90 mg collagenase A (Worthington), and 0.075 mg DNase I (Sigma) per ml in PBS containing 10% FBS, for 90 minutes at 37°C. Cells were then disaggregated by pipetting, and filtered through a 70 µm cell strainer.

### ii) Foetal liver tissue collection and processing

Foetal liver was de-identified, discarded material obtained from elective termination of a second trimester pregnancies following informed consent. Specimen age for this study (17 weeks) is denoted as developmental age, two weeks less than gestational age, and was determined by ultrasound or estimated by the date of the last menstrual period. Tissues were harvested into PBS 5% FBS (Hyclone), Ciprofloxacin HCl (10 ng/mL, Sigma), amphotericin B (2.50 µg/mL, Invitrogen), 1% penicillin/streptomycin, transported on ice and processed the same day. Tissues were mechanically dissociated using scalpels and syringes. Mononuclear cells were enriched on a Ficoll layer according to manufacturer's protocol (GE Healthcare Biosciences AB) and strained through a 70 µm mesh.

### iii) Antibody staining and flow cytometric sorting

Cells were stained with the following antibodies: human-CD45-APC-H7 cl.2D1 (Biolegend 368516; 1:100), CD34-APC cl. 581 (BD 555824; 1:20), CD90-BV421 cl. 5E10 (BD 562556; 1:100), CD38-PE-Cy7 cl. HIT2 (BD 560677; 1:100), CD43-FITC cl.1G10 (BD 555475; 1:20), GPI-80-PE cl. 3H9 (MBL D087-5; 1:50). The indicated populations (Figure 15c, d) were sorted into RLT buffer (Qiagen) using a BD FACS Aria cell sorter and snap frozen at -80°C.

### iii) RNA extraction and library preparation and RNA-sequencing.

Total RNA from 500-2000 sorted cells was extracted using the RNeasy Mini kit (Qiagen) and library was constructed using Ovation Rna-seq system System v2 (Nugen), followed by KAPA LTP Library Preparation Kit. Libraries were sequenced using HIseq-2000 (Illumina) to obtain single end 50 bp long reads. Demultiplexing of the reads based on the barcoding was performed using in house Unix shell script. Sequencing was performed at the University of California, Los Angeles, core sequencing facility.

### Cord blood cells

Samples of human umbilical cord blood from healthy subjects were obtained from the Mercy Hospital for Women, Studley Road, Heidelberg, Victoria 3084, under auspices of the hospital Human Research Ethics Committee. Mononuclear cells or flow sorted CD34⁺ cells were isolated and cryopreservation for subsequent use in transcriptional profiling or transplantation assays.

### Analysis of bone marrow homing of CFSE-labeled differentiated cells

### i) CFSE labeling of differentiated hPSCs

Differentiated *RUNX1C* ^{GFP/w} and *RUNX1C* ^{GFP/GFP} cells were flow sorted at d14 based on CD34 and GFP expression, or at earlier time points on the basis of CD34 and CD41 expression. Viable cells were labeled with 5-(and 6)-carboxyfluorescein diacetate succinimidyl ester (CFSE; Molecular Probes). Briefly, cell populations were resuspended in PBS 0.5% heat-inactivated (HI) foetal calf serum (FCS; Hyclone, Logan, UT, USA) at a density of 1 × 10⁶ cells/mL and pre-incubated at 37°C for 3 minutes. CFSE was diluted to 5 mM in dimethyl sulfoxide (DMSO) and then to 5 µM in PBS. CFSE was added to the cells to give a final concentration of 0.5 µM, and the dye solution/cell mixture was incubated at 37°C for a further 10 minutes. Staining was stopped by adding 10 times the dye solution/cell volume of ice-cold PBS containing 20% FCS.

### ii) Irradiation of immunodeficient mice

The ability of cells to reconstitute haematopoiesis was analyzed in NSG mice receiving a near-lethal dose of irradiation (2.75 Gy), delivered from two opposing ¹³⁷Cs sources (Gammacell 40; Atomic Energy of Canada, Ottawa, ON, Canada) at a dose rate of 1.4 Gy/minute.

### iii) Cell homing

Sorted, CFSE-labeled cells were transplanted in 0.2 ml of PBS by injection into the lateral tail vein of near-lethally irradiated NSG mice, with 3 recipients per sorted cell fraction, for each of 4 experiments. As a result, the data accumulated are the result of multiple sorts at each time point. On average, 1 × 10⁵ sorted, CFSE⁺ cells were co-injected with 2 × 10⁶ irradiated (15 Gy), unlabelled human cord-blood mononuclear cells. The homing ability was analysed 15 hours post-transplant. For the analysis of homing, bone marrow samples were prepared from individual mice as described below. The analysis of the proportion of CFSE⁺ donor cells in each fraction was performed using the number of live white blood cells (WBC) as the denominator for the total number of cells analysed by flow cytometry. No statistical method was used to predetermine sample size and the experiments were not randomized. The investigators were not blinded to allocation during experiments and outcome assessment.

### iv) Isolation of hematopoietic cells

NSG mice were humanely killed by cervical dislocation. Bone marrow was routinely collected from femurs, tibiae and iliac crests. These bones were thoroughly ground in a mortar and pestle in phosphate-buffered saline (PBS) supplemented with 2% heat inactivated FCS. Bone fragments were washed, and the supernatant cell suspension was filtered through a 40 µM filter (Becton Dickinson, NJ, USA). The marrow was centrifuged (400 g, 5 minutes) and resuspended in fresh buffer. The cell supernatant was refiltered through a 40 µM filter and diluted to 1 × 10⁷ cells/mL PBS supplemented with 2% heat inactivated FCS (buffer). Donor cells labeled with CFSE that homed to the bone marrow were identified by flow cytometry.

### Statistical analysis

Experiments were analysed using GraphPad Prism versions 5 and 6 (GraphPad Software Inc.) and Microsoft Excel (Microsoft corporation). Tests for statistical significance are listed with each experiment and included two sided Student's t test for paired analyses and ANOVA for experiments with multiple comparisons of one or more grouped variables, accompanied by the post-tests (Dunnett, Tukey, Holmes-Sidak) indicated as appropriate by the software. No statistical method was used to predetermine sample size.

### RESULTS

### RUNX1C is expressed in human hESC-derived haematopoietic cells

To identify haematopoietic stem/progenitor cells during hPSC differentiation, reporter lines targeting GFP to the *RUNX1C* locus (Figure 1a and Figure 2a-c) were generated. This choice was based on observations that *Runx1c* marked mouse haematopoietic progenitor cells, and that disruption of one allele did not adversely affect haematopoiesis. Differentiation of heterozygous *RUNX1C*^{GFP/w} cells as spin embryoid bodies (EBs) in APEL medium supplemented with haematopoietic growth factors was indistinguishable from differentiation of unmodified cells. GFP was restricted to *RUNX1C-*expressing cells, validating the reporter line (Figure 1b and Figure 2d). RUNX1C⁺ cells appeared between differentiation day (d) 8 and 11, initially expressing CD34, although by d15 most RUNX1C⁺ cells expressed CD45 (Figure 1c,d). They did not express mesodermal (PDGFRα) or vascular (TIE2/TEK) markers (Figure 1c,d), confirming the haematopoietic specificity of *RUNX1C* expression.

Sequential expression of the haematopoietic cell surface markers CD34, CD43 and CD41, antedated *RUNX1C* expression (Figure 3a,b). The earliest RUNX1C⁺ cells expressed these markers, and by d16 many RUNX1C⁺ cells co-expressed VE-CADHERIN and CD45, considered markers of 'pre-HSCs' in the mouse AGM (Figure 3c,d).

### RUNX1C expression identifies CD34⁺ cells with clonogenic and bone marrow homing ability

Given the hematopoietic specificity of the *RUNX1C* isoform, homozygous *RUNX1C*^{GFP/GFP} cells to assess whether *RUNX1C* was required for human haematopoiesis (Figure 2e-h) were generated. *RUNX1C*^{GFP/w} and *RUNX1C*^{GFP/GFP} lines displayed similar morphology, GFP and cell surface marker expression (Figure 1b and Figure 3e-h). Clonogenic frequency was enriched in GFP⁺34^{lo} populations derived from both *RUNX1C*^{GFP/w} and *RUNX1C*^{GFP/GFP} cultures, with similar colony morphology in each case (Figure 1 e-i and Figure 3i). These experiments indicated that although *RUNX1C* expression marked CD34⁺ clonogenic cells, these precursors formed independently of *RUNX1C.*

Homing to bone marrow of d14 differentiated cells; an ability needed for long-term engraftment was evaluated. Significant homing was observed in GFP⁺CD34^{lo} cells sorted from the heterozygous, but not the null line (Figure 1j). The frequency of bone marrow homing (0.79±0.09%) was approximately half that reported for CD34⁺ cells in cord blood (CB) (1.7%). These results demonstrate that bone marrow homing activity resides in RUNX1C⁺34^{lo} cells, and that this is compromised in the absence of *RUNX1C.* However, despite their clonogenic activity and bone marrow homing ability, the RUNX1C⁺34^{lo} cells were unable to engraft.

### hPSC-derived and cord blood progenitor cells display discordant HOX gene expression

To explore the reasons for engraftment failure of hPSC-derived hematopoietic cells, transcriptional profiles of cells sorted from d14 *RUNX1C*^{GFP/w} and *RUNX1C*^{GFP/GFP} EBs with repopulation-competent CB CD34⁺ cells were compared. *RUNX1C*^{GFP/w} and *RUNX1C*^{GFP/GFP} EBs were transcriptionally nearly identical, judged by hierarchical clustering of sorted fractions, scatter plot analysis, Pearson correlation coefficients (Figure 4a-e) and the expression of transcription factors and cell surface proteins (Figure 5a). To explain the functional differences between hPSC- and CB-derived CD34⁺ cells, differentially expressed genes were sought, identifying 273 probes (249 genes) up-regulated and 292 probes (270 genes) downregulated in CB CD34⁺ cells. These up-regulated CB CD34⁺ genes with a list of genes expressed in CB HSCs were compared. Thirty-two genes in common, including transcription factors *(BCL11A, HLF, HOXA5, HOXA9* and *HOXA10),* cell surface receptors *(FLT3, PROM1, IGLL1, HTR1F* and *RYR3),* and signaling pathway intermediates (*CTNND1, GAB2, SH3BP4, PRKACB* and *ABL1*) (Figure 5b,c) were identified. Given the early developmental divergence of the primitive and definitive haematopoietic programs, it was hypothesized that expression of transcription factors influencing early cell fate decisions might best mark cells committed to definitive haematopoiesis. Although both *BCL11A* and *HLF* are expressed in foetal haematopoietic stem and progenitor cells, they do not regulate early cell fate. Moreover, neither gene was selectively expressed in nascent mesoderm (Figure 4h,i), arguing against a role in directing definitive haematopoietic commitment.

Conversely, *HOXA5, HOXA9* and *HOXA10* are expressed in the primitive streak and also in HSCs. Furthermore, deletion of *Hoxa9* or the whole *Hoxa* cluster in mesoderm is associated with severe haematopoietic defects. Lastly, failure to express *HOXA* genes was a major difference between hPSC-derived haematopoietic cells and CB CD34⁺ cells (Figure 4f).

*HOXB,* but not *HOXA*, genes were first expressed at d2-3 of hPSC differentiation, coincident with the expression of *MIXL1* and *BRACHYURY (T)* primitive streak genes, and the *CDX* genes that regulate *HOX* expression (Figure 4g,h). Analysis of d4 differentiated cultures confirmed that *CDX* and *HOXB* genes were expressed in *MIXL1⁺* cells (Figure 4i). Therefore, it was hypothesized that *HOXA* gene expression could be used as a surrogate marker to identify culture conditions that generated definitive blood cells.

### HOXA expression in mesoderm is induced by a combination of WNT agonist and ACTIVIN antagonist

The effects of adding the WNT agonist, CHIR99021 (CHIR), and the ACTIVIN receptor-like kinase (ALK) inhibitor, SB431542 (SB), from differentiation days 2 to 4, the period during which *HOX* gene expression is initiated (Figure 4g) were explored. Compared with control cultures, SB addition increased *HOXA3, HOXA7* and *HOXA9* expression, whilst CHIR increased *HOXA9* and *HOXA10* expression (Figure 5d and Figure 6a). When the two molecules were combined (denoted SB/CHIR), greater up-regulation of *HOXA* expression was observed than with either alone (Figure 5d and Figure 6a). Mediating the changes in *HOXA* expression, transient up-regulation in *CDX* gene expression (Figure 5d, e and Figure 6b) were observed. Treatment with SB/CHIR also suppressed primitive haematopoiesis, evidenced as a reduction in CD43⁺ haematopoietic cells at d7 (Figure 5f).

### RNA-Seq analysis confirms enhanced HOXA gene transcription as a major consequence of SB/CHIR treatment

It was hypothesized that the SB/CHIR protocol (Figure 7a) would pattern the haemogenic endothelium from which definitive blood cells arose. To facilitate visualization of the endothelial to haematopoietic transition, a dual reporter line (*SOX17*^{mCHERRY/w}*RUNX1C*^{GFP/w}) in which mCHERRY was targeted to *SOX17* (given its expression in mouse AGM), and GFP to *RUNX1C* (Figure 2i,j) was generated.

The transcriptional profiles of d10 control and SB/CHIR differentiated *SOX17*^{mCHERRY/w}*RUNX1C*^{GFP/w} cells were compared, sorting cultures on CD34, CD43 and SOX17 expression into haematopoietic (43⁺), endothelial (SOX17⁺34⁺ and SOX17⁻34⁺) and CD34⁻ adherent cells (SOX17⁺34⁻ and SOX17⁻34⁻) (Figure 6c). Multidimensional scaling (MDS) plots showed that control and SB/CHIR samples with the same surface phenotype expressed similar genes, and clustered together in the first two dimensions, with culture treatment separating control from SB/CHIR populations in the third and fourth dimensions (Figure 7b, c). In keeping with this, many of the genes enriched in CD34⁺ endothelial fractions were common to both control and SB/CHIR culture conditions (Figure 6d, e). Genes differentially expressed between SB/CHIR and control cultures were observed in all populations, with many of the differentially transcribed genes common to both SOX17⁺ and SOX17⁻ fractions (Figure 6f, g).

*HOXA* cluster genes were up-regulated in all populations sorted from SB/CHIR cultures (Figure 7d and Figure 8a-g). More highly transcribed members included *HOXA5* and *HOXA9-HOXA13,* especially in the endothelial fractions (Figure 7d). Furthermore, the gene ontology terms enriched in genes differentially expressed in SB/CHIR derived endothelia reflected embryonic patterning and almost exclusively comprised homeobox genes (Figure 7e). These data confirmed that *HOXA* genes were major targets up-regulated by SB/CHIR.

### Endothelia from SB/CHIR treated cultures express genes indicative of aortic fate

To define the endothelium developing under control and SB/CHIR conditions, these populations were examined for the expression of genes defining umbilical cord arterial and venous endothelia (Figure 8h). At this differentiation stage, arterial and venous genes were transcribed in all d10 endothelia irrespective of *SOX17* expression or culture conditions (Figure 8h), suggesting that the arterial-venous lineage bifurcation occurs later than d10 of hPSC differentiation.

Importantly, genes were not identified whose expression indicated that SB/CHIR directed endothelium towards an aorta-like fate, from which definitive haematopoeitic cells could subsequently arise. The angiotensin II type 2 receptor *(AGTR2),* expressed in foetal rat aorta, was transcribed in SB/CHIR patterned SOX17⁺ endothelium, whilst the type 1 receptor *(AGTR1)* was expressed in control endothelia (Figure 7f). The transcription factor *NKX2-3,* and matrix metallopeptidase 9 (*MMP9*), both expressed in spleen but not in the major arteries, were more highly expressed in control endothelium (Figure 7g). Similarly, control endothelium expressed both *FGF23* and its co-receptor, *KLOTHO,* whilst SB/CHIR SOX17⁺ endothelium expressed only *KLOTHO,* in keeping with human aorta (Figure 7h). *SELECTINS* were also differentially expressed, with *SELE* up-regulated in SB/CHIR endothelium and *SELP* and *SELL* more highly expressed in control endothelia (Figure 7i). In zebrafish, *SELE* expression localizes to sites of definitive haematopoiesis in the posterior trunk and the AGM-like caudal hematopoietic tissue. Synthesis of retinoic acid, dependent upon *ALDH1A2* expression, is a hallmark of definitive haemogenic endothelium in the mouse AGM. *ALDH1A2* expression was elevated in SB/CHIR endothelium, with higher *ALDH1A1* levels in control endothelia (Figure 7j). Taken together, these data indicate that aorta-like endothelial precursors were selectively generated in SB/CHIR cultures.

The transcriptional programs of the SOX17⁺34⁺ and SOX17⁻34⁺ SB/CHIR derived endothelial populations were then compared. It was observed that *HOXA* and *NOTCH* pathway genes were more highly expressed in SOX17⁺34⁺ cells, whilst haematopoietic transcription factors and surface markers were up-regulated in SOX17⁻34⁺ populations (Figure 6a). Using limit dilution experiments, the haemogenic potential of SB/CHIR endothelia (CD34⁺CD43⁻RUNX1C⁻SOX17^{±}), sorted on SOX17 expression into SOX17-bright, SOX17-dull and SOX17-negative fractions (Figure 9b) were compared. Populations were also stratified on the basis of CD73 expression, because prior studies have shown that CD73 distinguishes vascular from haemogenic endothelium. As expected, haematopoietic cells only arose from CD73⁻ endothelium, with the highest frequency seen in SOX17-dull and - negative fractions (Figure 9b and Table 2). These data identify a reciprocal relationship between SOX17 expression and haemogenic capacity, and suggest that this gene may be down regulated during the endothelial-haematopoietic transition.

**Table 2. Haemogenic precursor frequency in d10 SB/CHIR endothelium. See also Figure 9b.**

| **Cell phenotype** | **Cells/well** | **Haematopoietic cells** | **Clonal frequency*** |
|---|---|---|---|
| **73⁺34⁺⁺43⁻** | | | |
| **SOX17⁺⁺** | 1 | 0/288 | **<1/100,000** |
| | 3 | 0/288 | |
| | 10 | 0/240 | |
| | 1000 | 0/8 | |
| | 5000 | 0/8 | |
| | 10000 | 1/8 | |
| **SOX17⁺** | 1 | 0/24 | **<1/100,000** |
| | 3 | 0/24 | |
| | 1000 | 0/5 | |
| | 5000 | 0/3 | |
| **SOX17⁻** | 1 | 0/288 | **<1/100,000** |
| | 3 | 0/288 | |
| | 10 | 0/240 | |
| | 1000 | 0/5 | |
| **73^{lo}/⁻34⁺⁺43⁻** | | | |
| **SOX17⁺⁺** | 1 | 0/192 | **1:509** |
| | 3 | 1/288 | |
| | 10 | 2/120 | |
| | 300 | 3/8 | |
| | 1000 | 7/8 | |
| | 5000 | 4/4 | |
| | | | |
| **SOX17⁺** | 1 | 0/24 | **1:54** |
| | 3 | 0/24 | |
| | 10 | 8/48 | |
| | 300 | 6/6 | |
| | 1000 | 2/2 | |
| **SOX17⁻** | 1 | 0/192 | **1: 65** |
| | 3 | 0/288 | |
| | 10 | 2/136 | |
| | 300 | 6/6 | |
| | 1000 | 2/2 | |

| | | | |
|---|---|---|---|
| * Poisson distribution was used to calculate clonal frequency, which was equated to the cell number giving 37% negative wells. | | | |

Adherent CD34⁻ cells from control and SB/CHIR cultures expressed a host of growth factor genes including *WNT3, WNT5A, BMP4, JAG1, KITLG* and *VEGF.* Additional genes such as *CXCL12, DLL1, FGF2,* and *ALDH1A2* were selectively up-regulated in SB/CHIR patterned CD34⁻ cells, highlighting the potential supportive role that these cells might play (Figure 7k).

### A pulse of SB/CHIR increases chromatin accessibility of HOX cluster genes

The mechanisms underpinning the sustained increase in *HOXA* expression induced by SB/CHIR were explored. Evaluation of CpG methylation in d10 SOX17⁺ and SOX17⁻ endothelia and SOX17⁻34⁻ cells by microarray showed that the *HOX* clusters were hypomethylated in all populations in both treatment groups, indicating that changes in methylation did not play a major role in *HOXA* gene induction by SB/CHIR (Figure 9c and Figure 10a,b,d-f). This analysis was extended using the Assay for Transposase-Accessible Chromatin with high throughput sequencing (ATAC-Seq), to determine whether *HOX* gene loci differed in chromatin accessibility between control and SB/CHIR-treated endothelia. MDS plots showed that samples clustered by phenotype and by culture treatment (Figure 10c). Of the loci that were more accessible in SB/CHIR cultures, 13 in *SOX17⁺* and 22 in *SOX17⁻* endothelium mapped to *HOX* clusters, with 8 and 10 respectively localized to the *HOXA* cluster. These data indicate that a more accessible chromatin configuration underpinned the enhanced *HOXA* gene expression in response to SB/CHIR.

Correlating the RNA-Seq and ATAC-Seq experiments revealed that the increased chromatin accessibility was accompanied by selective gene expression (Figure 9c and Figure 10d-f). For example, the highest expression across the *HOXA* cluster induced by SB/CHIR was observed for *HOXA9* and *HOXA10*. These data suggest that the *CDX* genes induced by SB/CHIR (Figure 5d, e and Figure 6a, b) predominantly up-regulated posterior *HOXA* genes, and that other factors were needed to enhance anterior *HOXA* transcription.

Overall, these analyses indicate that SB/CHIR changes the transcriptional profile of multiple cell populations within treated cultures, opening chromatin in the *HOXA* cluster and generating SOX17⁺ endothelial cells that bear markers of human aortic cells.

### T cells are generated from SB/CHIR and control cultures

SB/CHIR treatment was correlated with T lymphoid differentiation capacity of d7 endothelial (SOX17⁻34⁺ and SOX17⁺34⁺) and haematopoietic (RUNX1C⁻43⁺ and RUNX1C⁺34^{lo}) cells using previously published protocols (Figure 11a-c). Endothelial cells from both control and SB/CHIR cultures formed an adherent layer of SOX17-expressing cells on OP9 DL4 cells prior to lymphoid development (Figure 11d,e). All sorted populations generated CD45⁺CD56⁻CD7⁺CD5^{±} T lymphoid progenitors and CD45⁺CD56^{hi}CD7^{±} NK cells, although production of CD4⁺CD8⁺CD56⁻ T cells appeared to be greater from SB/CHIR treated cultures (Figure 11f-j). These data are consistent with studies reporting generation of T cells using a range of protocols and with observations that murine T cell precursors arise from two distinct origins - from the pre-circulation mouse yolk sac and from the AGM.

### Haemogenic SOX17⁺ vascular structures arise exclusively from SB/CHIR treated EBs

When d7 EBs were transferred onto MATRIGEL^{™}-coated plates, it was that observed SOX17⁺ vascular structures from d10, exclusively in SB/CHIR cultures (Figure 12a). Haematopoietic cells were abundant in control cultures but virtually absent from SB/CHIR treated cultures, consistent with the suppression of primitive haematopoiesis (Figure 12a and Figure 6c). By day 14, extensive SOX17⁺ vessels were present in SB/CHIR cultures and RUNX1C⁺ blood cells were evident (Figure 12b). Over subsequent days, some regions of the SOX17⁺ vessels expanded and RUNX1C⁺ and RUNX1C⁻ blood cells were seen within, and emerging from, these structures (Figure 12c-h). Marrying these findings with RNA-Seq data showing a gene expression pattern in d10 SOX17⁺ vasculature predictive of a future aorta-like fate, suggested that the regional generation of blood cells resembled the emergence of haematopoietic cells from the AGM.

### SB/CHIR cultures retain cells expressing haematopoietic stem/progenitor cell surface markers and progenitors for erythroid colonies expressing foetal globin genes

Differences between control and SB/CHIR cultures were not restricted to the SOX17⁺ endothelium. The percentage of viable haematopoietic cells, compared to controls, was increased in SB/CHIR cultures. These cultures also included increased numbers of CD34⁺ cells, some of which co-expressed foetal liver HSC markers such as ACE and GPI80 (VNN2) or the FCER1A receptor, up-regulated in CB CD34⁺ cells (Figure 12i-l).

Clonogenic assays from d20-33 revealed that CFCs persisted in SB/CHIR cultures, with an initial myeloid preponderance followed later by predominantly erythroid or mixed colonies, frequently forming multifocal bursts (Figure 13a-h). When MATRIGEL^{™} was included in the methylcellulose colony cultures, the erythroid colonies were frequently seen in close apposition to SOX17⁻ cystic structures (Figure 13e,f), consistent with the observation that the non-hematopoietic component of SB/CHIR cultures expressed supportive growth factors.

Globin gene expression between colonies derived from d23-26 SB/CHIR cultures and primitive haemangioblast colonies generated from control cultures at d4 were compared, and marked reduction in *EPSILON* globin and increase in *BETA* globin expression in the former (Figure 13i,j) was observed, suggesting that cells arising following SB/CHIR treatment represented a later developmental stage.

*HOXA9* and *HOXA10* expression levels in the haematopoietic and stromal components of d23-26 SB/CHIR cultures and the d39-44 derived haematopoietic colonies were similar to in vitro differentiated CB CD34⁺ cells, whilst minimal *HOXA* expression was seen cells from control cultures (Figure 13k). This demonstrated that *HOXA* expression induced by SB/CHIR persisted for over 40 days in vitro. In control cultures, a yolk sac-type of embryonic globin gene expression pattern persisted (Figure 13l), whilst in cells derived from SB/CHIR cultures, the *GAMMA*/*EPSILON* ratio was elevated, reflecting down-regulation of *EPSILON* globin, the first of the β globin switches. Moreover, in the SB/CHIR derived haematopoietic colonies, the *ALPHA*/*ZETA* ratio was increased, indicating that down-regulation of *ZETA* globin represented a separate switch from embryonic to adult α globin (Figure 13l). These data place the late SB/CHIR colonies at a foetal-like developmental stage between the yolk sac-like embryonic precursors and perinatal CB cells. The suppression of *EPSILON* correlated with expression of *KLF1* and *BCL11A,* genes associated with the silencing of *GAMMA* globin. It was observed that SB/CHIR cultures up-regulated both genes, similar to CB (Figure 13m). These results (summarized in Figure 13n) indicate that the cultures encompassed two globin switches - *EPSILON* to *GAMMA* and *ZETA* to *ALPHA* - that were dependent upon SB/CHIR mesodermal patterning.

### Nascent haematopoietic progenitors transiently co-express SOX17 and RUNX1C.

It was hypothesized that haematopoietic progenitors emerging from the SOX17⁺ vascular structures would progress from a SOX17⁺RUNX1C⁻ phenotype, via a SOX17⁺RUNX1C⁺ intermediate, to a more mature SOX17⁻RUNX1C⁺ phenotype. In support of this, CHERRY⁺GFP⁺ haematopoietic cells were observed by microscopy, a result confirmed by flow cytomtery (Figure 14a,b). Flow cytometric analysis also showed that the frequency of SOX17⁺RUNX1C⁻ cells decreased between day 14 and 26, whilst the percentage of SOX17⁻RUNX1C⁺ increased (Figure 14c). Phenotype was correlated with clonogenic frequency, assaying cells sorted on the basis of SOX17, RUNX1C, CD34 and the stem/progenitor cell marker, KIT. Clonogenic cells at d14 and d18 were predominantly myeloid, and enriched in the SOX17⁺RUNX1C⁺34⁺ and SOX17⁻RUNX1C⁺34⁺ populations. KIT expression, which was low at these time points, did not enrich further for clonogenic cells (Figure 14d). In d22 and d26 cultures, however, KIT expression strongly selected for clonogenic cells for all SOX17 and RUNX1C phenotypes (Figure 14d). Whilst the SOX17⁺RUNX1C⁻34⁺KIT⁺ colony forming cells remained myeloid restricted, increasing numbers of multifocal erythroid and mixed colonies emerged from SOX17⁺RUNX1C⁺34⁺KIT⁺ and SOX17⁻RUNX1C⁺34⁺KIT⁺ populations (Figure 14d). This later emergence of erythroid colonies was consistent with the timecourse observed with unsorted cultures (Figure 13g).

### hPSC-derived stem/progenitor cells share expression of key haematopoietic genes with human AGM.

The transcriptional profiles of SOX17 and RUNX1C populations from SB/CHIR cultures with populations that included developing human HSCs sorted from a human 5 week AGM (equivalent to mouse E11.5) and a human 17 week foetal liver (FL) were compared. The AGM sample was fractionated on CD34, CD90 and CD43 into 34⁺90⁺43⁻ endothelium (AGM En), a 34⁺90⁺43⁺ population of cells transiting from haemogenic endothelium to haematopoietic stem/progenitors (AGM S/P), and 34⁺90⁻43⁺ committed progenitor cells (AGM Pr1). The CD43⁺CD45⁺ FL sample was sorted further on CD34, CD38 and CD90 to enrich for haematopoietic stem/progenitor cells (34⁺38^{lo/-}90⁺) (FL S/P). Day 18 hPSC samples included similar populations to the AGM, with SOX17⁺RUNX1C⁻34⁺90⁺ endothelial (d18 S⁺R⁻ En), SOX17⁺RUNX1C⁺34⁺90⁺ haematopoietic stem/progenitor (d18 S⁺R⁺ S/P), and SOX17⁻RUNX1C⁺34⁺90^{lo} committed progenitor (d18 S⁻R⁺ Pr1) fractions. In addition, d22 samples were included, enriched for committed progenitor cells, SOX17⁺RUNX1C⁺34⁺KIT⁺ (d22 S⁺R⁺ Pr2) and SOX17⁻RUNX1C⁺34⁺KIT⁺ (d22 S⁻R⁺ Pr3) (Figure 15a-d). The RUNX1C⁺ populations from day 18 and day 22 also expressed CD45 and CD43.

If the differentiation cultures indeed generated AGM-like haematopoiesis, it was anticipated that the day 18 S⁺R⁻ En and S⁺R⁺ S/P cells would share expression of key genes with the AGM En and S/P fractions, whilst the more mature day 18 S⁻R⁺ Pr1, day 22 S⁺R⁺ Pr2 and S⁻R⁺ Pr3 samples might show greater similarity to the AGM Pr1 committed progenitors.

It was observed that most hematopoietic and vascular cell surface markers expressed on FL S/P cells were shared with AGM S/P and d18 S⁺R⁺ S/P cells (Figure 16a). However, a cohort of markers shared by the AGM En and S/P fractions and the d18 S⁺R⁻ En and S⁺R⁺ S/P cells were poorly expressed on FL S/P and inconsistently present on committed progenitor cells. These included endothelial and haematopoietic genes, integrins, growth factor receptors (*APLNR, LIFR, OSMR*), and the previously identified aorta markers *KLOTHO* and *E-SELECTIN* (Figure 7h,i). Similarly, a group of haematopoietic transcription factors poorly expressed on FL S/P were more highly expressed by the AGM En and S/P fractions and d18 S⁺R⁻ En and S⁺R⁺ S/P cells, including F-box *SOX* genes, *HIF3A, MEIS2* and *GATA6* (Figure 16b).

Very similar patterns of gene expression in corresponding AGM and d18 hPSC En and S/P samples were observed for the *NOTCH, BMP*/*TGFβ* and *WNT* signaling genes (Figure 16c-e). *WNT* expression was strongly biased to the non-canonical pathway, known to antagonize canonical signals, consistent with the argument that *WNT* signals are inhibited to enable emergence of HSCs from the mouse AGM. The retinoic acid synthesis enzyme *ALDH1A1* was more highly expressed in FL S/P cells, whilst *ALDH1A2* was preferentially expressed in the AGM En and S/P and d18 S⁺R⁻ En and S⁺R⁺ S/P cells. Several retinoic acid pathway genes were more highly expressed in AGM En and S/P than in d18 S⁺R⁻ En and S⁺R⁺ S/P cells (Figure 16f). Similarly, *HOXA2-HOXA4* genes remained more highly expressed in AGM En and S/P than in d18 S⁺R⁻ En and S⁺R⁺ S/P cells (Figure 15e and Figure 16h). Significantly, patterns of globin gene expression were virtually identical in AGM and d18 hPSC samples, with residual EPSILON globin observed in Pr1 fractions (Figure 16g).

Finally, the list of genes differentially expressed between CB and the *HOXA*⁻ RUNX1C⁺34⁺ cells (Figure 5c) was revisited. Most of the genes (29/32) were expressed in FL S/P cells, indicating considerable similarity with CB. Day18 S⁺R⁻ En and S⁺R⁺ S/P cells bore greater similarity to the AGM samples than to FL S/Ps, with 15-20 of the 32 genes expressed in the AGM En, S/P and Pr1 populations and 12-26 expressed in the three d18 hPSC samples (Figure 15f). Taken together, the transcriptional profiling data argue strongly that the in vitro derived d18 S⁺R⁻ En and S⁺R⁺ S/P cells represent emerging definitive haematopoietic cells at a similar developmental stage to human AGM En and S/P populations.

### Expression of anterior and posterior HOXA genes requires a combination of retinoic acid signaling and SB/CHIR

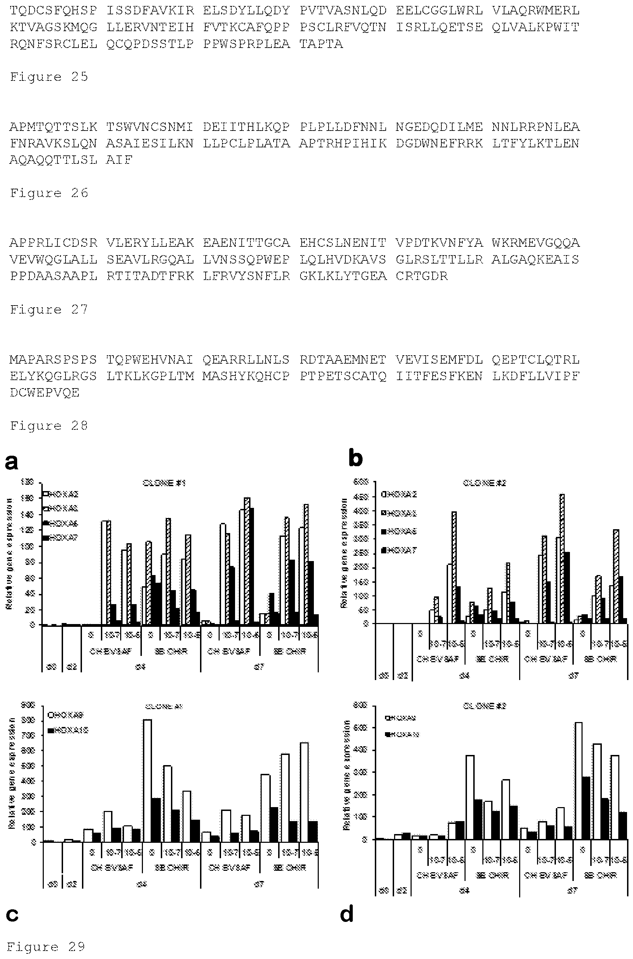

hPSCs were differentiated in medium supplemented with CHIR99021, BMP4, VEGF, SCF, ACTIVIN A and FGF2 (denoted CH BVSAF in Figure 29) from d0-4, or with an additional pulse of SB431542 and CHIR99021 (denoted SB CHIR in Figure 29) from d2-4. Cultures also were treated with the retinoic acid analogue EC23 at 0, 10⁻⁵ M or 10⁻⁷ M concentrations from d2-4 (denoted 0, 10-5 and 10-7, respectively in Figure 29). Samples were harvested at d0, d2, d4 and d7 of differentiation and cDNA was analysed for HOXA2, HOXA3, HOXA5 and HOXA7, which are considered 'anterior' genes, and HOXA9 and HOXA10, which are considered 'posterior' genes. Gene expression was normalized to GAPDH and is shown in arbitrary units in Figure 29.

In the absence of retinoic acid signaling, SB/CHIR, but not CH BVSAF, induced low levels of anterior (Figure 29a,b) and high levels of posterior (Figure 29c,d) HOXA genes.

Retinoic acid signaling from d2-4 induced predominantly anterior HOXA genes irrespective of SB CHIR treatment (Figure 29).

Combining EC23 with SB CHIR induced a balance of both anterior and posterior HOXA genes (Figure 29).

Both 10⁻⁵ M and 10⁻⁷ M concentrations of EC23 were effective (Figure 29).

A comparison of Figure 16h, representing cells cultured in the absence of retinoic acid signaling, with Figure 29, representing cells cultured in the presence of retinoic acid signaling, demonstrates that retinoic acid signaling improves the HOXA expression patterning of cells differentiated according to the procedure disclosed herein, thereby producing cells that replicate AGM HOXA expression patterning with greater fidelity.

### Treating acute myeloid leukaemia

Haematopoietic stem/progenitor cells produced according to the present disclosure will be administered to a subject diagnosed with acute myeloid leukaemia (AML) at a dose of 3 x 10⁶ cells/kg who has undergone myeloablative therapy according to standard protocols prior to allogeneic HSC transplantation. Administration of the haematopoietic stem/ progenitor cells will result in an equivalent or improved response in the subject.

### Treating acute lymphocytic leukaemia

Haematopoietic stem/progenitor cells produced according to the present disclosure will be administered to a subject diagnosed with acute lymphocytic leukaemia (ALL) at a dose of 3 x 10⁶ cells/kg who has undergone myeloablative therapy according to standard protocols prior toallogeneic HSC transplantation. Administration of the haematopoietic stem/ progenitor cells will result in an equivalent or improved response in the subject.

## Claims

1. A method for differentiating a pluripotent stem cell (PSC) into a definitive haematopoietic stem/progenitor cell, the method comprising increasing *HOXA* gene expression by culturing the PSC in a medium comprising a WNT agonist and an ACTIVIN antagonist for about 2 days, wherein increased *HOXA* gene expression is relative to a PSC not cultured in a medium comprising a WNT agonist and an ACTIVIN antagonist.

2. The method of claim 1, wherein the *HOXA* gene is one or more of *HOXA3, HOXA5, HOXA7, HOXA9, HOXA10, HOXA11,* and *HOXA13.*

3. The method of claim 1 or claim 2, wherein culturing the PSC in a medium comprising a WNT agonist and an ACTIVIN antagonist comprises adding the WNT agonist and the ACTIVIN antagonist to the medium at about day 3.

4. The method of any one of claims 1 to 3, wherein the WNT agonist is CHIR99021, optionally wherein the medium comprises about 2 µM, about 2.1 µM, about 2.2 µM, about 2.3 µM, about 2.4 µM, about 2.5 µM, about 2.6 µM, about 2.7 µM, about 2.8 µM, about 2.9 µM, about 3 µM, about 3.1 µM, about 3.2 µM, about 3.3 µM, about 3.4 µM, about 3.5 µM, about 3.6 µM, about 3.7 µM, about 3.8 µM, about 3.9 µM, about 4 µM, about 4.1 µM, about 4.2 µM, about 4.3 µM, about 4.4 µM, about 4.5 µM, about 4.6 µM, about 4.7 µM, about 4.8 µM, about 4.9 µM, or about 5 µM CHIR99021; or 3 µM CHIR99021.

5. The method of any one of claims 1 to 4, wherein the ACTIVIN antagonist is SB431542, optionally wherein the medium comprises about 2 µM, about 2.1 µM, about 2.2 µM, about 2.3 µM, about 2.4 µM, about 2.5 µM, about 2.6 µM, about 2.7 µM, about 2.8 µM, about 2.9 µM, about 3 µM, about 3.1 µM, about 3.2 µM, about 3.3 µM, about 3.4 µM, about 3.5 µM, about 3.6 µM, about 3.7 µM, about 3.8 µM, about 3.9 µM, about 4 µM, about 4.1 µM, about 4.2 µM, about 4.3 µM, about 4.4 µM, about 4.5 µM, about 4.6 µM, about 4.7 µM, about 4.8 µM, about 4.9 µM, or about 5 µM SB431542; or 3-4 µM SB431542.

6. The method of any one of claims 1 to 5, wherein the medium further comprises bone morphogenic protein 4 (BMP4), vascular endothelial growth factor (VEGF), stem cell factor (SCF), ACTIVIN A and fibroblast growth factor 2 (FGF2), and culturing is from about day 0 to about day 4, optionally wherein the medium comprises about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL BMP4, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 65 ng/mL, or about 70 ng/mL VEGF, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 70 ng/mL, about 80 ng/mL, about 90 ng/mL, about 100 ng/mL, about 110 ng/mL, or about 120 ng/mL SCF, about 1 ng/mL, about 2 ng/mL, about 3 ng/mL, about 4 ng/mL, about 5 ng/mL, about 6 ng/mL, about 7 ng/mL, about 8 ng/mL, about 9 ng/mL, about 10 ng/mL, about 11 ng/mL, about 12 ng/mL, about 13 ng/mL, about 14 ng/mL, about 15 ng/mL, about 16 ng/mL, about 17 ng/mL, about 18 ng/mL, about 19 ng/mL, about 20 ng/mL, about 21 ng/mL, about 22 ng/mL, about 23 ng/mL, about 24 ng/mL, about 25 ng/mL, or about 30 ng/mL ACTIVIN A, and about 1 ng/mL, about 2 ng/mL, about 3 ng/mL, about 4 ng/mL, about 5 ng/mL, about 6 ng/mL, about 7 ng/mL, about 8 ng/mL, about 9 ng/mL, about 10 ng/mL, about 11 ng/mL, about 12 ng/mL, about 13 ng/mL, about 14 ng/mL, about 15 ng/mL, about 16 ng/mL, about 17 ng/mL, about 18 ng/mL, about 19 ng/mL, or about 20 ng/mL FGF2; or about 20-40 ng/mL BMP4, about 20-30 ng/mL VEGF, about 40 ng/mL SCF, about 10-20 ng/nL ACTIVIN A, and about 10 ng/mL FGF2.

7. The method of any one of claims 1 to 6, wherein the medium further comprises a retinoid or retinoic acid analogue from day 2, day 3, day 4 or day 5.

8. The method of any one of claims 1 to 7, wherein the PSC is cultured as an embryoid body.

9. The method of any one of claims 1 to 8, further comprising replacing the medium with medium comprising BMP4, VEGF, SCF, FGF2 and insulin-like growth factor 2 (IGF2) at about day 5, optionally wherein the medium comprises about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, or about 60 ng/mL BMP4, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 65 ng/mL, or about 70 ng/mL VEGF, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 70 ng/mL, about 80 ng/mL, about 90 ng/mL, about 100 ng/mL, about 110 ng/mL, or about 120 ng/mL SCF, about 1 ng/mL, about 2 ng/mL, about 3 ng/mL, about 4 ng/mL, about 5 ng/mL, about 6 ng/mL, about 7 ng/mL, about 8 ng/mL, about 9 ng/mL, about 10 ng/mL, about 11 ng/mL, about 12 ng/mL, about 13 ng/mL, about 14 ng/mL, about 15 ng/mL, about 16 ng/mL, about 17 ng/mL, about 18 ng/mL, about 19 ng/mL, or about 20 ng/mL FGF2, and about 20 ng/mL, about 25 ng/mL, about 30 ng/mL, about 35 ng/mL, about 40 ng/mL, about 45 ng/mL, or about 50 ng/mL IGF2; or about 20 ng/mL BMP4, about 50 ng/mL VEGF, about 50 ng/mL SCF, about 10 ng/mL FGF2, and about 30 ng/mL IGF2, optionally comprising culturing the PSC for about 2 days.

10. The method of any one of claims 1 to 9, further comprising transferring the PSC to a culture surface comprising an extracellular matrix protein at about day 7 and culturing the PSC adherently in medium comprising VEGF, SCF, FGF2, IGF2, interleukin 6 (IL-6), thrombopoietin (TPO), FLT3 receptor ligand (FLT3L), and optionally interleukin 3 (IL-3) and/or erythropoietin (EPO), optionally wherein the medium comprises about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 65 ng/mL, or about 70 ng/mL VEGF, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 70 ng/mL, about 80 ng/mL, about 90 ng/mL, about 100 ng/mL, about 110 ng/mL, or about 120 ng/mL SCF, about 10 ng/mL, about 15 ng/mL, about 20 ng/mL, about 25 ng/mL, about 30 ng/mL, about 35 ng/mL, about 40 ng/mL, about 45 ng/mL, or about 50 ng/mL IL-6, about 10 ng/mL, about 15 ng/mL, about 20 ng/mL, about 25 ng/mL, about 30 ng/mL, about 35 ng/mL, about 40 ng/mL, about 45 ng/mL, or about 50 ng/mL TPO, about 1 ng/mL, about 2 ng/mL, about 3 ng/mL, about 4 ng/mL, about 5 ng/mL, about 6 ng/mL, about 7 ng/mL, about 8 ng/mL, about 9 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, or about 70 ng/mL FLT3L, about 1 ng/mL, about 2 ng/mL, about 3 ng/mL, about 4 ng/mL, about 5 ng/mL, about 6 ng/mL, about 7 ng/mL, about 8 ng/mL, about 9 ng/mL, about 10 ng/mL, about 11 ng/mL, about 12 ng/mL, about 13 ng/mL, about 14 ng/mL, about 15 ng/mL, about 16 ng/mL, about 17 ng/mL, about 18 ng/mL, about 19 ng/mL, or about 20 ng/mL FGF2, and about 20 ng/mL, about 25 ng/mL, about 30 ng/mL, about 35 ng/mL, about 40 ng/mL, about 45 ng/mL, or about 50 ng/mL IGF2, and optionally about 10 ng/mL, about 15 ng/mL, about 20 ng/mL, about 25 ng/mL, about 30 ng/mL, about 35 ng/mL, about 40 ng/mL, about 45 ng/mL, or about 50 ng/mL IL-3 and/or about 1 U/mL, about 2 U/mL, about 3 U/mL, about 4 U/mL, about 5 U/mL, about 6 U/mL, about 7 U/mL, about 8 U/mL, about 9 U/mL, or about 10 U/mL EPO; or about 50 ng/mL VEGF, about 50 ng/mL SCF, about 25 ng/mL IL-6, about 30 ng/mL TPO, about 30 ng/mL FLT3L, about 10 ng/mL FGF2, and about 30 ng/mL IGF2, and optionally about 25-30 ng/mL IL-3 and/or about 3 U/mL EPO.

11. The method of any one of claims 1 to 10, wherein the medium is APEL medium.

12. The method of any one of claims 1 to 11, wherein the definitive haematopoietic stem/progenitor cell is capable of producing a SOX17⁺ blood vessel cell or a RUNX1C⁺ blood cell.

13. The method of any one of claims 1 to 12, wherein the PSC is an embryonic stem cell (ESC) or an induced PSC (iPSC), optionally wherein the PSC is human.

14. The method of claim 1 or claim 2, wherein culturing the PSC in a medium comprising a WNT agonist and an ACTIVIN antagonist comprises adding the WNT agonist and the ACTIVIN antagonist to the medium, timed to overlap with the peak expression of primitive streak genes.

## Patentansprüche

1. Verfahren zur Differenzierung einer pluripotenten Stammzelle (PSC) zu einer definitiven hämatopoetische Stamm-/Vorläuferzelle, wobei das Verfahren die Erhöhung der HOXA-Genexpression durch Kultivierung der PSC in einem Medium, das einen WNT-Agonisten und einen ACTIVIN-Antagonisten umfasst, für etwa 2 Tage umfasst, wobei die erhöhte HOXA-Genexpression eine PSC betrifft, die nicht in einem Medium kultiviert wurde, das einen WNT-Agonisten und einen ACTIVIN-Antagonisten umfasst.

2. Verfahren nach Anspruch 1, wobei es sich bei dem *HOXA*-Gen um eines oder mehrere von *HOXA3, HOXA5, HOXA7, HOXA9, HOXA10, HOXA11* und *HOXA13* handelt.

3. Verfahren nach Anspruch 1 oder Anspruch 2, wobei die Kultivierung des PSC in einem Medium, das einen WNT-Agonisten und einen ACTIVIN-Antagonisten umfasst, das Hinzufügen des WNT-Agonisten und des ACTIVIN-Antagonisten zu dem Medium etwa am dritten Tag umfasst.

4. Verfahren nach einem der Ansprüche 1 bis 3, wobei der WNT-Agonist CHIR99021 ist, wobei das Medium gegebenenfalls etwa 2 µM, etwa 2,1 µM, etwa 2,2 µM, etwa 2,3 µM, etwa 2,4 µM, etwa 2,5 µM, etwa 2,6 µM, etwa 2.7 µM, etwa 2,8 µM, etwa 2,9 µM, etwa 3 µM, etwa 3,1 µM, etwa 3,2 µM, etwa 3,3 µM, etwa 3,4 µM, etwa 3,5 µM, etwa 3,6 µM, etwa 3,7 µM, etwa 3,8 µM, etwa 3,9 µM, etwa 4 µM, etwa 4,1 µM, etwa 4,2 µM, etwa 4,3 µM, etwa 4,4 µM, etwa 4,5 µM, etwa 4,6 µM, etwa 4,7 µM, etwa 4,8 µM, etwa 4,9 µM, oder etwa 5 µM CHIR99021; oder 3 µM CHIR99021 umfasst.

5. Verfahren nach einem der Ansprüche 1 bis 4, wobei der ACTIVIN-Antagonist SB431542 ist, wobei das Medium gegebenenfalls etwa 2 µM, etwa 2.1 µM, etwa 2,2 µM, etwa 2.3 µM, etwa 2,4 µM, etwa 2,5 µM, etwa 2,6 µM, etwa 2,7 µM, etwa 2,8 µM, etwa 2,9 µM, etwa 3 µM, etwa 3,1 µM, etwa 3,2 µM, etwa 3,3 µM, etwa 3,4 µM, etwa 3,5 µM, etwa 3,6 µM, etwa 3,7 µM, etwa 3,8 µM, etwa 3,9 µM, etwa 4 µM, etwa 4,1 µM, etwa 4,2 µM, etwa 4,3 µM, etwa 4,4 µM, etwa 4,5 µM, etwa 4,6 µM, etwa 4,7 µM, etwa 4,8 µM, etwa 4,9 µM, oder etwa 5 µM SB431542; or 3-4 µM SB431542 umfasst.

6. Verfahren nach einem der Ansprüche 1 bis 5, wobei das Medium ferner Knochenmorphogenetisches Protein 4 (BMP4), vaskulären endothelialen Wachstumsfaktor (VEGF), Stammzellfaktor (SCF), ACTIVIN A und Fibroblasten-Wachstumsfaktor 2 (FGF2) umfasst, und das Kultivieren von etwa Tag 0 bis etwa Tag 4 erfolgt, gegebenenfalls wobei das Medium etwa 5 ng/mL, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, etwa 50 ng/mL, etwa 55 ng/mL, etwa 60 ng/mL BMP4, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, etwa 50 ng/mL, etwa 55 ng/mL, etwa 60 ng/mL, etwa 65 ng/mL, oder etwa 70 ng/mL VEGF, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, etwa 50 ng/mL, etwa 55 ng/mL, etwa 60 ng/mL, etwa 70 ng/mL, etwa 80 ng/mL, etwa 90 ng/mL, etwa 100 ng/mL, etwa 110 ng/mL, oder etwa 120 ng/mL SCF, etwa 1 ng/mL, etwa 2 ng/mL, etwa 3 ng/mL, etwa 4 ng/mL, etwa 5 ng/mL, etwa 6 ng/mL, etwa 7 ng/mL, etwa 8 ng/mL, etwa 9 ng/mL, etwa 10 ng/mL, etwa 11 ng/mL, etwa 12 ng/mL, etwa 13 ng/mL, etwa 14 ng/mL, etwa 15 ng/mL, etwa 16 ng/mL, etwa 17 ng/mL, etwa 18 ng/mL, etwa 19 ng/mL, etwa 20 ng/mL, etwa 21 ng/mL, etwa 22 ng/mL, etwa 23 ng/mL, etwa 24 ng/mL, etwa 25 ng/mL, oder etwa 30 ng/mL ACTIVIN A, und etwa 1 ng/mL, etwa 2 ng/mL, etwa 3 ng/mL, etwa 4 ng/mL, etwa 5 ng/mL, etwa 6 ng/mL, etwa 7 ng/mL, etwa 8 ng/mL, etwa 9 ng/mL, etwa 10 ng/mL, etwa 11 ng/mL, etwa 12 ng/mL, etwa 13 ng/mL, etwa 14 ng/mL, etwa 15 ng/mL, etwa 16 ng/mL, etwa 17 ng/mL, etwa 18 ng/mL, etwa 19 ng/mL, oder etwa 20 ng/mL FGF2; oder etwa 20-40 ng/mL BMP4, etwa 20-30 ng/mL VEGF, etwa 40 ng/mL SCF, etwa 10-20 ng/nL ACTIVIN A, und etwa 10 ng/mL FGF2 umfasst.

7. Verfahren nach einem der Ansprüche 1 bis 6, wobei das Medium ferner ein Retinoid oder ein Retinsäureanalogon ab Tag 2, Tag 3, Tag 4 oder Tag 5 umfasst.

8. Verfahren nach einem der Ansprüche 1 bis 7, wobei das PSC als embryoidaler Körper kultiviert wird.

9. Das Verfahren nach einem der Ansprüche 1 bis 8, ferner umfassend das Ersetzen des Mediums durch ein Medium, umfassend BMP4, VEGF, SCF, FGF2 und insulinähnlichen Wachstumsfaktor 2 (IGF2) an etwa Tag 5, gegebenenfalls wobei das Medium umfasst etwa 5 ng/mL, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, etwa 50 ng/mL, etwa 55 ng/mL, oder etwa 60 ng/mL BMP4, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, etwa 50 ng/mL, etwa 55 ng/mL, etwa 60 ng/mL, etwa 65 ng/mL, oder etwa 70 ng/mL VEGF, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, etwa 50 ng/mL, etwa 55 ng/mL, etwa 60 ng/mL, etwa 70 ng/mL, etwa 80 ng/mL, etwa 90 ng/mL, etwa 100 ng/mL, etwa 110 ng/mL, oder etwa 120 ng/mL SCF, etwa 1 ng/mL, etwa 2 ng/mL, etwa 3 ng/mL, etwa 4 ng/mL, etwa 5 ng/mL, etwa 6 ng/mL, etwa 7 ng/mL, etwa 8 ng/mL, etwa 9 ng/mL, etwa 10 ng/mL, etwa 11 ng/mL, etwa 12 ng/mL, etwa 13 ng/mL, etwa 14 ng/mL, etwa 15 ng/mL, etwa 16 ng/mL, etwa 17 ng/mL, etwa 18 ng/mL, etwa 19 ng/mL, oder etwa 20 ng/mL FGF2, und etwa 20 ng/mL, etwa 25 ng/mL, etwa 30 ng/mL, etwa 35 ng/mL, etwa 40 ng/mL, etwa 45 ng/mL, oder etwa 50 ng/mL IGF2; oder etwa 20 ng/mL BMP4, etwa 50 ng/mL VEGF, etwa 50 ng/mL SCF, etwa 10 ng/mL FGF2, und etwa 30 ng/mL IGF2, gegebenenfalls umfassend die Kultivierung des PSC für etwa 2 Tage.

10. Verfahren nach einem der Ansprüche 1 bis 9, ferner umfassend das Übertragen der PSC auf eine Kulturfläche, umfassend ein extrazelluläres Matrixprotein, an etwa Tag 7 und das adhäsiv Kultivieren der PSC in einem Medium, umfassend VEGF, SCF, FGF2, IGF2, Interleukin 6 (IL-6), Thrombopoietin (TPO), FLT3-Rezeptorligand (FLT3L) und gegebenenfalls Interleukin 3 (IL-3) und/oder Erythropoietin (EPO), wobei das Medium gegebenenfalls umfasst etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, etwa 50 ng/mL, etwa 55 ng/mL, etwa 60 ng/mL, etwa 65 ng/mL, oder etwa 70 ng/mL VEGF, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, etwa 50 ng/mL, etwa 55 ng/mL, etwa 60 ng/mL, etwa 70 ng/mL, etwa 80 ng/mL, etwa 90 ng/mL, etwa 100 ng/mL, etwa 110 ng/mL, oder etwa 120 ng/mL SCF, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, oder etwa 50 ng/mL IL-6, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, oder etwa 50 ng/mL TPO, etwa 1 ng/mL, etwa 2 ng/mL, etwa 3 ng/mL, etwa 4 ng/mL, etwa 5 ng/mL, etwa 6 ng/mL, etwa 7 ng/mL, etwa 8 ng/mL, etwa 9 ng/mL, etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, etwa 50 ng/mL, etwa 55 ng/mL, etwa 60 ng/mL, oder etwa 70 ng/mL FLT3L, etwa 1 ng/mL, etwa 2 ng/mL, etwa 3 ng/mL, etwa 4 ng/mL, etwa 5 ng/mL, etwa 6 ng/mL, etwa 7 ng/mL, etwa 8 ng/mL, etwa 9 ng/mL, etwa 10 ng/mL, etwa 11 ng/mL, etwa 12 ng/mL, etwa 13 ng/mL, etwa 14 ng/mL, etwa 15 ng/mL, etwa 16 ng/mL, etwa 17 ng/mL, etwa 18 ng/mL, etwa 19 ng/mL, oder etwa 20 ng/mL FGF2, und etwa 20 ng/mL, etwa 25 ng/mL, etwa 30 ng/mL, etwa 35 ng/mL, etwa 40 ng/mL, etwa 45 ng/mL, oder etwa 50 ng/mL IGF2, und gegebenenfalls etwa 10 ng/mL, etwa 15 ng/mL, etwa 20 ng/mL, etwa 25 ng/mL, etwa 30 ng/mL, etwa 35 ng/mL, etwa 40 ng/mL, etwa 45 ng/mL, oder etwa 50 ng/mL IL-3 und/oder etwa 1 U/mL, etwa 2 U/mL, etwa 3 U/mL, etwa 4 U/mL, etwa 5 U/mL, etwa 6 U/mL, etwa 7 U/mL, etwa 8 U/mL, etwa 9 U/mL, oder etwa 10 U/mL EPO; oder etwa 50 ng/mL VEGF, etwa 50 ng/mL SCF, etwa 25 ng/mL IL-6, etwa 30 ng/mL TPO, etwa 30 ng/mL FLT3L, etwa 10 ng/mL FGF2, und etwa 30 ng/mL IGF2, und gegebenenfalls etwa 25-30 ng/mL IL-3 und/oder etwa 3 U/mL EPO.

11. Verfahren nach einem der Ansprüche 1 bis 10, wobei das Medium APEL-Medium ist.

12. Verfahren nach einem der Ansprüche 1 bis 11, wobei die definitive hämatopoetische Stamm-/Vorläuferzelle in der Lage ist, eine SOX17⁺-Blutgefäßzelle oder eine RUNX1C⁺-Blutzelle zu produzieren.

13. Verfahren nach einem der Ansprüche 1 bis 12, wobei die PSC eine embryonale Stammzelle (ESC) oder eine induziert PSC (iPSC) ist, gegebenenfalls wobei die PSC menschlich ist.

14. Verfahren nach Anspruch 1 oder Anspruch 2, wobei das Kultivieren der PSC in einem Medium, umfassend einen WNT-Agonisten und einen ACTIVIN-Antagonisten, das Hinzufügen des WNT-Agonisten und des ACTIVIN-Antagonisten zu dem Medium zeitlich abgestimmt, um mit der Peak-Expression von Primitivstreifen-Genen zu überlappen, umfasst.

## Revendications

1. Procédé de différenciation d'une cellule souche pluripotente (PSC) en une cellule souche/progénitrice hématopoïétique définitive, le procédé comprenant l'augmentation de l'expression du gène *HOXA* par culture de la PSC dans un milieu comprenant un agoniste de WNT et un antagoniste d'ACTIVINE pendant environ 2 jours, dans lequel l'augmentation de l'expression du gène *HOXA* est par rapport à une PSC non cultivée dans un milieu comprenant un agoniste de WNT et un antagoniste d'ACTIVINE.

2. Procédé selon la revendication 1, dans lequel le gène *HOXA* est un ou plusieurs parmi *HOXA3, HOXA5, HOXA7, HOXA9, HOXA10, HOXA11* et *HOXA13.*

3. Procédé selon la revendication 1 ou la revendication 2, dans lequel la culture de la PSC dans un milieu comprenant un agoniste de WNT et un antagoniste d'ACTIVINE comprend l'ajout de l'agoniste de WNT et de l'antagoniste d'ACTIVINE au milieu environ au jour 3.

4. Procédé selon l'une quelconque des revendications 1 à 3, dans lequel l'agoniste de WNT est CHIR99021, éventuellement dans lequel le milieu comprend environ 2 µM, environ 2,1 µM, environ 2,2 µM, environ 2,3 µM, environ 2,4 µM, environ 2,5 µM, environ 2,6 µM, environ 2,7 µM, environ 2,8 µM, environ 2,9 µM, environ 3 µM, environ 3,1 µM, environ 3,2 µM, environ 3,3 µM, environ 3,4 µM, environ 3,5 µM, environ 3,6 µM, environ 3,7 µM, environ 3,8 µM, environ 3,9 µM, environ 4 µM, environ 4,1 µM, environ 4,2 µM, environ 4,3 µM, environ 4,4 µM, environ 4,5 µM, environ 4,6 µM, environ 4,7 µM, environ 4,8 µM, environ 4,9 µM, ou environ 5 µM de CHIR99021 ; ou 3 µM de CHIR99021.

5. Procédé selon l'une quelconque des revendications 1 à 4, dans lequel l'antagoniste d'ACTIVINE est SB431542, éventuellement dans lequel le milieu comprend environ 2 µM, environ 2,1 µM, environ 2,2 µM, environ 2,3 µM, environ 2,4 µM, environ 2,5 µM, environ 2,6 µM, environ 2,7 µM, environ 2,8 µM, environ 2,9 µM, environ 3 µM, environ 3,1 µM, environ 3,2 µM, environ 3,3 µM, environ 3,4 µM, environ 3,5 µM, environ 3,6 µM, environ 3,7 µM, environ 3,8 µM, environ 3,9 µM, environ 4 µM, environ 4,1 µM, environ 4,2 µM, environ 4,3 µM, environ 4,4 µM, environ 4,5 µM, environ 4,6 µM, environ 4,7 µM, environ 4,8 µM, environ 4,9 µM, ou environ 5 µM de SB431542 ; ou 3-4 µM de SB431542.

6. Procédé selon l'une quelconque des revendications 1 à 5, dans lequel le milieu comprend en outre la protéine morphogénique osseuse 4 (BMP4), le facteur de croissance endothéliale vasculaire (VEGF), le facteur de cellules souches (SCF), l'ACTIVINE A et le facteur de croissance fibroblastique 2 (FGF2), et la culture a lieu d'environ le jour 0 à environ le jour 4, éventuellement dans lequel le milieu comprend environ 5 ng/mL, environ 10 ng/mL, environ 15 ng/mL, environ 20 ng/mL, environ 25 ng/mL, environ 30 ng/mL, environ 35 ng/mL, environ 40 ng/mL, environ 45 ng/mL, environ 50 ng/mL, environ 55 ng/mL, environ 60 ng/mL de BMP4, environ 10 ng/mL, environ 15 ng/mL, environ 20 ng/mL, environ 25 ng/mL, environ 30 ng/mL, environ 35 ng/mL, environ 40 ng/mL, environ 45 ng/mL, environ 50 ng/mL, environ 55 ng/mL, environ 60 ng/mL, environ 65 ng/mL, ou environ 70 ng/mL de VEGF, environ 10 ng/mL, environ 15 ng/mL, environ 20 ng/mL, environ 25 ng/mL, environ 30 ng/mL, environ 35 ng/mL, environ 40 ng/mL, environ 45 ng/mL, environ 50 ng/mL, environ 55 ng/mL, environ 60 ng/mL, environ 70 ng/mL, environ 80 ng/mL, environ 90 ng/mL, environ 100 ng/mL, environ 110 ng/mL, ou environ 120 ng/mL de SCF, environ 1 ng/mL, environ 2 ng/mL, environ 3 ng/mL, environ 4 ng/mL, environ 5 ng/mL, environ 6 ng/mL, environ 7 ng/mL, environ 8 ng/mL, environ 9 ng/mL, environ 10 ng/mL, environ 11 ng/mL, environ 12 ng/mL, environ 13 ng/mL, environ 14 ng/mL, environ 15 ng/mL, environ 16 ng/mL, environ 17 ng/mL, environ 18 ng/mL, environ 19 ng/mL, environ 20 ng/mL, environ 21 ng/mL, environ 22 ng/mL, environ 23 ng/mL, environ 24 ng/mL, environ 25 ng/mL, ou environ 30 ng/mL d'ACTIVINE A, et environ 1 ng/mL, environ 2 ng/mL, environ 3 ng/mL, environ 4 ng/mL, environ 5 ng/mL, environ 6 ng/mL, environ 7 ng/mL, environ 8 ng/mL, environ 9 ng/mL, environ 10 ng/mL, environ 11 ng/mL, environ 12 ng/mL, environ 13 ng/mL, environ 14 ng/mL, environ 15 ng/mL, environ 16 ng/mL, environ 17 ng/mL, environ 18 ng/mL, environ 19 ng/mL ou environ 20 ng/mL de FGF2 ; ou environ 20-40 ng/mL de BMP4, environ 20-30 ng/mL de VEGF, environ 40 ng/mL de SCF, environ 10-20 ng/NL d'ACTIVINE A et environ 10 ng/mL de FGF2.

7. Procédé selon l'une quelconque des revendications 1 à 6, dans lequel le milieu comprend en outre un rétinoïde ou un analogue d'acide rétinoïque à partir du jour 2, du jour 3, du jour 4 ou du jour 5.

8. Procédé selon l'une quelconque des revendications 1 à 7, dans lequel la PSC est cultivée sous la forme d'un corps embryoïde.

9. Procédé selon l'une quelconque des revendications 1 à 8, comprenant en outre le remplacement du milieu par un milieu comprenant BMP4, VEGF, SCF, FGF2 et le facteur de croissance analogue à l'insuline 2 (IGF2) environ au jour 5, éventuellement dans lequel le milieu comprend environ 5 ng/mL, environ 10 ng/mL, environ 15 ng/mL, environ 20 ng/mL, environ 25 ng/mL, environ 30 ng/mL, environ 35 ng/mL, environ 40 ng/mL, environ 45 ng/mL, environ 50 ng/mL, environ 55 ng/mL, ou environ 60 ng/mL de BMP4, environ 10 ng/mL, environ 15 ng/mL, environ 20 ng/mL, environ 25 ng/mL, environ 30 ng/mL, environ 35 ng/mL, environ 40 ng/mL, environ 45 ng/mL, environ 50 ng/mL, environ 55 ng/mL, environ 60 ng/mL, environ 65 ng/mL, ou environ 70 ng/mL de VEGF, environ 10 ng/mL, environ 15 ng/mL, environ 20 ng/mL, environ 25 ng/mL, environ 30 ng/mL, environ 35 ng/mL, environ 40 ng/mL, environ 45 ng/mL, environ 50 ng/mL, environ 55 ng/mL, environ 60 ng/mL, environ 70 ng/mL, environ 80 ng/mL, environ 90 ng/mL, environ 100 ng/mL, environ 110 ng/mL, ou environ 120 ng/mL de SCF, environ 1 ng/mL, environ 2 ng/mL, environ 3 ng/mL, environ 4 ng/mL, environ 5 ng/mL, environ 6 ng/mL, environ 7 ng/mL, environ 8 ng/mL, environ 9 ng/mL, environ 10 ng/mL, environ 11 ng/mL, environ 12 ng/mL, environ 13 ng/mL, environ 14 ng/mL, environ 15 ng/mL, environ 16 ng/mL, environ 17 ng/mL, environ 18 ng/mL, environ 19 ng/mL, ou environ 20 ng/mL de de FGF2, et environ 20 ng/mL, environ 25 ng/mL, environ 30 ng/mL, environ 35 ng/mL, environ 40 ng/mL, environ 45 ng/mL, ou environ 50 ng/mL d'IGF2 ; ou environ 20 ng/mL de BMP4, environ 50 ng/mL de VEGF, environ 50 ng/mL de SCF, environ 10 ng/mL de FGF2, et environ 30 ng/mL d'IGF2, éventuellement comprenant la culture de la PSC pendant environ 2 jours.

10. Procédé selon l'une quelconque des revendications 1 à 9, comprenant en outre le transfert de la PSC sur une surface de culture comprenant une protéine de matrice extracellulaire environ au jour 7 et la culture de la PSC par adhérence dans un milieu comprenant VEGF, SCF, FGF2, IGF2, l'interleukine 6 (IL-6), la thrombopoïétine (TPO), le ligand du récepteur FLT3 (FLT3L), et éventuellement l'interleukine 3 (IL-3) et/ou l'érythropoïétine (EPO), éventuellement dans lequel le milieu comprend environ 10 ng/mL, environ 15 ng/mL, environ 20 ng/mL, environ 25 ng/mL, environ 30 ng/mL, environ 35 ng/mL, environ 40 ng/mL, environ 45 ng/mL, environ 50 ng/mL, environ 55 ng/mL, environ 60 ng/mL, environ 65 ng/mL, ou environ 70 ng/mL de VEGF, environ 10 ng/mL, environ 15 ng/mL, environ 20 ng/mL, environ 25 ng/mL, environ 30 ng/mL, environ 35 ng/mL, environ 40 ng/mL, environ 45 ng/mL, environ 50 ng/mL, environ 55 ng/mL, environ 60 ng/mL, environ 70 ng/mL, environ 80 ng/mL, environ 90 ng/mL, environ 100 ng/mL, environ 110 ng/mL, ou environ 120 ng/mL de SCF, environ 10 ng/mL, environ 15 ng/mL, environ 20 ng/mL, environ 25 ng/mL, environ 30 ng/mL, environ 35 ng/mL, environ 40 ng/mL, environ 45 ng/mL ou environ 50 ng/mL d'IL-6, environ 10 ng/mL, environ 15 ng/mL, environ 20 ng/mL, environ 25 ng/mL, environ 30 ng/mL, environ 35 ng/mL, environ 40 ng/mL, environ 45 ng/mL ou environ 50 ng/mL de TPO, environ 1 ng/mL, environ 2 ng/mL, environ 3 ng/mL, environ 4 ng/mL, environ 5 ng/mL, environ 6 ng/mL, environ 7 ng/mL, environ 8 ng/mL, environ 9 ng/mL, environ 10 ng/mL, environ 15 ng/mL, environ 20 ng/mL, environ 25 ng/mL, environ 30 ng/mL, environ 35 ng/mL, environ 40 ng/mL, environ 45 ng/mL, environ 50 ng/mL, environ 55 ng/mL, environ 60 ng/mL, ou environ 70 ng/mL de FLT3L, environ 1 ng/mL, environ 2 ng/mL, environ 3 ng/mL, environ 4 ng/mL, environ 5 ng/mL, environ 6 ng/mL, environ 7 ng/mL, environ 8 ng/mL, environ 9 ng/mL, environ 10 ng/mL, environ 11 ng/mL, environ 12 ng/mL, environ 13 ng/mL, environ 14 ng/mL, environ 15 ng/mL, environ 16 ng/mL, environ 17 ng/mL, environ 18 ng/mL, environ 19 ng/mL, ou environ 20 ng/mL de FGF2, et environ 20 ng/mL, environ 25 ng/mL, environ 30 ng/mL, environ 35 ng/mL, environ 40 ng/mL, environ 45 ng/mL, ou environ 50 ng/mL d'IGF2, et éventuellement environ 10 ng/mL, environ 15 ng/mL, environ 20 ng/mL, environ 25 ng/mL, environ 30 ng/mL, environ 35 ng/mL, environ 40 ng/mL, environ 45 ng/mL ou environ 50 ng/mL d'IL-3 et/ou environ 1 U/mL, environ 2 U/mL, environ 3 U/mL, environ 4 U/mL, environ 5 U/mL, environ 6 U/mL, environ 7 U/mL, environ 8 U/mL, environ 9 U/mL ou environ 10 U/mL d'EPO ; ou environ 50 ng/mL de VEGF, environ 50 ng/mL de SCF, environ 25 ng/mL d'IL-6, environ 30 ng/mL de TPO, environ 30 ng/mL de FLT3L, environ 10 ng/mL de FGF2 et environ 30 ng/mL d'IGF2, et éventuellement environ 25-30 ng/mL d'IL-3 et/ou environ 3 U/mL d'EPO.

11. Procédé selon l'une quelconque des revendications 1 à 10, dans lequel le milieu est un milieu APEL.

12. Procédé selon l'une quelconque des revendications 1 à 11, dans lequel la cellule souche/progénitrice hématopoïétique définitive est capable de produire une cellule de vaisseau sanguin SOX17⁺ ou une cellule sanguine RUNX1C⁺ .

13. Procédé selon l'une quelconque des revendications 1 à 12, dans lequel la PSC est une cellule souche embryonnaire (ESC) ou une PSC induite (iPSC), éventuellement dans lequel la PSC est humaine.

14. Procédé selon la revendication 1 ou la revendication 2, dans lequel la culture de la PSC dans un milieu comprenant un agoniste de WNT et un antagoniste d'ACTIVINE comprend l'ajout de l'agoniste de WNT et de l'antagoniste d'ACTIVINE au milieu, programmé pour se chevaucher dans le temps avec l'expression maximale des gènes de ligne primitive.
